# EUROPEAN PATENT APPLICATION

(11) **EP 1 982 987 A1**
(43) Date of publication of application: **22.10.2008**
(21) Application number: 07384021.7
(22) Date of filing: 16.04.2007
(51) Int. Cl.: C07D 491/20, A61P 23/00, A61P 25/00, A61K 31/438

(54) **Spiro-pyrano-pyrazole derivatives**

(71) Applicant: Laboratorios del Dr. Esteve S.A., 08041 Barcelona (ES)
(72) Inventor: Wünsch, Bernard Prof. Dr, 48161 Münster (DE); Schepmann, Dirk Dr., 48147 Münster (DE); Schläger, Torsten, 48151 Münster (DE); Zamanillo-Castenedo, Daniel Dr., 08041 Barcelona (ES)
(74) Representative: Peters, Hajo

(57) **Abstract**

The present invention relates to compounds of formula (I) having pharmacological activity towards the sigma (σ) receptor, and more particularly to some spiro-pyrano-pyrazole derivatives, to processes of preparation of such compounds, to pharmaceutical compositions comprising them, and to their use in therapy and prophylaxis, in particular for the treatment of psychosis.

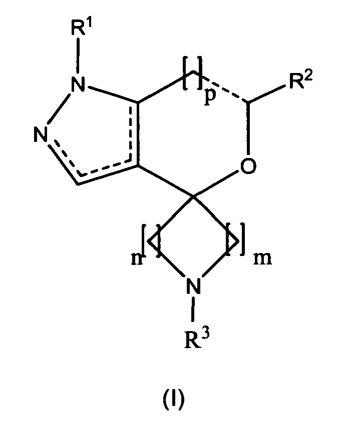

## Description

### FIELD OF THE INVENTION

The present invention relates to compounds having pharmacological activity towards the sigma (σ) receptor, and more particularly to some spiro-pyrano-pyrazole derivatives, to processes of preparation of such compounds, to pharmaceutical compositions comprising them, and to their use in therapy and prophylaxis, in particular for the treatment of psychosis.

### BACKGROUND OF THE INVENTION

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

There is still a need to find compounds that have pharmacological activity towards the sigma receptor, being both effective and selective, and having good "drugability" properties, i.e. good pharmaceutical properties related to administration, distribution, metabolism and excretion.

The closest technique known comprises benzimidazoles of WO2003035065 for the inhibition of kinases. Tetrahydro-pyranopyrazole compounds displaying cannabinoid modulating activity are disclosed in WO2007001939 and FR2875230. None of the them presents spiro-pyrano-pyrazole variants or analogues.

Spiropiperidines are known as potent ligands to sigma receptors (Maier et al, J Med Chem, 2002, 45, 438-448 and Maier et al, J Med Chem, 2002, 45, 4923-4930). However, such spiropiperidines show benzofuran and benzopyran rings.

### SUMMARY OF THE INVENTION

We have now found a family of structurally distinct spiro[benzopyran] or spiro[benzofuran] Derivatives which are particularly selective inhibitors of the sigma receptor.

The invention is directed to compounds of general formula (I), 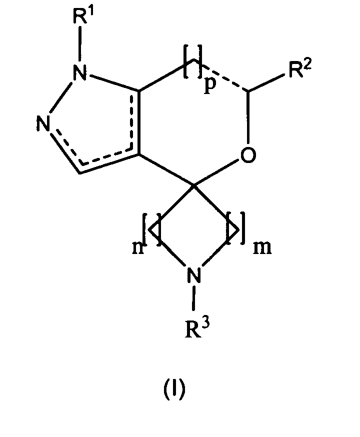 wherein
m is selected from 1, 2 or 3, n is selected from 1, 2 or 3, and m + n is either 3, 4 or 5;
p is selected from 0 or 1;
the dotted line ......... is either a double or a single bond;
if p is 1, the dotted line ......... is either a double or a single bond;
if p is 0, the dotted line ......... is a single bond;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; O-R with R being H or an optionally at least monosubstituted, linear or branched C₁₋₆-aliphatic group;
R³ is selected from hydrogen; an optionally at least monosubstituted, linear or branched C₁₋₁₈-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl; or COOR' with R' being either H or C₁₋₄-alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In the context of this invention aliphatic group or aliphatic radical includes alkyl, alkenyl and alkinyl.

In the context of this invention, alkyl radical or group is understood as meaning saturated, linear or branched hydrocarbons, which can be unsubstituted or mono- or polysubstituted. Alkenyl and alkinyl groups, on the other hand include groups like e.g. -CH=CH-CH₃ or -C=C-CH₃, while the saturated alkyl encompasses e.g. -CH₃ and -CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

In the context of this invention alkyl-cycloalkyl is understood as meaning a cycloalkyl group (see above) being connected to another atom through a C₁₋₆-alkyl group (see above), whereas the C₁₋₆-alkyl-group is always saturated and unsubstituted, and linear or branched.

In connection with alkyl or aliphatic group - unless defined otherwise - the term substituted in the context of this invention is understood as meaning replacement of at least one hydrogen radical by F, Cl, Br, I, NH₂, SH or OH, "polysubstituted" (more than once substituted) radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. "Optionally at least monosubstituted" means either "monosubstituted", "polysubstituted" or - if the option is not fulfilled - "unsubstituted".

The term (CH₂)₃₋₆ is to be understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through a C₁₋₆-alkyl-group (see above), whereas the C₁₋₆-alkyl-group is always saturated and unsubstituted, and linear or branched.

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through a C₁₋₆-alkyl group (see above), whereas the C₁₋₆-alkyl-group is always saturated and unsubstituted, and linear or branched.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, NO₂, COOH; NRₓR_{y}, with Rₓ and R_{y} independently being either H or a saturated or unsaturated, linear or branched, substituted or unsubstituted C₁₋₆-alkyl; a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted -S-C₁₋₆₋ alkyl; a saturated or unsaturated, linear or branched, substituted or unsubstituted-C(O)-C₁₋₆-alkyl-group; a saturated or unsaturated, linear or branched, substituted or unsubstituted -C(O)-O-₁₋₆-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl. "Optionally at least monosubstituted" means either "monosubstituted", "polysubstituted" or - if the option is not fulfilled - "unsubstituted".

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids and in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Any compound that is a prodrug of a compound of formula (I) is within the scope of the invention. The term "prodrug" is used in its broadest sense and encompasses those Derivatives that are converted in vivo to the compounds of the invention. Such Derivatives would readily occur to those skilled in the art, and include, depending on the functional groups present in the molecule and without limitation, the following Derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a preferred embodiment of the compound according to the invention according to general formula I R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferably R¹ is selected from CH₃ or phenyl.

In another preferred embodiment of the compound according to the invention according to general formula I R² is selected from OR with R being H or an optionally at least monosubstituted, linear or branched C₁₋₆-aliphatic group; especially R² is selected from OR with R being H or a linear or branched C₁₋₄-alkyl group, more preferably R² is selected from OH or OCH₃.

In another preferred embodiment of the compound according to the invention according to general formula I R³ is selected from H; an optionally at least monosubstituted, linear or branched C₁₋₁₈-aliphatic group; an optionally at least monosubstituted alkyl-aryl; an optionally at least monosubstituted alkyl-heterocyclyl; or an optionally at least monosubstituted alkyl-cycloalkyl.

In a preferred embodiment of the compound according to the invention according to general formula I m and n are selected from 1 or 2 and m+n are selected from 3 or 4.

In another very preferred embodiment of the compound according to the invention the compounds are compounds according to general formula la 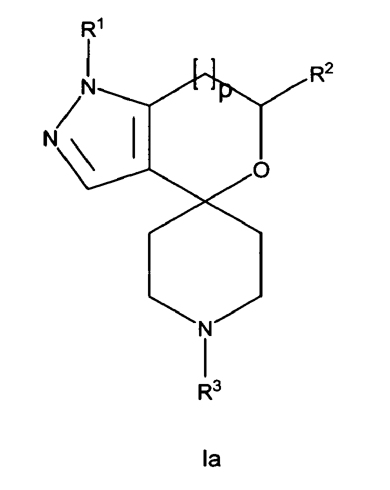 wherein
p is selected from 0 or 1;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; or OR with R being H or an optionally at least monosubstituted, linear or branched C₁₋₆-aliphatic group;
R³ is selected from hydrogen; an optionally at least monosubstituted, linear or branched C₁₋₁₈-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; an optionally at least monosubstituted alkyl-cycloalkyl; or COOR' with R' being either H or C₁₋₄-alkyl.

In another preferred embodiment of the compound according to the invention according to general formula la R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferably R¹ is selected from CH₃ or phenyl.

In another preferred embodiment of the compound according to the invention according to general formula Ia R² is selected from H; OR with R being H or an optionally at least monosubstituted, linear or branched C₁₋₆-alkyl group; especially R² is selected from H; OH or a linear or branched OC₁₋₄-alkyl group; more preferably R² is selected from H, OH or OCH₃.

In another preferred embodiment of the compound according to the invention according to general formula Ia R³ is selected from H; an optionally at least monosubstituted, linear or branched C₁₋₁₈-aliphatic group; an optionally at least monosubstituted alkyl-aryl; an optionally at least monosubstituted alkyl-heterocyclyl; or an optionally at least monosubstituted alkyl-cycloalkyl.

In another preferred embodiment of the compound according to the invention according to general formula la R³ is selected from
hydrogen, C₁₋₁₀-alkyl, linear or branched; C₁₋₁₀-alkenyl, linear or branched; optionally at least mono-substituted C₁₋₆-alkyl-aryl; optionally at least mono-substituted C₁₋₆-alkyl-heterocyclyl; or optionally at least mono-substituted C₁₋₆-alkyl-cycloalkyl;
preferably R³ is selected from hydrogen; C₁₋₁₀alkyl, linear or branched; C₃₋₈-alkenyl, linear or branched; optionally at least mono-substituted C₁₋₆-alkyl-aryl; optionally at least mono-substituted C₁₋₆-alkyl-heterocyclyl; or optionally at least mono-substituted C₁₋₆-alkyl-C₄₋₈-cycloalkyl.

In another highly preferred embodiment of the compound according to the invention the compounds are compounds according to general formula la 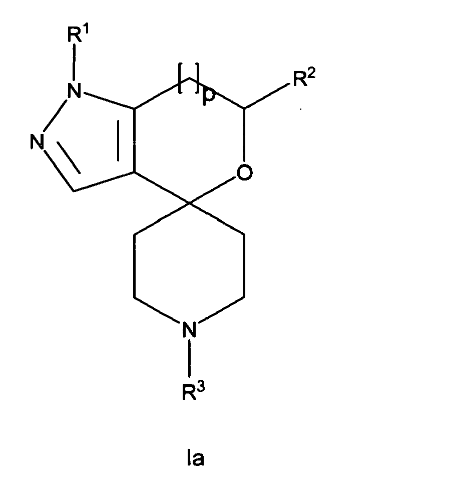 wherein
p is selected from 0 or 1;
R¹ is selected from linear or branched C₁₋₄-alkyl; or optionally at least monosubstituted aryl;
R² is selected from H; OH or a linear or branched OC₁₋₄-alkyl group;
R³ is selected from hydrogen; C₁₋₁₀-alkyl, linear or branched; C₃₋₈-alkenyl, linear or branched; optionally at least mono-substituted C₁₋₆-alkyl-aryl; optionally at least mono-substituted C₁₋₆-alkyl-heterocyclyl; or optionally at least mono-substituted C₁₋₆-alkyl-C₄₋₈-cycloalkyl.

In another preferred embodiment of the compound according to the invention the compounds according to general formula I are selected from:
- 6'-Methoxy-1'-phenyl-4',6'-dihydro-1'H-spiro[piperidin-4,4'-furo[3,4-c]pyrazole];
- 1-Benzyl-6'-methoxy-1'-phenyl-4',6'-dihydro-1'H-spiro[piperidin-4,4'-furo[3,4-c]pyrazole];
- 1-Butyl-6'-methoxy-1'-phenyl-4',6'-dihydro-1'H-spiro[piperidin-4,4'-furo[3,4-c]pyrazole];
- 1-Benzyl-6'-methoxy-1'-phenyl-6',7'-dihydro-1'H-spiro[piperidin-4,4'-pyrano[4,3-c]pyrazole];
- 6'-Methoxy-1'-phenyl-1-propyl-6',7'-dihydro-1'H-spiro[piperidin-4,4'-pyrano[4,3-c]pyrazole];
- 1-Isopropyl-6'-methoxy-1'-phenyl-6',7'-dihydro-1'H-spiro[piperidin-4,4'-pyrano[4,3-c]pyrazole];
- 1-Butyl-6'-methoxy-1'-phenyl-6',7'-dihydro-1'H-spiro[piperidin-4,4'-pyrano[4,3-c]pyrazole];
- 1-isobutyl-6'-methoxy-1'-phenyl-6',7'-dihydro-1'H-spiro[piperidin-4,4'-pyrano[4,3-c]pyrazole];
- 6'-Methoxy-1-pentyl-1'-phenyl-6',7'-dihydro-1'H-spiro[piperidin-4,4'-pyrano[4,3-c]pyrazole];
- 1-Isopentyl-6'-methoxy-1'-phenyl-6',7'-dihydro-1'H-spiro[piperidin-4,4'-pyrano[4,3-c]pyrazole];
- 6'-Methoxy-1-(3-methylbut-2-en-1-yl)-1'-phenyl-6',7'-dihydro-1'H-spiro[piperidin-4,4'-pyrano[4,3-c]pyrazole];
- 6'-Methoxy-1-octyl-1'-phenyl-6',7'-dihydro-1'H-spiro[piperidin-4,4'-pyrano[4,3-c]pyrazole];
- 1-(Cyclohexan-1-ylmethyl)-6'-methoxy-1'-phenyl-6',7'-dihydro-1'H-spiro[piperidin-4,4'-pyrano[4,3-c]pyrazole];
- 6'-Methoxy-1'-phenyl-1-(2-phenylethyl)-6',7'-dihydro-1'H-spiro[piperidin-4,4'-pyrano[4,3-c]pyrazole];
- 6'-Methoxy-1'-phenyl-1-(3-phenylpropyl)-6',7'-dihydro-1'H-spiro[piperidin-4,4'-pyrano[4,3-c]pyrazole];
- 6'-Methoxy-1'-phenyl-1-(4-phenylbutyl)-6',7'-dihydro-1'H-spiro[piperidin-4,4'-pyrano[4,3-c]pyrazole];
- 1-(Furan-2-ylmethyl)-6'-methoxy-1'-phenyl-6',7'-dihydro-1'H-spiro[piperidin-4,4'-pyrano[4,3-c]pyrazole];
- 6'-Methoxy-1-(4-methoxybenzyl)-1'-phenyl-6',7'-dihydro-1'H-spiro[piperidin-4,4'-pyrano[4,3-c]pyrazole];
- 1-(4-Fluorbenzyl)-6'-methoxy-1'-phenyl-6',7'-dihydro-1'H-spiro[piperidin-4,4'-pyrano[4,3-c]pyrazole];
- 1-Benzyl-6'-methoxy-1'-methyl-6',7'-dihydro-1'H-spiro[piperidin-4,4'-pyrano[4,3-c]pyrazole];
- 6'-Methoxy-1'-methyl-6',7'-dihydro-1'H-spiro[piperidin-4,4'-pyrano[4,3-c]pyrazole];
- 6'-Methoxy-1'-methyl-1-(3-phenylpropyl)-6',7'-dihydro-1'H-spiro[piperidin-4,4'-pyrano[4,3-c]pyrazole];
- 1-(4-Fluorbenzyl)-6'-methoxy-1'-methyl-6',7'-dihydro-1'H-spiro[piperidin-4,4'-pyrano[4,3-c]pyrazole];
- 1-Isopentyl-6'-methoxy-1'-methyl-6',7'-dihydro-1'H-spiro[piperidin-4,4'-pyrano[4,3-c]pyrazole];
- 6'-Methoxy-1'-methyl-1-propyl-6',7'-dihydro-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]-6'-ol;
- 1-Benzyl-1'-methyl-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]; or
- 1'-Methyl-6',7'-dihydro-1'H-spiro[piperidin-4,4'-pyrano[4,3-c]pyrazole];
- 1-Benzyl-6'-hydroxy-1'-phenyl-6',7'-dihydro-1'H-spiro[piperidin-4,4'-pyrano[4,3-c]pyrazole];
- 1-Benzyl-1'-phenyl-6',7'-dihydro-1'H-spiro[piperidin-4,4'-pyrano[4,3-c]pyrazole];
- 1'-phenyl-6',7'-dihydro-1'H-spiro[piperidin-4,4'-pyrano[4,3-c]pyrazole];
- 1-isopentyl-1'-phenyl-6',7'-dihydro-1'H-spiro[piperidin-4,4'-pyrano[4,3-c]pyrazole];
- 1-(3-methylbut-2-en-1-yl)-1'-phenyl-6',7'-dihydro-1'H-spiro[piperidin-4,4'-pyrano[4,3-c]pyrazole];
- 1'-phenyl-1-(3-phenylpropyl)-6',7'-dihydro-1'H-spiro[piperidin-4,4'-pyrano[4,3-c]pyrazole];
- 1-isopentyl-1'-methyl-6',7'-dihydro-1'H-spiro[piperidin-4,4'-pyrano[4,3-c]pyrazole];
- 1-(3-methylbut-2-en-1-yl)-1'-methyl-6',7'-dihydro-1'H-spiro[piperidin-4,4'-pyrano[4,3-c]pyrazole];
- 1'-methyl-1-(3-phenylpropyl)-6',7'-dihydro-1'H-spiro[piperidin-4,4'-pyrano[4,3-c]pyrazole];
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
preferably from
- 6'-Methoxy-1'-phenyl-4',6'-dihydro-1'H-spiro[piperidin-4,4'-furo[3,4-c]pyrazole];
- 1-Benzyl-6'-methoxy-1'-phenyl-4',6'-dihydro-1'H-spiro[piperidin-4,4'-furo[3,4-c]pyrazole];
- 1-Butyl-6'-methoxy-1'-phenyl-4',6'-dihydro-1'H-spiro[piperidin-4,4'-furo[3,4-c]pyrazole];
- 1-Benzyl-6'-methoxy-1'-phenyl-6',7'-dihydro-1'H-spiro[piperidin-4,4'-pyrano[4,3-c]pyrazole];
- 6'-Methoxy-1'-phenyl-1-propyl-6',7'-dihydro-1'H-spiro[piperidin-4,4'-pyrano[4,3-c]pyrazole];
- 1-Isopropyl-6'-methoxy-1'-phenyl-6',7'-dihydro-1'H-spiro[piperidin-4,4'-pyrano[4,3-c]pyrazole];
- 1-Butyl-6'-methoxy-1'-phenyl-6',7'-dihydro-1'H-spiro[piperidin-4,4'-pyrano[4,3-c]pyrazole];
- 1-Isobutyl-6'-methoxy-1'-phenyl-6',7'-dihydro-1'H-spiro[piperidin-4,4'-pyrano[4,3-c]pyrazole];
- 6'-Methoxy-1-pentyl-1'-phenyl-6',7'-dihydro-1'H-spiro[piperidin-4,4'-pyrano[4,3-c]pyrazole];
- 1-Isopentyl-6'-methoxy-1'-phenyl-6',7'-dihydro-1'H-spiro[piperidin-4,4'-pyrano[4,3-c]pyrazole];
- 6'-Methoxy-1-(3-methylbut-2-en-1-yl)-1'-phenyl-6',7'-dihydro-1'H-spiro[piperidin-4,4'-pyrano[4,3-c]pyrazole];
- 6'-Methoxy-1-octyl-1'-phenyl-6', 7'-dihydro-1'H-spiro[piperidin-4,4'-pyrano[4, 3-c]pyrazole];
- 1-(Cyclohexan-1-ylmethyl)-6'-methoxy-1'-phenyl-6', 7'-dihydro-1'H-spiro[piperidin-4,4'-pyrano[4,3-c]pyrazole];
- 6'-Methoxy-1'-phenyl-1-(2-phenylethyl)-6',7'-dihydro-1'H-spiro[piperidin-4,4'-pyrano[4,3-c]pyrazole];
- 6'-Methoxy-1'-phenyl-1-(3-phenylpropyl)-6',7'-dihydro-1'H-spiro[piperidin-4,4'-pyrano[4,3-c]pyrazole];
- 6'-Methoxy-1'-phenyl-1-(4-phenylbutyl)-6',7'-dihydro-1'H-spiro[piperidin-4,4'-pyrano[4,3-c]pyrazole];
- 1-(Furan-2-ylmethyl)-6'-methoxy-1'-phenyl-6',7'-dihydro-1'H-spiro[piperidin-4,4'-pyrano[4,3-c]pyrazole];
- 6'-Methoxy-1-(4-methoxybenzyl)-1'-phenyl-6',7'-dihydro-1'H-spiro[piperidin-4,4'-pyrano[4,3-c]pyrazole];
- 1-(4-Fluorbenzyl)-6'-methoxy-1'-phenyl-6',7'-dihydro-1'H-spiro[piperidin-4,4'-pyrano[4,3-c]pyrazole];
- 1-Benzyl-6'-methoxy-1'-methyl-6', 7'-dihydro-1'H-spiro[piperidin-4,4'-pyrano[4,3-c]pyrazole];
- 6'-Methoxy-1'-methyl-6',7'-dihydro-1'H-spiro[piperidin-4,4'-pyrano[4,3-c]pyrazole];
- 6'-Methoxy-1'-methyl-1-(3-phenylpropyl)-6',7'-dihydro-1'H-spiro[piperidin-4,4'-pyrano[4,3-c]pyrazole];
- 1-(4-Fluorbenzyl)-6'-methoxy-1'-methyl-6',7'-dihydro-1'H-spiro[piperidin-4,4'-pyrano[4,3-c]pyrazole];
- 1-Isopentyl-6'-methoxy-1'-methyl-6',7'-dihydro-1'H-spiro[piperidin-4,4'-pyrano[4,3-c]pyrazole];
- 6'-Methoxy-1'-methyl-1-propyl-6',7'-dihydro-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]-6'-ol;
- 1-Benzyl-1'-methyl-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]; or
1'-Methyl-6',7'-dihydro-1'H-spiro[piperidin-4,4'-pyrano[4,3-c]pyrazole];
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

In general the processes are described below in the experimental part. The starting materials are commercially available or can be prepared by conventional methods.

A preferred aspect of the invention is also a process for the production of a compound according to the invention according to general formula I, wherein a compound of formula III 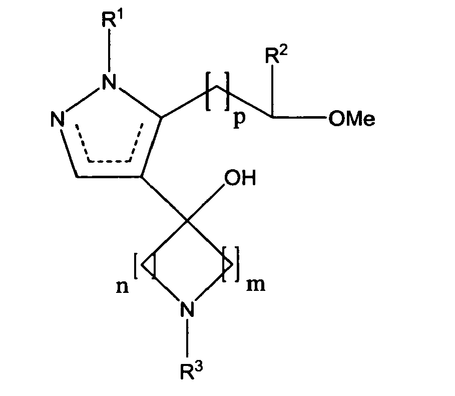 , wherein R¹, R², R³, m, n and p and the dotted line are as defined above is reacted with acid, preferably p-toluol sulphonic acid, to form a compound according the formula I. It is preferred if in this process in the compound according to formula III
- R³ is C(O)OR' or benzyl; and/or
- R² is OCH₃; and/or
- R¹ is C₁₋₆ alkyl or aryl; and/or
- m and n are 2.

A preferred aspect of the invention is also a process for the production of a compound according to the invention according to general formula la, wherein a compound of formula IIIa 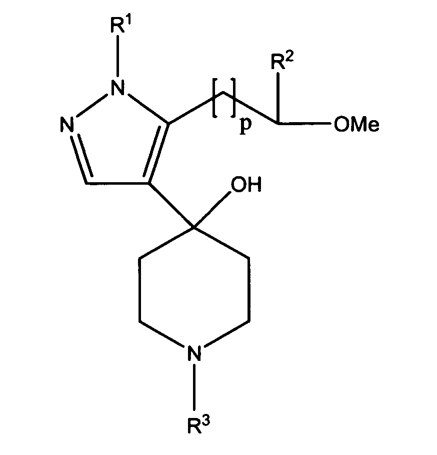 , wherein R¹, R², R³ and p are as defined above is reacted with acid, preferably p-toluol sulphonic acid, to form acompound according the formula Ia. It is preferred if in the compound acccording to general formula IIIa
- R³ is C(O)OR' or benzyl; and/or
- R² is OCH₃; and/or
- R¹ is C₁₋₆ alkyl or aryl.

It is further preferred if in the process above as a next step a compound according to formula I or Ia in which p is 0 and R³ is C(O)OR' is reacted with a KOH solution in water to form a compound according to formulas I or Ia with R³ being H.

It is further preferred if in the process above as a next step a compound according to formula I or Ia in which p is 1 and R³ is benzyl is reacted with NH₄⁺ HCOO⁻ with a Pd/C catalysator to form a compound according to formulas I or Ia with R³ being H.

It is further preferred if in the process above as a next step a compound according to formula I or Ia in which R³ is hydrogen is reacted with a compound R³-X with X being a leaving group and K₂CO₃ under reflux to form a compound according to formulas I or Ia with R³ being as defined above, except H.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

Another aspect of the invention refers to a pharmaceutical composition which comprises a compound according to the invention or a pharmaceutically acceptable salt, prodrug, isomer or solvate thereof, and a pharmaceutically acceptable carrier, adjuvant or vehicle. The present invention thus provides pharmaceutical compositions comprising a compound of this invention, or a pharmaceutically acceptable salt, derivative, prodrug or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to the use of a compound according to the invention in the manufacture of a medicament.

Another aspect of the invention refers to the use of a compound according to the invention in the manufacture of a medicament for the treatment or prophylaxis of a sigma receptor mediated disease or condition. A preferred embodiment of this is this use wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

A preferred embodiment of this is this use wherein the disease is pain, especially neuropathic pain, inflammatory pain or other pain conditions, allodynia and/or hyperalgesia, especially mechanical allodynia.
Another aspect of the invention refers to the use of a compound according o the invention as pharmacological tool or as anxiolytic or immunosuppressant.

The term "pharmacological tool" refers to the property of compounds of the invention through which they are particularly selective ligands for Sigma receptors which implies that compound of formula I, described in this invention, can be used as a model for testing other compounds as Sigma ligands, ex. a radiactive ligands being replaced, and can also be used for modeling physiological actions related to Sigma receptors.

Another aspect of this invention relates to a method of treating or preventing a sigma receptor mediated disease which method comprises administering to a patient in need of such a treatment a therapeutically effective amount of a compound as above defined or a pharmaceutical composition thereof. Among the sigma mediated diseases that can be treated are diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, pain, especially neuropathic pain, inflammatory pain or other pain conditions, allodynia and/or hyperalgesia, especially mechanical allodynia, psychotic condition, schizophrenia; inflammation, autoimmune diseases or cancer; disorders of food ingestion, the regulation of appetite, for the reduction, increase or maintenance of body weight, for the prophylaxis and/or treatment of obesity, bulimia, anorexia, cachexia or type II diabetes, preferably type II diabetes caused by obesity. The compounds of the invention can also be employed as pharmacological tool or as anxiolytic or immunosuppressant.

The compounds of the invention may be synthesized following one of the three Reaction Schemes (A, B, C or D) set out below, wherein R is equivalent to R³:

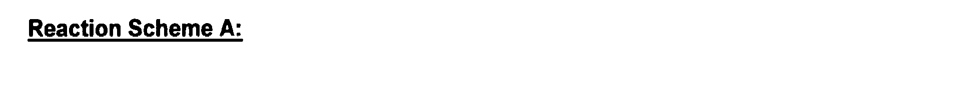

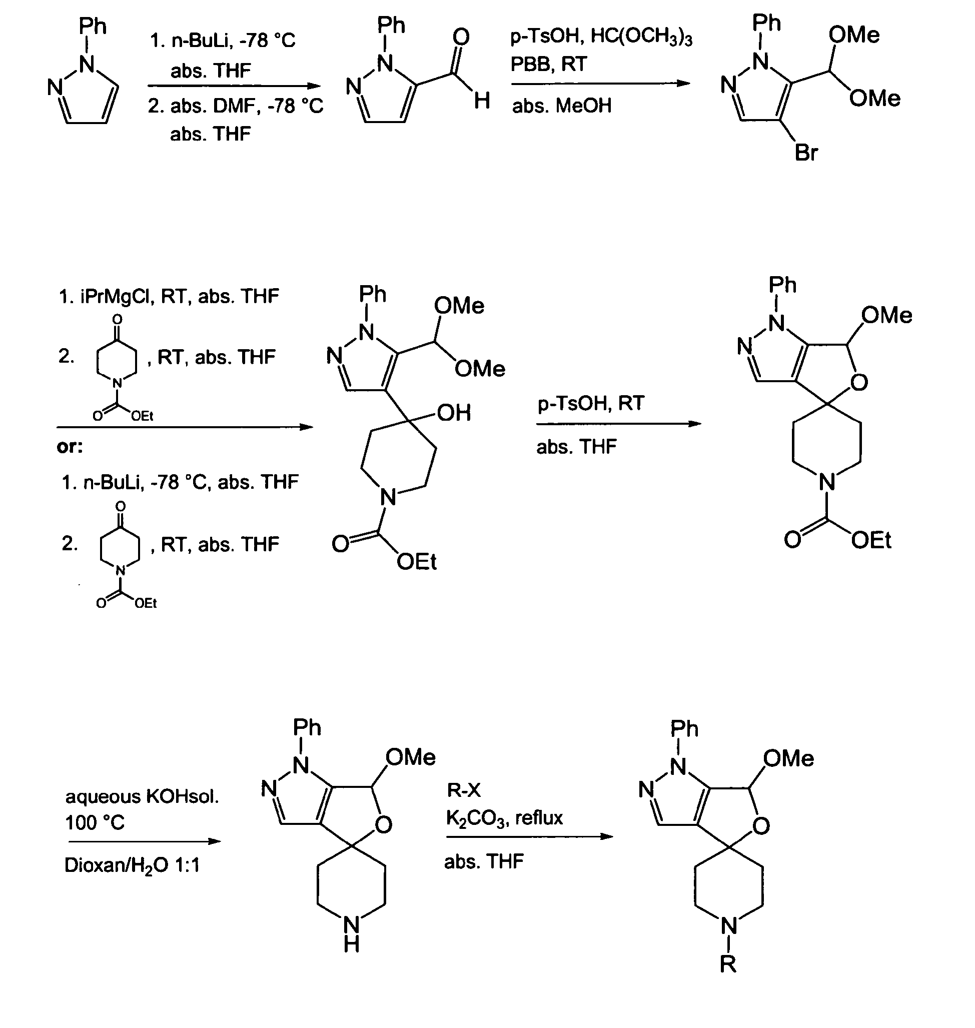

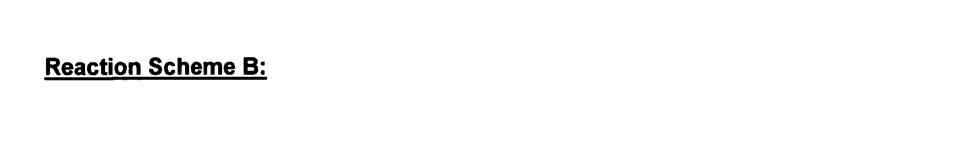

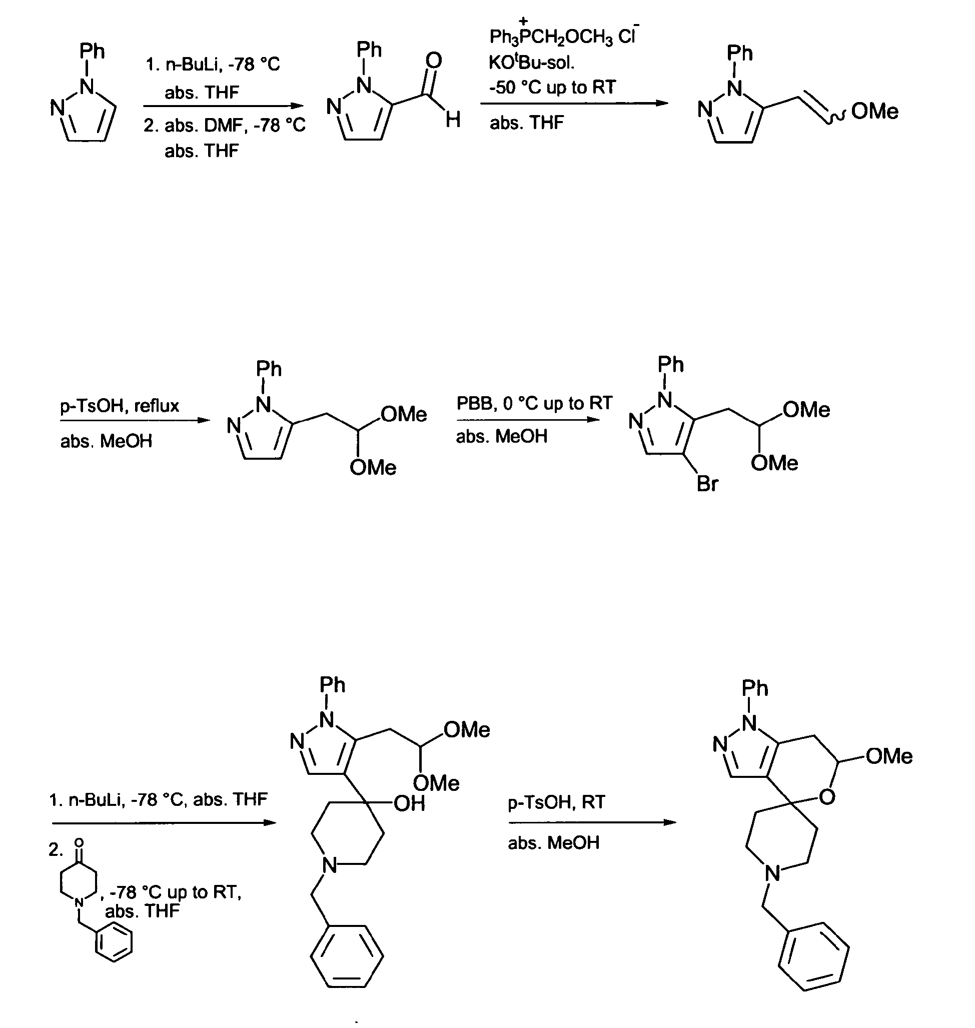

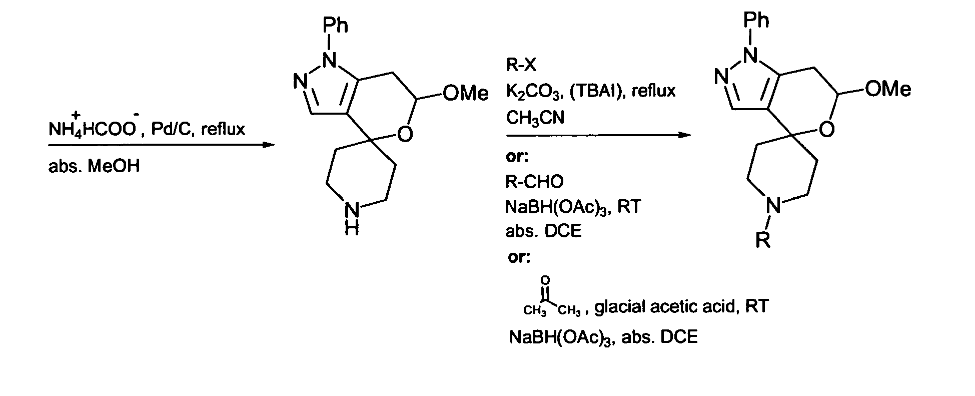

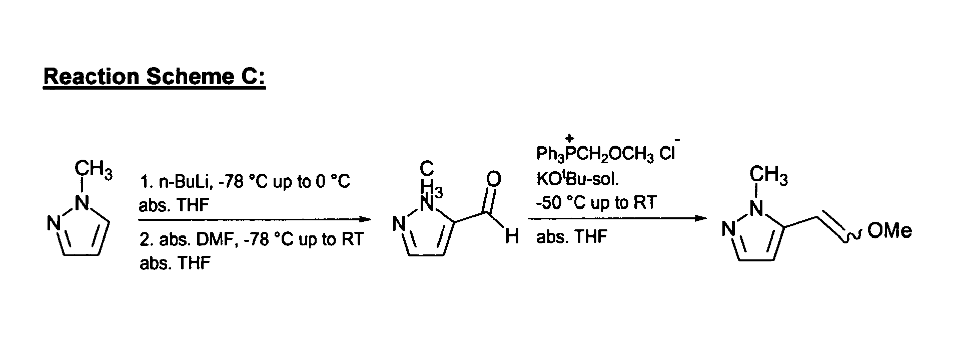

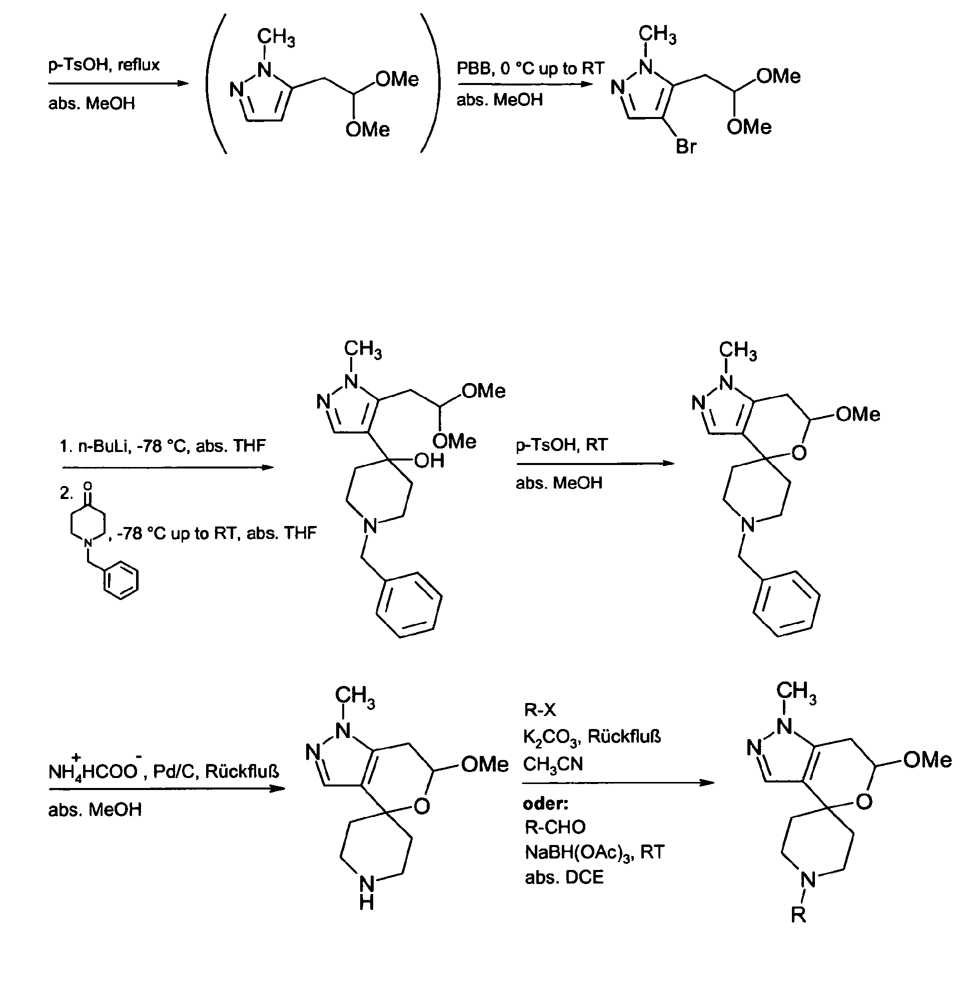

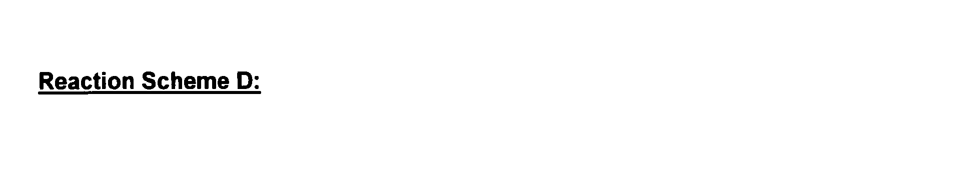

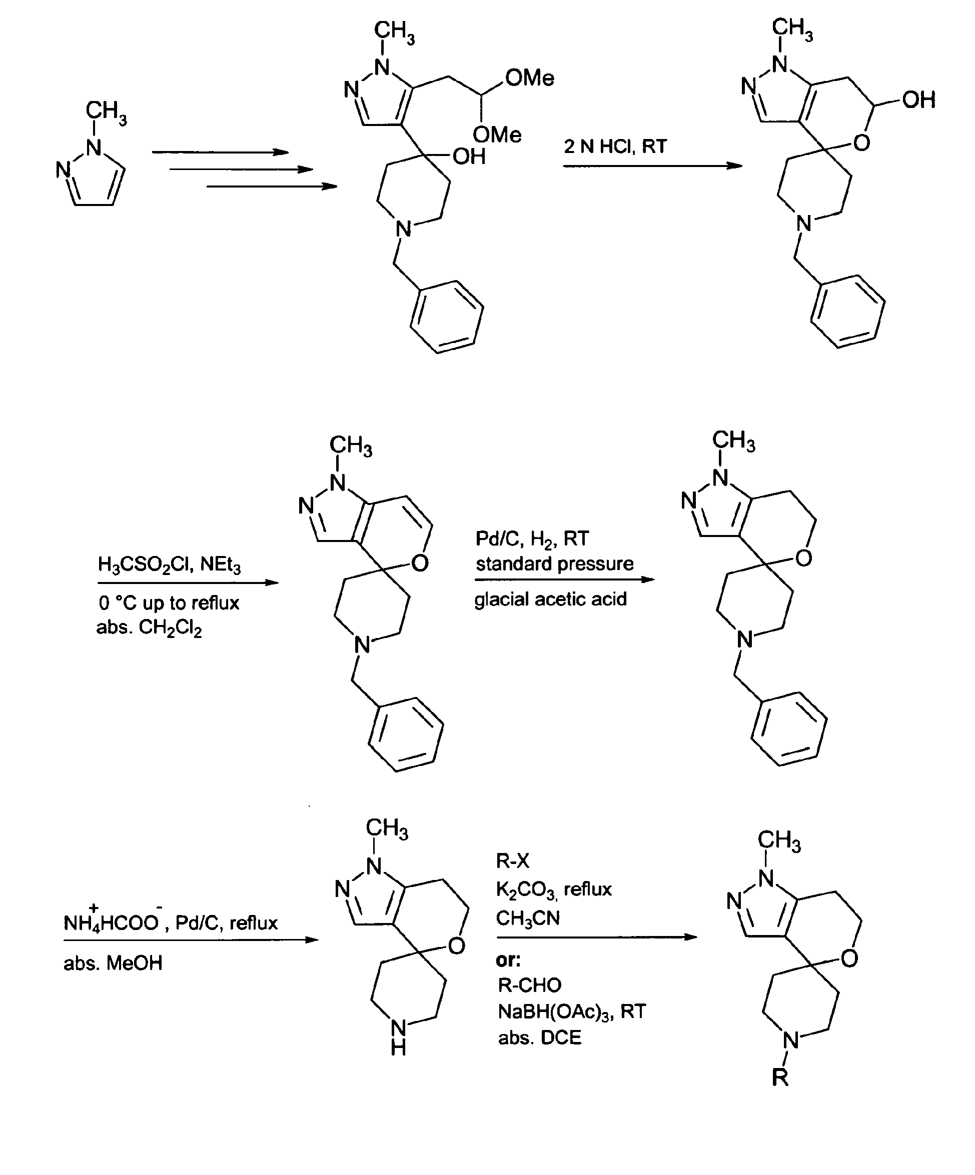

The present invention is illustrated below with the aid of examples. These illustrations are given solely by way of example and do not limit the general spirit of the present invention.

### EXAMPLES:

### General Experimental Part (Methods and Equipment of the synthesis and analysis

All solvents used for synthesis were p. a. quality.

The separation of mixtures of substances using thin-layer chromatography was done on glass plates layered by silica gel. Analysis of the plates was done after treatment with iodine gas or incubation with Dragendorff's reagent under UV light.

As a rule synthesized products were purified using flash-chromatography.

Melting points were measured using capillaries. As sometimes the products were mixtures of diastereoisomers, themelting point: had to be expressed as a range.

IR-spectra were measured using the FT-IR-480 Plus Fourier Transform Spectrometer with ATR (Fa. Jasco). All substances were either measured directly as solids or in oil.

NMR-Spectra were measured using Mercury-400BB (Fa. Varian) at a temperature of 21 °C. δ, measured in ppm, is based on the signal TMS measured in comparison to the residue signal (CHCl₃) of the solvent (CDCl₃):
- ¹H-NMR-Spectroscopy:: δ (TMS) = δ (CHCl₃) - 7.26
- ¹³C-NMR-Spectroscopy:: δ (TMS) = δ (CHCl₃) - 77.0

Mass-spectra (MS) were measured using the GCQ Finnigan MAT (Fa. Finnigan) with Xcalibur Version 1.1. The method of ionisation is shown in brackets: El = electronic ionisation (70 eV); CI = Chemical ionisation (Isobutane or NH₃, 170 eV).

Elementary analysis was done with VarioEL (Fa. Elementar).

Before using HPLC the maximum absorption of the substances was measured using the UV/Vis-Spectra done with a 50Bio Cary Spektrophotometer (Fa. Varian).

For determination of the purity and for the separation of the diastereomers a HPLC Hitachi L6200A Intelligent Pump with a UV-Detector (Merck) was used.

Synthesis was done in the synthetic microwave Discover (Fa. CEM).

Some reactions were done using protective gas.

Reactions at -78°C were done in an acetone bath in a Dewar.

### Example A: 1-Phenylpyrazole-5-carbaldehyd

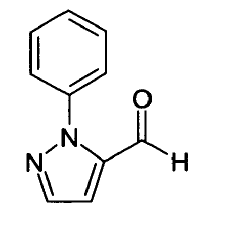

### Experimental procedure:

Under N₂-atmosphere a solution of n-butyllithium in heptane (2.61 M, 5.3 mL, 13.9 mmol) is slowly added to a cooled (- 78 °C) solution of 1-phenyl-1H-pyrazole (2.0 g, 13.9 mmol) in abs. THF (60 mL). The reaction mixture was stirred for 2 h at -78 °C. Subsequently a Solution of abs. DMF (1.1 mL, 13.9 mmol) in abs. THF (8 mL) is slowly added at -78 °C. This mixture was stirred for 1 h at -78 °C, before it was slowly heated to room temperature and was stirred for a further 18 h. Then it was hydrolysed with water (10 mL) and subsequently extracted three times with CH₂Cl₂. The organic phases were dried (Na₂SO₄), filtered and the solvent was removed under vacuum. The crude product (2.48 g) was purified using flash-chromatography (Ø= 6 cm, h = 12 cm, n-hexane:ethylacetate = 8:2, 30 mL, R_{f} = 0.24).

Yellow Oil, that crystalized in the cold to a yellow solid, melting point: 31 °C,

Yield: 2.15 g (90%)

C₁₀H₈N₂O (172.2)

**MS** (EI): m/z (rel.Int) = 172 [M⁺, 100], 144 [M - CO, 69].

**IR** (neat): ν̃ (cm⁻¹) = 3063 (C-H _{aromat.}), 2923 (C-H _{aliphat.}), 2854 (C-H), 1683 (C=O), 1596, 1517, 1499 (C=C), 763, 694 (C-H).

**¹H-NMR** (CDCl₃): δ (ppm) = 7.11 (d, J = 2.1 Hz, 1H, Pyrazole-4-C*H*), 7.46 - 7.56 (m, 5H, Phenyl-C*H*), 7.76 (d, J = 2.0 Hz, 1H, Pyrazole-3-C*H*), 9.88 (s, 1 H, C*H*O).

**¹³C-NMR** (CDCl₃): δ (ppm) = 112.5 (1 C, Pyrazole-4-CH), 125.8 (2 C, Phenyl-*C*H, ortho), 129.4 (1 C, Phenyl-CH, para), 129.6 (2 C, Phenyl-CH, meta), 139.0 (1 C, Phenyl-*C*, quartär), 140.3 (1 C, Pyrazole-5-*C*), 140.7 (1 C, Pyrazole-3-*C*H), 180.2 (1 C, *C*HO).

### Example B: 4-Brom-1-phenylpyrazole-5-carbaldehyd-dimethylacetal

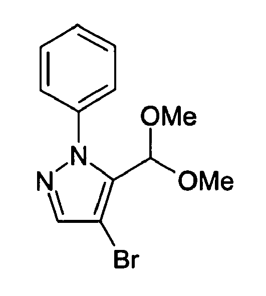

### Experimental procedure:

The aldehyde (B) (2.30 g, 13.4 mmol), trimethylortoformiate (4.4 mL, 40.1 mmol) and p-toluolsulfonic acid monohydrate (127.1 mg, 0.67 mmol) were dissolved in abs. methanol (260 mL). After slow addition of pyridiniumbromide-perbromide (PBB) (4.3 g, 13.4 mmol) the solution was stirred for 90 h at room temperature. Subsequently a saturated NaHCO₃-Solution (25 mL) was added, diluted with water and 3x extracted with CH₂Cl₂. The organic phases were dried (K₂CO₃) and the solvent removed under vacuum. The crude product (4.08 g) was purified using flash-chromatography (Ø=8 cm, h = 12 cm, n-hexane:ethylacetate = 9:1, 40 mL, R_{f} = 0.20).

Colourless solid, melting point: 33 °C, yield: 3.67 g (93%)
C₁₂H₁₃BrN₂O₂ (297.2)

| | C | H | N |
|---|---|---|---|
| Calc. | 48.5 | 4.41 | 9.43 |
| Found | 48.5 | 4.17 | 9.32 |

**MS** (EI): m/z (rel. Int.) = 298 [⁸²Br-M⁺, 28], 296 [⁷⁹Br-M⁺, 25], 267 [⁸²Br-M- OCH₃, 93], 265 [⁷⁹Br-M - OCH₃, 100], 235 [⁸²Br-M- 2x OCH₃, 49], 233 [⁷⁹Br-M - 2x OCH₃, 47].
**IR** (neat): ν̃ (cm⁻¹) = 3062 (C _{aromat.}), 2933 (C-H _{aliphat.}), 2830 (C-H), 1597, 1501 (C=C), 1090, 1065 (C-O), 761, 692 (C-H).
**¹H-NMR** (CDCl₃): δ (ppm) = 3.37 (s, 6 H, ArCH(OC*H₃)₂*), 5.36 (s, 1 H, ArC*H*(OCH₃)₂), 7.39 - 7.49 (m, 3 H, Phenyl-C*H*), 7.53 - 7.59 (m, 2 H, Phenyl-C*H*, ortho), 7.63 (s, 1 H, Pyrazole-3C*H*).
**¹³C-NMR** (CDCl₃): δ (ppm) = 54.7 (2 C, ArCH(OCH₃)₂), 95.9 (1 C, Pyrazole-4-*C*), 98.5 (1 C, Ar*C*H(OCH₃)₂), 125.6 (2 C, Phenyl-*C*H, ortho), 128.7 (1 C, Phenyl-*C*H, para), 129.1 (2 C, Phenyl-*C*H, meta), 136.3 (1 C, Phenyl-*C*, quartär), 140.1 (1 C, Pyrazole-5-*C*), 141.2 (1 C, Pyrazole-3-*C*H).

### Example C: Ethyl-4-(5-dimethoxymethyl -1-phenylpyrazole-4-yl)-4-hydroxy-piperidin-1-carboxylat

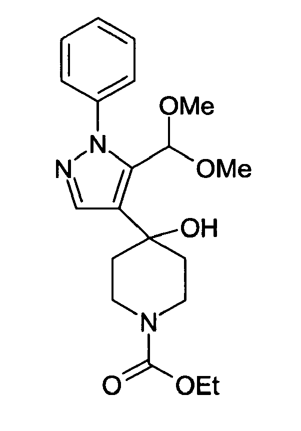

### Experimental procedure:

### Method 1:

To a solution of the bromated acetal (B) (1.50 g, 5.05 mmol) in abs. THF (100 mL) it was added under N₂-atmosphere isopropyl magnesiumchloride in THF (2 M, 2.8 mL, 5.60 mmol). The reaction mixture was stiired for 90 h at room temperature. Subsequently a solution of 4-Oxo-piperidine-1-carboxylic acid ethyl ester (1.12 g, 6.56 mmol) in abs. THF (65 mL) was added dropwise. After 24 h of stirring at room temperature water was added until no further precipitate was developing. The precipitate was filtered off and the mixture was extracted 3x with CH₂Cl₂. The organic phases were dried over K₂CO₃ filtered and reduced in vacuum. The crude product (2.35 g) was purified using flash-chromatography (Ø=8 cm, h = 15 cm, Cyclohexan:Ethylacetat = 1:1, 40 mL, R_{f} = 0,13).
Colourless Oil, Yield 623.4 mg (32%)

### Method 2:

Under N₂-atmosphäre n-butyllithium in heptane (2.61 M, 130.0 µL, 0.34 mmol) was slowly added at -78 °C to a Solution of the bromated acetals B (100 mg, 0.34 mmol) in abs THF (7 mL). The reaction mixture was stirred for 15 minutes. Subsequently a solution of 4-Oxo-piperidine-1-carboxylic acid ethyl ester (58.21 mg, 0.34 mmol) in abs. THF (7 mL) was added dropwise at -78 °C. After 2 h of stiring at -78 °C the mixture was heated to room temperature and immediately mixed with water until no further precipitate is formed. Following extraction with CH₂Cl₂ the organic phases were dried over K₂CO₃ and filtered. The solvent was removed under vacuum and the crude product (0.14 g) was purified using flash-chromatography (Ø= 3 cm, h = 15 cm, n-Hexane:Ethylacetate = 3:7, 20 mL, R_{f} = 0.23).
Colourless Oil, Yield 0.030 g (23%)
C₂₀H₂₇N₃O₅ (389.5)
**MS** (EI): m/z (rel. Int.) = 358 [M - OCH₃, 44], 343 [M - CH₃CH₂OH, 55], 326 [M - 2x OCH₃, 100].
**IR** (neat): ν̃ (cm⁻¹) = 3448 (O-H), 2980, 2933 (C-H _{aliphat.}), 1693 (C=O), 1598, 1545, 1500 (C=C), 1093, 1072 (C-O), 765, 696 (C-H).
**¹H-NMR** (DMSO-d₆): δ (ppm) = 1.17 (t, J = 7.1 Hz, 3H, OCH₂C*H*₃), 1.77 (br d, J = 12.5 Hz, 2H, N(CH₂C*H*_{*2 eq*.})₂), 1.88 (td, J = 12.9/4.5 Hz, 2H, N(CH₂C*H*_{2 *ax*.})₂), 3.10 - 3.21 (m, 2H, N(C*H*₂CH₂)₂)*, 3.20 (s, 6H, ArCH(OC*H*₃)₂), 3.74 - 3.87 (m, 2H, N(C*H*₂CH₂)₂), 4.02 (q, J = 7.1 Hz, 2H, OC*H*₂CH₃), 5.09 (s, 1H, ArC*H*(OCH₃)₂), 7.37 (tt, J = 7.2/1.8 Hz, 1H, Phenyl-C*H*, para), 7.40 - 7.45 (m, 2H, Phenyl-C*H*, meta), 7.48 - 7.52 (m, 2H, Phenyl-C*H*, ortho), 7.58 (s, 1H, Pyrazole-3-C*H*).
* The both protons of the piperidine rings neighbouring the nitrogen are overlaid by the signal of the methoxy group. They became visible producing an NMR-spectrum at 50°C.

### Example 1: Ethyl-6'-methoxy-1'-phenyl-4',6'-dihydro-1'H-spirolpiperidin-4,4'-furo[3,4-c]pyrazolel-1-carboxylate

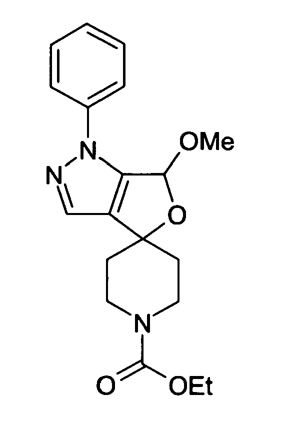

### Experimental procedure:

Under N₂-atmosphere a solution of 0.1 M p-toluolsulphonsic acid monohydrate (900µL, 0.09 mmol) predried over Na₂SO₄ at room temperature was added to a solution example C (704.4 mg, 1.81 mmol) in abs. THF (36 mL). Subsequently it was stirred for 24 h at room temperature. Following the addition of 0.1 M NaOH- solution up to reaching pH 10 it was repeatedly extracted with CH₂Cl₂. Subsequently the organic phases were dried over K₂CO₃, filtered and the Solvent removed in vacuum. The crude product (0.700 g) was purified using flash-chromatography (Ø= 5 cm, h = 15 cm, n-Hexane:Ethylacetate = 1:1, 30 mL, R_{f} = 0.43).
Colourless Oil, Yield 233 mg (36%)
C₁₉H₂₃N₃O₄ (357.5)
**MS** (EI): m/z (rel. Int) = 357 [M⁺, 61], 342 [M - CH₃, 70], 325 [M - HOCH₃, 100], 297 [M - OCH₃ - CH₃CH₂, 41].
**IR** (neat): ν̃ (cm⁻¹) = 2954, 2930 (C-H _{aliphat.}), 2829 (C-H), 1692 (C=O), 1600, 1566, 1514 (C=C), 1098, 1083 (C-O), 756, 711 (C-H).
**¹H-NMR** (DMSO-d₆): δ (ppm) = 1.18 (t, J = 7.1 Hz, 3H, OCH₂C*H*₃), 1.66 - 1.90 (m, 4H, N(CH₂C*H₂*)₂), 3.34 (s, 3H, OC*H₃*), 3.43 - 3.65 (m, 4H, N(C*H*₂CH₂)₂), 4.05 (q, J = 7.1 Hz, 2H, OC*H*₂CH₃), 6.38 (s, 1 H, Ar-C*H*), 7.31 (t, J = 7.1 Hz, 1H, Phenyl-C*H*, para), 7.46 - 7.53 (m, 2H, Phenyl-C*H*, meta), 7.66 - 7.71 (m, 2H, Phenyl-C*H*, ortho), 7.79 (s, 1H, Pyrazole-3-C*H*).

### Example 2: 6'-Methoxy-1'-phenyl-4',6'-dihydro-1'H-spiro[piperidin-4,4'-furo[3,4-c]pyrazole]

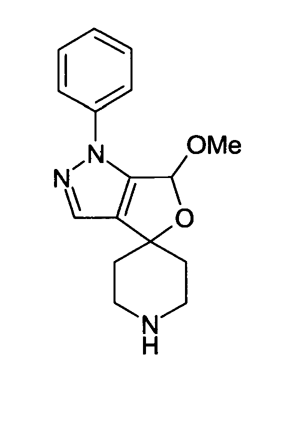

### Experimental procedure:

To a solution of example 1 (343 mg, 0.96 mmol) in dioxane/H₂O 1:1 (96 mL) an aqueous KOH-solution (2 M, 30 mL) was added. The solution was heated for 18 h at 100 °C. After diluting with water, it was extracted with ethylacetate, dried over K₂CO₃, filtered and subsequently the solvent was removed in vacuum. The crude product (298 mg) was purified using flash-chromatography (Ø= 4 cm, h = 15 cm, Methanol + 2% NH_{3 conc.,} 30 mL, R_{f} = 0.21) and subsequently nach after removing te solvent up to dryness and another dissolution in ethylacetate was filtered through a membrane filter. Further reduction resulted in a slightly yellow solid.
Slightly yellow solid, melting point: 81 °C, Yield 210 mg (77%)
C₁₆H₁₉N₃O₂ (285.4)

| | C | H | N |
|---|---|---|---|
| Calc. | 67.4 | 6.71 | 14.7 |
| Found | 67.1 | 6.73 | 14.5 |

**MS** (EI): m/z (rel. Int) = 285 [M⁺, 60], 270 [M - CH₃, 100], 77 [Phenyl, 10].
**IR** (neat): ν̃ (cm⁻¹) = 3309 (N-H), 3063 (C-H _{aromat.}), 2942 (C-H _{aliphat.}), 2826 (C-H), 1599, 1563, 1512 (C=C), 1080, 1061 (C-O), 755, 710 (C-H).
**¹H-NMR** (DMSO-d₆): δ (ppm) = 1.65 - 1.77 (m, 4H, N(CH₂C*H*₂)₂), 2.67 - 2.78 (m, 2H, N(C*H*₂CH₂)₂), 2.87 - 2.97 (m, 2H, N(C*H*₂CH₂)₂), 3.34 (s, 3H, OC*H*₃), 6.35 (s, 1H, ArC*H*), 7.30 (tt, J = 7.4/1.2 Hz, 1H, Phenyl-C*H*, para), 7.45 - 7.52 (m, 2H, Phenyl-C*H*, meta), 7.66 - 7.71 (m, 2H, Phenyl-C*H*, ortho), 7.73 (s, 1 H, Pyrazole-3-C*H*).

The Signal of the NH-protons was not detectable.

### Example 3: 1-Benzyl-6'-methoxy-1'-phenyl-4',6'-dihydro-1'H-spiro[piperidin-4,4'-furo[3,4-c]pyrazole]

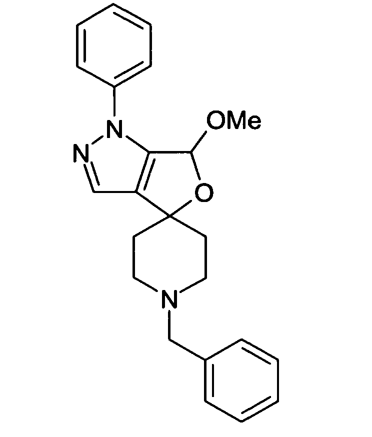

### Experimental procedure:

Benzyl bromide (29.9 µL, 0.25 mmol) and K₂CO₃ (232.2 mg, 1.68 mmol) were added to a solution of example 2 (60.0 mg, 0.21 mmol) in abs. THF (7 mL). This mixture was heated under reflux for 24 h. Then it was filtered and the Solvent removed under vacuum. The crude product (99.7 mg) was purified using flash-chromatography (Ø= 3 cm, h = 15 cm, n-Hexane:Ethylacetate = 1:1, 20 mL, R_{f}= 0.20).
Yellow resin, Yield 56 mg (71%)
C₂₃H₂₅N₃O₂ (375.5)

| | C | H | N |
|---|---|---|---|
| Calc. | 73.6 | 6.71 | 11.2 |
| Found | 73.6 | 6.82 | 11.1 |

**MS** (EI): m/z (rel. Int) = 375 [M⁺, 60], 360 [M - CH₃, 100], 344 [M - OCH₃, 17], 284 [M - Benzyl, 17], 91 [Benzyl, 93].
**IR** (neat): ν̃ (cm⁻¹) = 3061 (C-H _{aromat.}), 2923 (C-H _{aliphat.}), 2804 (C-H), 1600, 1565, 1512 (C=C), 1080, 1061 (C-O), 755, 738 (C-H).
**¹H-NMR** (CDCl₃): δ (ppm) = 1.86 - 2.12 (m, 4H, N(CH₂C*H₂*)*₂*), 2.49 - 2.80 (m, 4H, N(C*H*₂CH₂)₂), 3.46 (s, 3H, OC*H*₃), 3.62 (s, 2H, NC*H*₂Ph), 6.16 (s, 1 H, ArC*H*), 7.24 - 7.46 (m, 9H, aromat. C*H*), 7,73 (dd, J = 8.4/0.8 Hz, 2H, Phenyl-C*H*, ortho).

### Example 4: 1-Butyl-6'-methoxy-1'-phenyl-4',6'-dihydro-1'H-spiro[piperidin-4,4'-furo[3,4-c]pyrazole]

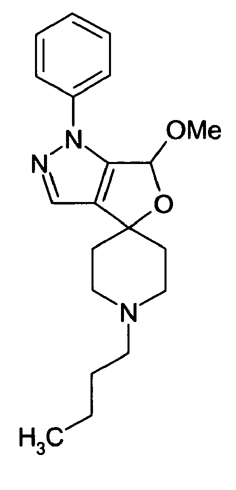

### Experimental procedure:

Butyl-bromide (48.4 µL, 0.45 mmol) and K₂CO₃ (410.5 mg, 2.97 mmol) were added to a solution example 3 (106.0 mg, 0.37 mmol) in abs. THF (12 mL). This mixture was heated under reflux for 49 h. Then it was filtered and the solvent removed under vacuum. The crude product (133.3 mg) was purified using flash-chromatography (Ø = 3 cm, h = 18 cm, Ethylacetat + 10% Methanol, 20 mL, R_{f} = 0.18).
Yellow Oil, Yield 74 mg (59%)
C₂₀H₂₇N₃O₂ (341.5)

| | C | H | N |
|---|---|---|---|
| Calc. | 70.4 | 7.97 | 12.3 |
| Found | 70.3 | 8.01 | 12.2 |

**MS** (EI): m/z (rel. Int) = 341 [M⁺, 5], 298 [M - Propyl, 100].
**IR** (neat): ν̃ (cm⁻¹) = 3064 (C-H _{aromat.}), 2927 (C-H _{aliphat.}), 2804 (C-H), 1601, 1564, 1513 (C=C), 1086, 1062 (C-O), 754, 712 (C-H).
**¹H-NMR** (CDCl₃): δ (ppm) = 0.94 (t, J = 7.3 Hz, 3H, NCH₂CH₂CH₂C*H*₃), 1.30 - 1.41 (m, 2H, NCH₂CH₂C*H*₂CH₃), 1.48 - 1.60 (m, 2H, NCH₂C*H*₂CH₂CH₃), 1.90 - 2.12 (m, 4H, N(CH₂C*H*₂)₂), 2.38 - 2.50 (m, 2H, NC*H*₂CH₂CH₂CH₃), 2.51 - 2.81 (m, 4H, N(C*H*₂CH₂)₂), 3.46 (s, 3H, OC*H*₃)), 6.16 (s, 1H, ArC*H*), 7.25 (t, J = 7.4 Hz, 1H, Phenyl-C*H*, para), 7.41 - 7.44 (m, 2H, Phenyl-C*H*, meta), 7.46 (s, 1H, Pyrazole-3-C*H*), 7.74 (d, J = 7.7 Hz, 2H, Phenyl-C*H*, ortho).

### Example D: 5-(2-Methoxyvinyl)-1-phenylpyrazole

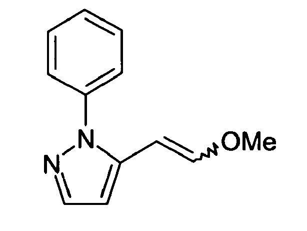

### Experimental procedure:

Under N₂-atmosphere dried MeOCH₂P⁺Ph₃Cl⁻ (6.22 g, 18.1 mmol) was weighed out and suspended in abs. THF (60 mL) for 30 minutes. Subsequently the suspension was cooled to -50 °C and following that KO^{t}Bu-Solution in THF (1M, 16.5 mL, 16.5 mmol) was slowly added dropwise. After stirrig for another 15 minutes, the aldehyd of example A (1.42 g, 8.3 mmol) was dissolved in abs. THF (40 mL) and added dropwise at -50 °C. The reaction mixture was slowly heated to room temperature over night while sttirring. After adittion of water (∼ 25 mL) it was extracted 3-times using ethylacetatet. The organic phases were dried over K₂CO₃, filtered and the solvent removed under vacuum. The crude product (7.59 g) was purified using flash-chromatography (Ø= 8 cm, h = 12 cm, n-hexane:ethylacetate = 9:1, 40 mL, R_{f} = 0.10).
Colourless Oil, Yield 1.53 g (92%)
C₁₂H₁₂N₂O (200.3)
**MS** (EI): m/z (rel. Int) = 200 [M⁺, 93], 169 [M - OCH₃, 100].
**IR** (neat): ν̃ (cm⁻¹) = 3098 (C-H _{aromat.}), 2935 (C-H _{aliphat.}), 1644 (C=C _{Alken}), 1597, 1529 (C=C _{aromat.}), 1093 (C-O), 761, 696 (C-H).
**¹H-NMR** (DMSO-D₆): δ (ppm) = 3.56 (s, 3H, OC*H*₃, trans-Isomer), 3.77 (s, 3H, OC*H₃*, cis-Isomer), 5.18 (d, J = 6.8 Hz, 1 H, C*H*=CHOCH₃, cis-Isomer), 5.59 (d, J = 12.8 Hz, 1H, C*H*=CHOCH₃, trans-Isomer), 6.37 (d, J = 6.8 Hz, 1H, CH=C*H*OCH₃, cis-Isomer), 6.47 (d, J = 1,6 Hz, 1H, Pyrazole-4-C*H*, trans-Isomer), 6,63 (d, J = 1.6 Hz, 1H, Pyrazole-4-C*H*, cis-Isomer), 7.26 (d, J = 12.8 Hz, 1 H, CH=C*H*OCH₃, trans-isomer), 7.39 - 7.54 (m, 10H, aromat. H, cis-Isomer (5H), trans-Isomer (5H)), 7.55 (d, J = 1.6 Hz, 1H, Pyrazole-3-C*H*, trans-Isomer), 7.58 (d, J = 1.2 Hz, 1H, Pyrazole-3-C*H*, cis-Isomer).

The ratio of the isomers cis/trans is 1:3.

### Example E: 2-(1-Phenylpyrazole-5-yl)acetaldehyddimethylacetal (11)

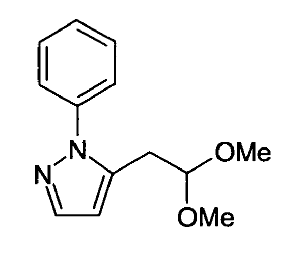

### Experimental procedure:

Example D (1.0 g, 5.0 mmol) was dissolved in abs. methanol (80 mL). Subsequently p-Toluol sulphonic acid monohydrate (475 mg, 2.5 mmol) was added and heated for 72 h under reflux. After addition of a saturated solution of NaHCO₃ up to approximately pH 10, it was extracted 3-times with CH₂Cl₂. The pooled organic phases were dried over K₂CO₃, and filtered. Folowing removal of the solvent under vacuum the Crude product (1.15 g) was pruried using flash chromatography. (Ø= 6 cm, h = 15, n-hexane:ethylacetate = 8:2, 40 mL, R_{f} = 0.12).
Colourless Oil, Yield 1.04 g (90%)
C₁₃H₁₆N₂O₂ (232.3)
**MS** (EI): m/z (rel. Int) = 233 [MH⁺, 10], 201 [M - OCH₃, 9], 171 [MH - 2x OCH₃, 58], 169 [M - OCH₃ - HOCH₃, 100].
**IR** (neat): ν̃ (cm⁻¹) = 3064 (C-H _{aromat.}), 2935 (C-H _{aliphat.}), 1599, 1535, 1500 (C=C), 1120, 1063 (C-O), 766, 696 (C-H).
**¹H-NMR** (DMSO-D₆): δ (ppm) = 2.92 (d, J = 5.9 Hz, 2H, C*H*₂CH(OCH₃)₂), 3.39 (s, 6H, CH₂CH(OC*H*₃)₂), 4.55 (t, J = 5.5 Hz, 1H, CH₂C*H*(OCH₃)₂), 6.39 (d, J = 1.6 Hz, 1H, Pyrazole-4-C*H*), 7.40 - 7.55 (m, 5H, Phenyl-C*H*), 7.59 (d, J = 1.6 Hz, 1H, Pyrazole-3-C*H*).
**¹³C-NMR** (DMSO-D₆): δ (ppm) = 30.5 (1C, *C*H₂CH(OCH₃)₂), 53.8 (2C, CH₂CH(OCH₃)₂), 103.6 (1C,CH₂*C*H(OCH₃)₂), 107.3 (1C, Pyrazole-4-CH), 126.0 (2C, Phenyl-*C*H, ortho), 128.6 (1C, Phenyl-*C*H, para), 129.9 (2C, Phenyl-*C*H, meta), 139.1 (1C, Phenyl-*C*, quartär), 140.2 (1C, Pyrazole-5-*C*H), 140.3 (1C, Pyrazole-3-*C*H).

### Example F: 2-(4-Brom-1-phenylpyrazole-5-yl)acetaldehyddimethylacetal

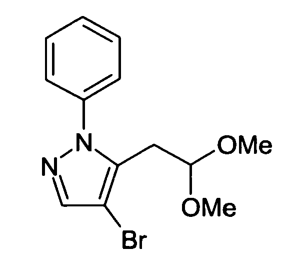

### Experimental procedure:

To a solution of example E (9.9 g, 42.8 mmol) in abs. methanol (500 mL) were added trimethyl-orthoformiate (7.0 mL, 64.3 mmol) and - cooling in ice at 0 °C - in batches Pyridinium bromide perbromide (PBB) (13.7 g, 42.8 mmol). The reaction mixture was stirred for 1 h at 0 °C, subsequently heated slowly to room temperature and then stirred for a further 4 h. After addition of a saturated solution of NaHCO₃ up to reaching approximately pH 10 and diluting with water, it was extracted 3 times with CH₂Cl₂. The organic phases were dried (K₂CO₃), filtered and the solvent was removed under vacuum. The crude product (13.3 g) was purified using flash-chromatography (Ø = 8 cm, h = 18 cm, n-hexane:ethylacetate = 8:2, 40 mL, R_{f} = 0.30).
Colourless Oil, Yield 12.7 g (95%)
C₁₃H₁₅BrN₂O₂ (311.2)

| | C | H | N |
|---|---|---|---|
| Calc. | 50.2 | 4.86 | 9.00 |
| Found | 50.2 | 4.93 | 9.02 |

**MS** (EI): m/z (rel. Int.) = 313 [⁸¹Br-MH⁺, 7], 311 [⁷⁹Br-MH⁺, 8], 281 [⁸¹Br-M - OCH₃, 9], 279 [⁷⁹Br-M - OCH₃, 12], 249 [⁸¹Br-MH - 2x -HOCH₃, 40], 247 [⁷⁹Br-M- 2x -HOCH₃, 36], 168 [M - Br - OCH₃ - HOCH₃, 100].
**IR** (neat): ν̃ (cm⁻¹) = 3065 (C-H _{aromat.}), 2933 (C-H _{aliphat.}), 1598, 1500 (C=C), 1119, 1072 (C-O), 766, 695 (C-H).
**¹H-NMR** (DMSO-D₆): δ (ppm) = 2.95 (d, J = 5.9 Hz, 2H, C*H*₂CH(OCH₃)₂), 3.10 (s, 6H, CH₂CH(OC*H*₃)₂), 4.40 (t, J = 5.7 Hz, 1H, CH₂C*H*(OCH₃)₂), 7.45 - 7.58 (m, 5H, Phenyl-C*H*), 7.76 (s, 1H, Pyrazole-3-C*H*).
**¹³C-NMR** (DMSO-D₆): δ (ppm) = 29.6 (1C, CH₂CH(OCH₃)₂), 54.2 (2C, CH₂CH(OCH₃)₂), 95.8 (1C, Pyrazole-4C), 102.7 (1C, CH₂CH(OCH₃)₂), 126.3 (2C, Phenyl-*C*H, ortho), 129.3 (1C, Phenyl-*C*H, para), 130.0 (2C, Phenyl-*C*H, meta), 137.4 (1C, Phenyl-*C*, quartär), 140.2 (1C, Pyrazole-5*C*), 140.5 (1C, Pyrazole-3-*C*H).

### Example G: 2- 4-(1-Benzyl-4-hydroxypiperidin-4-yl)-1-phenylpyrazole-5-yl]acetaldehyddimethylacetal

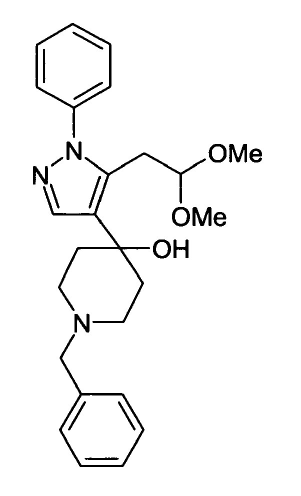

### Experimental procedure:

Under N₂-atmosphere n-butyl lithium in n-hexane (1.53 M, 10.5 mL, 16.1 mmol) was added slowly at -78 °C to a solution of the bromated Example F (5.0 g, 16.1 mmol) in abs. THF (80 mL). The reaction mixture was stirred for 15 minutes. Subsequently a solution of 1-Benzyl-piperidin-4-one (3.3 g, 17.7 mmol) in abs. THF (10 mL) was added at -78 °C. After stirring for 4.5 h at -78 °C the micture was heated to room temperature, stirred for a further hour and subsequently mixed with water until no further precipitate is forming (40 mL). Following extraction with CH₂Cl₂ the organic phases were dried over K₂CO₃ and filtered. The solvent was removed under vacuum and the crude product (8.3 g) purified using flash-chromatography (Ø=8 cm, h = 18 cm, ethylacetate:n-hexane = 8:2 + 2% N,N-Dimethylethylamine, 80 mL, R_{f} = 0.23).

Yellow Oil, crystallizing into a slightly yellow solid, melting point: 107°C, Yield 4.6 g (67%).
C₂₅H₃₁N₃O₃ (421.6)
**MS** (ESI): m/z (rel. Int.) = 422 [MH⁺, 100].
**IR** (neat): ν̃ (cm⁻¹) = 3445 (O-H), 3062, 3026 (C-H ₐᵣₒₘₐₜ), 2938 (C-H _{aliphat.}), 1599, 1501 (C=C), 1117, 1071 (C-O), 740, 697 (C-H).
**¹H-NMR** (DMSO-d₆): δ (ppm) = 1.77 (d breit, J = 12.5 Hz, 2H, N(CH₂C*H*₂)₂), 1.92 (td, J = 12.5/3.1 Hz, 2H, N(CH₂C*H*₂)₂), 2.42 (t, J = 10.6 Hz, 2H, N(C*H*₂CH₂)₂), 2.58 (d breit, J = 11.0 Hz, 2H, N(C*H*₂CH₂)₂), 3.04 (s, 6H, CH₂CH(OC*H*₃)₂), 3.12 (d, J = 5.5 Hz, 2H, C*H*₂CH(OCH₃)₂), 3.49 (s, 2H, NC*H*₂Ph), 4.50 (t, J = 5.8 Hz, 1H, CH₂C*H*(OCH₃)₂), 4.66 (s, 1H, O*H*), 7.21 - 7.27 (m, 1H, aromat. C*H*, para), 7.32 (m, 4H, aromat. C*H*, meta), 7.40 - 7.46 (m, 3H, aromat. C*H*, para/ortho), 7.49 (d, J = 7.0 Hz, 2H, aromat C*H*, ortho), 7.50 (s, 1H, Pyrazole-3-C*H*).

### Example 5: 1-Benzyl-6'-methoxy-1'-phenyl-6',7'-dihydro-1'H-spirofpiperidin-4,4'-pyrano[4,3-c]pyrazole]

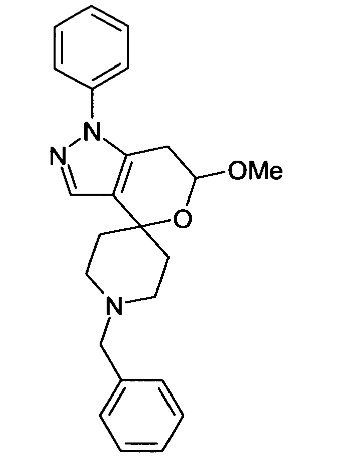

### Experimental procedure:

### Method 1:

To a solution of Example G (4.5 g, 10.7 mmol) in abs. Methanol (150 mL) p-Toluol sulphonic acid monohydrate (4.5 g, 23.5 mmol) was added and stirred at room temperature. After stirring for 21 h a solution of NaOH (0.5 M) was added upto reaching aproximately pH 10, dilluted with water and extracted 3 times with CH₂Cl₂. The pooled organic phases were dried over K₂CO₃, filtered and the solvent removed und vacuum. The crude product (4.9 g) was purified using flash-chromatography (Ø= 8 cm, h = 18 cm, ethylacetate, 80 mL, R_{f} = 0.27).
Coulorless solid,melting point: 151°C, Yield 3.0 g (73%).
C₂₄H₂₇N₃O₂ (389.5)

| | C | H | N |
|---|---|---|---|
| Calc. | 74.0 | 6.99 | 10.8 |
| Found | 73.7 | 6.91 | 10.5 |

**MS** (ESI): m/z (rel. Int.) = 390 [MH⁺, 100].
**IR** (neat): ν̃ (cm⁻¹) = 3028 (C-H _{aromat.}), 2919 (C-H _{aliphat.}), 1598, 1504 (C=C), 1114, 1059 (C-O), 739, 696 (C-H).
**¹H-NMR** (CDCl₃): δ (ppm) = 1.92 (dd, J = 14.1/3.1 Hz, 2H, N(CH₂C*H*₂)₂), 1.98 - 2.05 (m, 1H, N(CH₂C*H*_{2 axial})₂), 2.09 (td, J = 12.5/3.7 Hz, 1H, N(CH₂C*H*_{2 axial})₂), 2.45 (t breit, J = 11.7 Hz, 1H, N(C*H*₂CH₂)₂), 2.55 (t breit, J = 11.7 Hz, 1H, N(C*H*₂CH₂)₂), 2.77 (m, 2H, N(C*H*₂CH₂)₂), 2.88 (dd, J = 15.6/7.0 Hz, 1H, C*H*₂CHOCH₃), 2.96 (dd, J = 15.7/3.9 Hz, 1 H, C*H*₂CHOCH₃), 3.53 (s, 3H, OC*H*₃), 3.58 (s, 2H, NC*H*₂Ph), 4.84 (dd, J = 7.0/3.9 Hz, 1H, CH₂C*H*OCH₃), 7.23 - 7.28 (m, 1H, Phenyl-C*H*), 7.29 - 7.39 (m, 5H, Phenyl-C*H*), 7.40 - 7.47 (m, 4H, Phenyl-C*H*), 7.49 (s, 1H, Pyrazole-3-C*H*).
**¹³C-NMR** (CDCl₃): δ (ppm) = 31.2 (1C, *C*H₂CHOCH₃), 36.7 (1C, N(CH₂*C*H₂)₂), 39.5 (1C, N(CH₂*C*H₂)₂), 49.5 (1C, N(*C*H₂CH₂)₂), 49.6 (1C, N(*C*H₂CH₂)₂), 56.9 (1C, O*C*H₃), 63.7 (1C, N*C*H₂Ph), 71.9 (1C, Spiro-*C*), 77.5 (1C, Pyrazole-4-*C*), 96.8 (1C, CH₂*C*HOCH₃), 122.8 (2C, Phenyl-*C*H), 124.4 (1C, Phenyl-*C*, quartär), 127.3, 128.5, 129.5 (8C, Phenyl-*C*H), 133.8 (1C, Phenyl-*C*, quartär), 135.9 (1C, Pyrazole-3-*C*H), 139.5 (1C, Pyrazole-5-*C*).

### Example 6: 6'-Methoxy-1'-phenyl-6',7'-dihydro-1'H-spiro[piperidin-4,4'-pyrano[4,3-c]pyrazole]

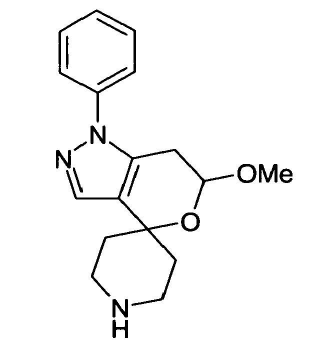

Experimental procedure:

### Method 1:

To a solution of erxample 5 (80 mg, 0.21 mmol) in abs. Methanol (5 mL) were added dried ammonium formiate (64.8 mg, 1.28 mmol) and 10% Pd/C (16 mg) in one portion. Subsequently it was stirred for 10 minutes under reflux and subsequently the catalysator was filtered through a fluted filter. Following thorough rinsing with methanol wurde the solvent was removed under vacuum. The crude product (63 mg) was purified using flash-chromatography (Ø= 2 cm, h = 15 cm, Methanol + 2% NH_{3 (conc.)}, 10 mL, R_{f} = 0.10). The fractions were subsequently dried under vacuum to dryness. Then the residue was dissolved again in CH₂Cl₂ and filtered through cotton wool. A further evaporation resulted in a yellow oil.
Yellow Oil, Yield 54 mg (88%)
C₁₇H₂₁NaO₂ (299.4)

| | C | H | N |
|---|---|---|---|
| Calc. | 68.2 | 7.07 | 14.0 |
| Found | 67.2 | 7.15 | 13.9 |

**MS** (ESI): m/z (rel. Int.) = 300 [MH⁺, 100], 598 [2 x M, 46].
**IR** (neat): ν̃ (cm⁻¹) = 3302 (N-H), 3061 (C-H _{aromat.}), 2921 (C-H _{aliphat.}), 2835 (C-H), 1598, 1576, 1503 (C=C), 1136 (C-O), 756, 694 (C-H).
**¹H-NMR** (DMSO-d₆): δ (ppm) = 1.55 - 1.68 (m, 2H, N(CH₂C*H*₂)₂), 1.79 - 1.92 (m, 2H, N(CH₂C*H*₂)₂), 2.75 (t breit, J = 11.0 Hz, 2H, N(C*H*₂CH₂)₂), 2.80 - 2.95 (m, 2H, N(C*H*₂CH₂)₂)*, 2.82 (dd, J = 15.7/7.0 Hz, 1H, C*H*₂CHOCH₃), 2.91 (dd, J = 15.7/3.9 Hz, 1H, C*H*₂CHOCH₃), 3.31 (s breit, 1 H, N*H*), 3.39 (s, 3H, OC*H*₃), 4.88 (dd, J = 6.7/3.5 Hz, 1H, CH₂C*H*OCH₃), 7.35 (t, J = 7.4 Hz, 1 H, Phenyl-C*H*, para), 7.44 - 7.55 (m, 4H, Phenyl-C*H*, meta, ortho), 7.59 (s, 1H, Pyrazole-3-C*H*).
* Signal is overlaid by the dd of the two methylene groups.

### Example 7: 6'-Methoxy-1'-phenyl-1-propyl-6',7'-dihydro-1'H-spiro[piperidin-4,4'-pyrano[4,3-c]pyrazole]

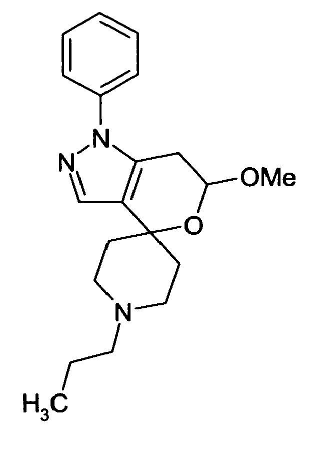

### Experimental procedure:

To a solution of the crude Example 6 (100 mg, 0.33 mmol) in acetonitrile (7 mL) were added propyl bromide (36.4 µL, 0.40 mmol) and K₂CO₃ (369.3 mg, 2.67 mmol). The mixture was heated under reflux for 40 h. Subsequently the K₂CO₃ was filtered off and the solvent was removed under vacuum. The residue was dissolved in CH₂Cl₂, diluted with water and extracted with a saturated solution of NaHCO₃. The pooled organic phases were dried over K₂CO₃, filtered and the solvent removed under vacuum. The crude product (118 mg) was purified using flash-chromatography (Ø=3 cm, h = 18 cm, ethylacetate + 2% N,N-Dimethylethylamine, 20 mL, R_{f} = 0.22).
Colourless resin, Yield 80.0 mg (70%) C₂₀H₂₇N₃O₂ (341.5)

| | C | H | N |
|---|---|---|---|
| Calc. | 70.4 | 7.97 | 12.3 |
| Found | 69.9 | 8.01 | 12.1 |

**MS** (ESI): m/z (rel. Int.) = 342 [MH⁺, 100].
**IR** (neat): ν̃ (cm⁻¹) = 2954, 2872 (C-H ₐₗᵢₚₕₐₜ.), 2808 (C-H), 1599, 1505 (C=C), 1139 (CO), 758, 694 (C-H).
**¹H-NMR** (CDCl₃): δ (ppm) = 0.94 (t, J = 7.4 Hz, 3H, NCH₂CH₂C*H*₃), 1.58 (m, 2H, NCH₂C*H*₂CH₃), 1.95 (dd, J = 13.9/2.5 Hz, 2H, N(CH₂C*H*₂)₂), 2.00 - 2.08 (m, 1H, N(CH₂C*H*₂)₂), 2.14 (td, J = 12.9/3.6 Hz, 1 H, N(CH₂C*H*₂)₂), 2.40 (t, J = 7.8 Hz, 2H, NC*H*₂CH₂CH₃), 2.42 (t breit, J = 11.7 Hz, 1H, N(C*H*₂CH₂)₂)*, 2.52 (t breit, J = 11.7 Hz, 1H, N(C*H*₂CH₂)₂), 2.79 - 2.85 (m, 2H, N(C*H*₂CH₂)₂), 2.90 (dd, J = 15.7/6.3 Hz, 1H, C*H*₂CHOCH₃), 2.98 (dd, J = 15.7/3.9 Hz, 1H, C*H*₂CHOCH₃), 3.56 (s, 3H, OC*H*₃), 4.85 (dd, J = 6.7/3.5 Hz, 1 H, CH₂C*H*OCH₃), 7.29 - 7.34 (m, 1 H, Phenyl-C*H*, para), 7.41 - 7.48 (m, 4H, Phenyl-C*H*), 7.50 (s, 1H, Pyrazole-3-C*H*).
*The Signal is overlaid by the triplett of the methylene group of the n-propyl radical.
**¹³C-NMR** (CDCl₃): δ (ppm) = 12.3 (1C, NCH₂CH₂*C*H₃), 20.5 (1C, NCH₂*C*H₂CH₃), 31.2 (1C, *C*H₂CHOCH₃), 36.6 (1C, N(CH₂*C*H₂)₂), 39.4 (1C, N(CH₂*C*H₂)₂), 49.5 (1C, N(*C*H₂CH₂)₂), 49.7 (1C, N(*C*H₂CH₂)₂), 56.9 (1C, O*C*H₃), 61.2 (1C, N*C*H₂CH₂CH₃), 71.9 (1C, Spiro-C), 77.4 (1C, Pyrazole-4-C), 96.9 (1C, CH₂*C*HOCH₃), 122.8 (2C, Phenyl-*C*H, ortho), 127.3 (1C, Phenyl-*C*H, para), 129.5 (2C, Phenyl-*C*H, meta), 133.8 (1C, Phenyl-*C*, quartär), 135.9 (1C, Pyrazole-3-*C*H), 139.5 (1C, Pyrazole-5-*C*).

### Example 8: 1-Isopropyl-6'-methoxy-1'-phenyl-6',7'-dihydro-1'H-spiro[piperidin-4,4'-pyrano[4,3-c]pyrazole]

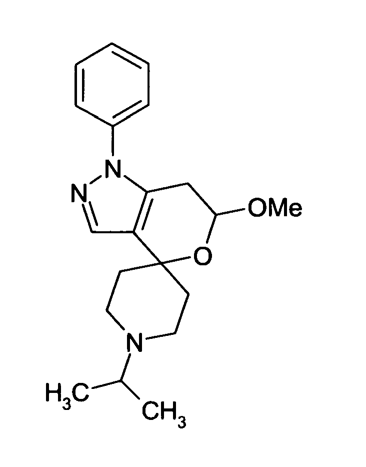

Experimental procedure:

### Method 1:

To a solution of the crude example **6** (150 mg, 0.50 mmol) in acetonitrile (15 mL) K₂CO₃ (553 mg, 4.0 mmol), tetrabutyl ammoniumiodide (37.0 mg, 0.10 mmol) and isopropyl bromide (56.4 µL, 0.60 mmol) were added. The mixture was heated for 48 h unter reflux. Subsequently the K₂CO₃ was filtered off and the solvent removed under vacuum. The residue was dissolved again in ethylacetate, diluted with water and several times extracted with ethylacetate. The pooled organic phases were dried over K₂CO₃, filtered and the solvent finally removed under vacuum. The crude product (218 mg) was purified using flash-chromatography (Ø= 4 cm, h = 18 cm, ethylacetate:methanol 3:7 + 2% N,N-Dimethylethylamine, 30 mL, R_{f} = 0.15).
Colourless resin, Yield 53 mg (31 %).

### Method 2:

To a solution of crude example 6 (150 mg, 0.50 mmol) in abs. dichloroethane (5 mL) acetone (44.2 µL, 0.60 mmol), sodium triacetoxy-borhydride (148.7 mg, 0.70 mmol) and glacial acetic acid (28.7 µL, 0.50 mmol) were added. The reaction mixture was stirred for 61 h at room temperature. After alkalizing with a solution of NaOH (0.5 N, 2 mL) and diluting with water it was extracted 3 times with CH₂Cl₂. The pooled organic phases were subsequently dried over K₂CO₃, filtered and the solvent removed under vacuum. The crude product (151 mg) was purified using flash-chromatography (Ø=3 cm, h = 18 cm, ethylacetate:methanol 1:1, 20 mL, R_{f} = 0.48).
Colourless resin, Yield 51 mg (30%).
C₂₀H₂₇N₃O₂ (341.5)

| | C | H | N |
|---|---|---|---|
| Calc. | 70.4 | 7.97 | 12.3 |
| Found | 69.8 | 8.00 | 11.9 |

**MS** (ESI): m/z (rel. Int.) = 342 [MH⁺, 100].
**IR** (neat): ν̃ (cm⁻¹) = 2960, 2920 (C-H _{aliphat.}), 2833 (C-H), 1599, 1505 (C=C), 1139, 1106 (C-O), 759, 695 (C-H).
**¹H-NMR** (CDCl₃): δ (ppm) = 1.13 (d, J = 6.3 Hz, 6H, CH(C*H*₃)₂), 1.88 - 2.15 (m, 4H, N(CH₂C*H*₂)₂), 2.60 - 2.86 (m, 5H, N(C*H*₂CH₂)₂ (4H), NC*H*(CH₃)₂ (1 H)), 2.90 (dd, J = 15.7/7.0 Hz, 1H, C*H*₂CHOCH₃), 2.98 (dd, J = 15.7/3.9 Hz, 1H, C*H*₂CHOCH₃), 3.56 (s, 3H, OC*H*₃), 4.86 (dd, J = 7.0/3.9 Hz, 1 H, CH₂C*H*OCH₃), 7.30 - 7.36 (m, 1 H, Phenyl-C*H*, para), 7.42 - 7.49 (m, 4H, Phenyl-C*H*), 7.51 (s, 1 H, Pyrazole-3-C*H*).

### Example 9: 1-Butyl-6'-methoxy-1'-phenyl-6',7'-dihydro-1'H-spiro[piperidin-4,4'-pyrano[4,3-c]pyrazole]

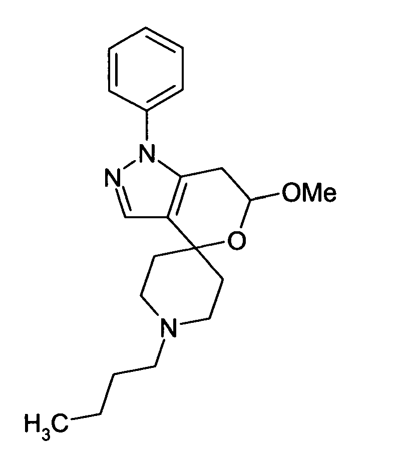

### Experimental procedure:

To a solution of crude example **6** (95 mg, 0.32 mmol) in acetonitrile (11 mL) butyl bromide (40.9 µL, 0.38 mmol) and K₂CO₃ (350.8 mg, 2.54 mmol)were added. This mixture was heated for 21 h under reflux. Subsequently it was filtered and the solvent removed under vacuum. The crude product (110 mg) was purified using flash-chromatography (Ø=3 cm, h = 15 cm, ethylacetate + 2% N,N-Dimethylethylamine, 20 mL, R_{f} = 0.28).

Slightly yellow solid, melting point: 78°C, Yield 90.6 mg (80%)
C₂₁H₂₉N₃O₂ (355.5)

| | C | H | N |
|---|---|---|---|
| Calc. | 71.0 | 8.22 | 11.8 |
| Found | 70.9 | 8.11 | 11.7 |

**MS** (ESI): m/z (rel. Int.) = 356 [MH⁺, 100].
IR (neat): ν̃ (cm⁻¹) = 2927, 2870 (C-H _{aliphat.}), 2807 (C-H), 1599, 1505 (C=C), 1139 (CO), 758, 694 (C-H).
**¹H-NMR** (CDCl₃): δ (ppm) = 0.95 (t, J = 7.0 Hz, 3H, NCH₂CH₂CH₂C*H*₃), 1.31 - 1.41 (m, 2H, NCH₂CH₂C*H*₂CH₃), 1.50 - 1.60 (m, 2H, NCH₂C*H*₂CH₂CH₃), 1.95 (dd, J = 13.3/2.3 Hz, 2H, N(CH₂C*H*₂)₂), 2.00 - 2.08 (m, 1H, N(CH₂C*H*₂)₂), 2.12 (td, J = 11.8/3.1 Hz, 1 H, N(CH₂C*H*₂)₂), 2.42 (t, J = 7.8 Hz, 3H, NC*H*₂CH₂CH₂CH₃, N(C*H*₂CH₂)₂), 2.51 (t breit, J = 11.7 Hz, 1H, N(C*H*₂CH₂)₂), 2.78 - 2.88 (m, 2H, N(C*H*₂CH₂)₂), 2.90 (dd, J = 15.7/6.7 Hz, 1H, C*H*₂CHOCH₃), 2.98 (dd, J = 15.7/3.1 Hz, 1H, C*H*₂CHOCH₃), 3.56 (s, 3H, OCH₃), 4.86 (dd, J = 7.0/ 3.5 Hz, 1 H, CH₂C*H*OCH₃), 7.29 - 7.36 (m, 1 H, Phenyl-C*H*), 7.41 - 7.51 (m, 5H, Phenyl-CH, Pyrazole-3-C*H*).
**¹³C-NMR** (CDCl₃): δ (ppm) = 14.3 (1C, NCH₂CH₂CH₂CH₃), 21.1 (1C, NCH₂CH₂CH₂CH₃), 29.8 (1C, NCH₂*C*H₂CH₂CH₃), 31.2 (1C, *C*H₂CHOCH₃), 36.7 (1C, N(CH₂CH₂)₂), 39.5 (1C, N(CH₂*C*H₂)₂), 49.6 (1C, N(*C*H₂CH₂)₂), 49.7 (1C, N(*C*H₂CH₂)₂), 56.9 (1C, O*C*H₃), 59.0 (1C, N*C*H₂CH₂CH₂CH₃), 71.9 (1C, Spiro-*C*), 77.4 (1C, Pyrazole-4-*C*), 96.6 (1C, CH₂*C*HOCH₃), 122.8 (2C, Phenyl-*C*H, ortho), 127.3 (1C, Phenyl-*C*H, para), 129.5 (2C, Phenyl-*C*H, meta), 133.7 (1C, Phenyl-*C*, quartär), 135.9 (1C, Pyrazole-3-*C*H), 139.5 (1C, Pyrazole-5-C).

### Example 10: 1-Isobutyl-6'-methoxy-1'-phenyl-6',7'-dihydro-1'H-spiro[piperidin-4,4'-pyrano[4,3-c]pyrazole]

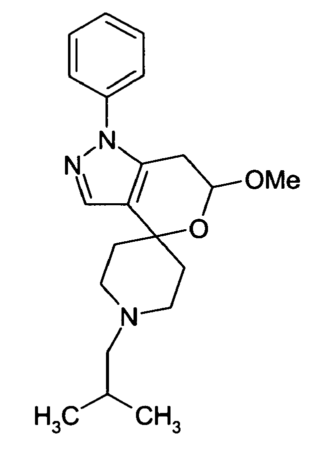

### Experimental procedure:

To a solution of crude example **6** (80 mg, 0.27 mmol) in abs. dichloro ethane (1.5 mL) were added consecutively freshly distilled isobutyl-aldehyde (24.4 µL, 0.27 mmol) and Sodiumtriacetoxyborhydride (84.9 mg, 0.40 mmol). This reaction mixture was stirred for 1 h at room temperature. After addition of a saturated solution of NaHCO₃ (8 mL) and water (∼ 4 mL), it was extracted 3 times with CH₂Cl₂. Subsequently the pooled organic phases were dried over K₂CO₃ and filtered. After evaporation of the solvent under vacuum, the crude product (102 mg) was purified using flash-chromatography (∅=3 cm, h = 20 cm, n-hexane:ethylacetate 1:1 + 2% N,N-Dimethylethylamine, 20 mL, R_{f} = 0.47).

Colourless solid, melting point: 143 °C, Yield 78 mg (82%)
C₂₁H₂₉N₃O₂ (355.5)

| | C | H | N |
|---|---|---|---|
| Calc. | 71.0 | 8.22 | 11.8 |
| Found | 70.7 | 8.18 | 11.7 |

**MS** (ESI): m/z (rel. Int.) = 356 [MH⁺, 100].
IR (neat): ν̃ (cm⁻¹) = 2961, 2863 (C-H _{aliphat.}), 2809 (C-H), 1600, 1504 (C=C), 1129 (CO), 756, 696 (C-H). **¹H-NMR (CDCl₃): δ (ppm)** = 0.94 (d, J = 6.7 Hz, 6H, CH₂CH(C*H*₃)₂), 1.78 - 1.90 (m, 1H, CH₂C*H*(CH₃)₂), 1.93 (dd breit, J = 13.7/2.3 Hz, 2H, N(CH₂C*H*₂)₂), 1.98 - 2.07 (m, 1H, N(CH₂C*H*₂)₂), 2.05 - 2.14 (m, 1 H, N(CH₂C*H*₂)₂)*, 2.17 (d, J = 7.0 Hz, 2H, C*H*₂CH(CH₃)₂), 2.38 (t breit, J = 11.3 Hz, 1H, N(C*H*₂CH₂)₂), 2.46 (t breit, J = 11.3 Hz, 1H, N(C*H*₂CH₂)₂), 2.69 - 2.79 (m, 2H, N(C*H*₂CH₂)₂), 2.91 (dd, J = 15.7/7.0 Hz, 1H, C*H*₂CHOCH₃), 2.98 (dd, J = 15.7/3.1 Hz, 1H, C*H*₂CHOCH₃), 3.56 (s, 3H, OC*H*₃), 4.85 (dd, J = 7.0/3.5 Hz, 1H, CH₂C*H*OCH₃), 7.30 - 7.37 (m, 1 H, Phenyl-C*H*, para), 7.41 - 7.50 (m, 4H, Phenyl-C*H*), 7.52 (s, 1H, Pyrazole-3-C*H*).
* The Signal is partly overlaid by the dublett of the i-butyl-group.
**¹³C-NMR** (CDCl₃): δ (ppm) = 21.3 (2C, NCH₂CH(*C*H₃)₂), 25.9 (1C, NCH₂*C*H(CH₃)₂), 31.2 (1C, *C*H₂CHOCH₃), 36.7 (1C, N(CH₂*C*H₂)₂), 39.6 (1C, N(CH₂*C*H₂)₂), 49.8 (1C, N(*C*H₂CH₂)₂), 50.1 (1C, N(*C*H₂CH₂)₂), 56.9 (1C, O*C*H₃), 67.5 (1C, N*C*H₂CH(CH₃)₂), 72.1 (1C, Spiro-*C*), 77.4 (1C, Pyrazole-4-*C*), 96.8 (1C, CH₂*C*HOCH₃), 122.8 (2C, Phenyl-*C*H, ortho), 127.3 (1C, Phenyl-*C*H, para), 129.5 (2C, Phenyl-*C*H, meta), 133.8 (1C, Phenyl-*C*, quartär), 135.9 (1C, Pyrazole-3-*C*H), 139.5 (1C, Pyrazole-5-*C*).

### Example 11: 6'-Methoxy-1-pentyl-1'-phenyl-6',7'-dihydro-1'H-spiro[piperidin-4,4'-pyrano[4,3-c]pyrazole]

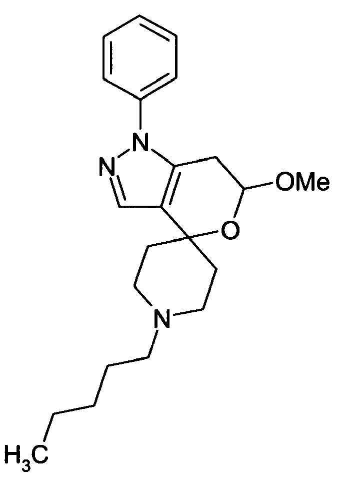

### Experimental procedure:

To a solution of crude example 6 (100 mg, 0.33 mmol) in abs. Dichloroethane (2 mL) were added consecutively freshly distilled valeraldehyde (35.6 µL, 0.33 mmol) and Sodiumtriacetoxyborhydride (106.2 mg, 0.50 mmol). This reaction mixture was stirred for 1.5 h at room temperature. Folowing addition of a saturated solution of NaHCO₃-(10 mL) and water (∼ 4 mL), it was extracted 3 times with CH₂Cl₂. Subsequently the pooled organic phases were dried over K₂CO₃ and filtered. After evaporation of the solvent under vacuum the crude product (134 mg) was purified by flash-chromatography (Ø=3 cm, h = 18 cm, n-hexane:ethylacetate 1:1 + 2% N,N-dimethylethylamine, 20 mL, R_{f} = 0.34).
Colourless solid, melting point: 87 °C, Yield 101 mg (80%)
C₂₂H₃₁N₃O₂ (369.6)

| | C | H | N |
|---|---|---|---|
| Calc. | 71.5 | 8.46 | 11.4 |
| Found | 71.3 | 8.51 | 11.2 |

**MS** (ESI): m/z (rel. Int.) = 370 [MH⁺, 100].
**IR** (neat): ν̃ (cm⁻¹) = 2925, 2858 (C-H ₐₗᵢₚₕₐₜ.), 2807 (C-H), 1599, 1505 (C=C), 1139, 1104 (C-O), 758, 695 (C-H).
**¹H-NMR** (CDCl₃): δ (ppm) = 0.85 (t, J = 7.0 Hz, 3H, NCH₂(CH₂)₃C*H*₃), 1.20 - 1.32 (m, 4H, NCH₂CH₂C*H*₂C*H*₂CH₃), 1.44 - 1.55 (m, 2H, NCH₂C*H*₂CH₂CH₂CH₃), 1.90 (dd breit, J = 14.1/2.3 Hz, 2H, N(CH₂C*H*₂)₂), 1.95 - 2.00 (m, 1H, N(CH₂C*H*₂)₂), 2.08 (t breit, J = 11.4 Hz, 1 H, N(CH₂C*H*₂)₂), 2.30 - 2.40 (m, 3H, NC*H*₂ (CH₂)₃CH₃, N(C*H*₂CH₂)₂), 2.43 (t breit, J = 11.0 Hz, 1H, N(C*H*₂CH₂)₂), 2.72 - 2.81 (m, 2H, N(C*H*₂CH₂)₂), 2.83 (dd, J = 15.7/6.3 Hz, 1H, C*H*₂CHOCH₃), 2.91 (dd, J = 15.7/3.9 Hz, 1H, C*H*₂CHOCH₃), 3.50 (s, 3H, OC*H*₃), 4.79 (dd, J = 6.7/3.5 Hz, 1 H, CH₂C*H*OCH₃), 7.22 - 7.28 (m, 1 H, Phenyl-C*H*, para), 7.35 - 7.42 (m, 4H, Phenyl-C*H*), 7.44 (s, 1H, Pyrazole-3-C*H*).
**¹³C-NMR** (CDCl₃): δ (ppm) = 14.3 (1C, NCH₂CH₂CH₂CH₂*C*H₃), 22.9 (1C, NCH₂CH₂CH₂*C*H₂CH₃), 27.1 (1C, NCH₂*C*H₂-CH₂CH₂CH₃), 30.1 (1C, NCH₂CH₂*C*H₂CH₂CH₃), 31.2 (1C, *C*H₂CHOCH₃), 36.7 (1C, N(CH₂*C*H₂)₂), 39.4 (1C, N(CH₂*C*H₂)₂), 49.6 (1C, N(*C*H₂CH₂)₂), 49.7 (1C, N(*C*H₂CH₂)₂), 56.9 (1C, O*C*H₃), 59.3 (1C, NCH₂CH₂CH₂CH₂CH₃), 71.9 (1C, Spiro-*C*), 77.4 (1C, Pyrazole-4-*C*), 96.9 (CH₂CHOCH₃), 122.8 (2C, Phenyl-*C*H, ortho), 127.3 (1C, Phenyl-*C*H, para), 129.5 (2C, Phenyl-*C*H, meta), 133.7 (1C, Phenyl-*C*, quartär), 135.9 (1C, Pyrazole-3-*C*H), 139.5 (1C, Pyrazole-5-*C*).

### Example 12: 1-Isopentyl-6'-methoxy-1'-phenyl-6',7'-dihydro-1'H-spiro[piperidin-4,4'-pyrano[4,3-c]pyrazole]

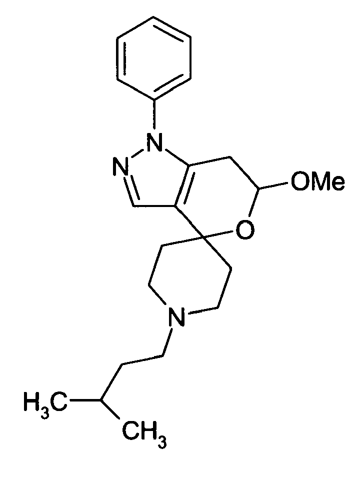

### Experimental procedure:

To a solution of crude example 6 (56 mg, 0.19 mmol) in abs. Dichlorethan (2 mL) were added consecutively freshly distilled Isovaleraldehyde (20.1 µL, 0.19 mmol) and Sodiumtriacetoxyborhydride (59.5 mg, 0.28 mmol). This reaction mixture was stirred for 1.5 h at room temperature. After addition of a saturated solution of NaHCO₃ (8 mL) and water (∼5 mL), it was extracted 3 times with CH₂Cl₂. Subsequently the pooled organic phases were dried over K₂CO₃ and filtered. After removal of the solvent under vacuum the crude product (61 mg) was purified using flash-chromatography (Ø=2 cm, h = 18 cm, Ethylacetat + 2% N,N-Dimethylethylamin, 10 mL, R_{f} = 0.39).

Colourless resin, Yield 48 mg (70%)
C₂₂H₃₁N₃O₂ (369.6)

| | C | H | N |
|---|---|---|---|
| Calc. | 71.5 | 8.46 | 11.4 |
| Found | 71.2 | 8.65 | 11.2 |

**MS** (ESI): m/z (rel. Int.) = 370 [MH⁺, 100].
**IR** (neat): ν̃ (cm⁻¹) = 3061 (C-H _{aromat.}), 2951, 2922, 2867 (C-H _{aliphat.}), 2808 (C-H), 1599, 1505 (C=C), 1141 (C-0), 757, 694 (C-H).
**¹H-NMR** (CDCl₃): δ (ppm) = 0.93 (d, J = 6.7 Hz, 6H, NCH₂CH₂CH-(C*H*₃)₂), 1.41 - 1.50 (m, 2H, NCH₂C*H*₂CH-(CH₃)₂), 1.57 - 1.66 (m, 1H, NCH₂CH₂C*H*(CH₃)₂), 1.96 (dd breit, J = 13.7/2.0 Hz, 2H, N(CH₂C*H*₂)₂), 2.02 - 2.08 (m, 1H, N(CH₂C*H*₂)₂), 2.09 - 2.17 (m, 1H, N(CH₂C*H*₂)₂), 2.37 - 2.55 (m, 4H, NC*H₂*CH₂CH(CH₃)₂ (2H), N(C*H*₂CH₂)₂), (2H)), 2,79 - 2.83 (m, 2H, N(C*H*₂CH₂)₂), 2.91 (dd, J = 15.7/7.0 Hz, 1H, C*H*₂CHOCH₃), 2.99 (dd, J = 15.7/3.9 Hz, 1H, C*H*₂CHOCH₃), 3.56 (s, 3H, OC*H*₃), 4.86 (dd, J = 6.7/3.5 Hz, 1 H, CH₂C*H*OCH₃), 7.30 - 7.35 (m, 1 H, Phenyl-C*H*, para), 7.41 - 7.48 (m, 4H, Phenyl-C*H*), 7.50 (s, 1H, Pyrazole-3-C*H*).
**¹³C-NMR** (CDCl₃): δ (ppm) = 23.0 (2C, NCH₂CH₂CH(*C*H₃)₂), 27.1 (2C, NCH₂CH₂*C*H(CH₃)₂), 31.2 (1C, *C*H₂CHOCH₃), 36.3 (1C, NCH₂*C*H₂CH(CH₃)₂), 36.7 (1C, N(CH₂*C*H₂)₂), 39.5 (1C, N(CH₂*C*H₂)₂), 49.7 (1C, N(*C*H₂CH₂)₂), 49.8 (1C, N(*C*H₂CH₂)₂), 56.9 (1C, O*C*H₃), 57.5 (1C, N*C*H₂CH₂CH(CH₃)₂), 71.9 (1C, Spiro-*C*), 77.4 (1C, Pyrazole-4-C), 96.9 (1C, CH₂*C*HOCH₃), 122.8 (2C, Phenyl-*C*H, ortho), 127.3 (1C, Phenyl-*C*H, para), 129.5 (2C, Phenyl-*C*H, meta), 133.7 (1C, Phenyl-C, quartär), 135.9 (1C, Pyrazole-3-*C*H), 139.5 (1C, Pyrazole-5-*C*).

### Example 13: 6'-Methoxy-1-(3-methylbut-2-en-1-yl)-1'-phenyl-6',7'-dihydro-1'H-spiro[piperidin-4,4'-pyrano[4,3-c]pyrazole]

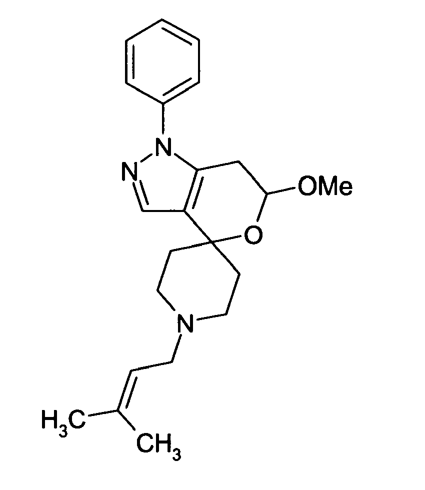

### Experimental procedure:

To a solution of crude example 6 (100 mg, 0.33 mmol) in acetonitrile (5 mL) 1-Bromo-3-methyl-but-2-en (47.0 µL, 0.40 mmol) and K₂CO₃ (369.3 mg, 2.67 mmol) were added. This mixture was heated for 23 h under reflux. Subsequently it was filtered and the solvent removed under vacuum. The crude product (142 mg) was purified using flash-chromatography (Ø= 3 cm, h = 18 cm, n-hexane:ethylacetate 3:7 + 2% N,N-Dimethylethylamine, 20 mL, R_{f} = 0.16).
Colourless Oil, Yield 89 mg (72 %)
C₂₂H₂₉N₃O₂ (367.5)

| | C | H | N |
|---|---|---|---|
| Calc. | 71.9 | 7.95 | 11.4 |
| Found | 71.6 | 7.97 | 11.2 |

**MS** (ESI): m/z (rel. Int.) = 368 [MH⁺, 100], 734 [2 x M⁺, 12].
**IR** (neat): ν̃ (cm⁻¹) = 3025 (C-H _{aromat.}), 2917 (C-H _{aliphat.}), 1599, 1505 (C=C), 1059, 1045 (C-O).
**¹H-NMR** (CDCl₃): δ (ppm) = 1.75 (s, 3H, (*H*₃C)₂C=CHCH₂N), 1.82 (s, 3H, (*H*₃C)₂C=CHCH₂N), 1.92 - 2.07 m, 2H, N(CH₂C*H*₂)₂), 2.09 - 2.24 (m, 2H, N(CH₂C*H*₂)₂), 2.48 (td, J = 11.8/2.8 Hz, 1 H, N(C*H*₂CH₂)₂), 2.56 (td, J = 11.8/2.8 Hz, 1H, N(C*H*₂CH₂)₂), 2.86 - 2.96 (m, 2H, N(C*H*₂CH₂)₂), 2.97 (dd, J = 15.7/6.7 Hz, 1H, C*H*₂CHOCH₃), 3,05 (dd, J = 15.7/3.8 Hz, 1H, C*H*₂CHOCH₃), 3.11 (d, J = 7.2 Hz, 2H, (H₃C)₂C=CHC*H*₂N), 3.64 (s, 3H, OC*H*₃), 4.92 (dd, J = 6.7/3.8 Hz, 1H, CH₂C*H*OCH₃), 5.39 (t breit, J = 7.2 Hz, 1H, (H₃C)₂C=C*H*CH₂N), 7.36 - 7.43 (m, 1H, Phenyl-C*H*, para), 7.50 - 7.54 (m, 4H, Phenyl-C*H*), 7.56 (s, 1H, Pyrazole-3-C*H*).

### Example 14: 6'-Methoxy-1-octyl-1'-phenyl-6',7'-dihydro-1'H-spiro[piperidin-4,4'-pyrano[4,3-c]pyrazole]

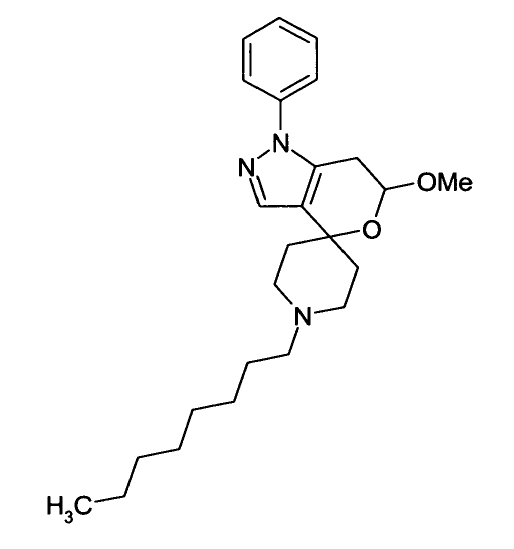

### Experimental procedure:

To a solution of crude example 6 (80 mg, 0.27 mmol) in acetonitrile (5 mL) 1-Bromoctane (55.6 µL, 0.32 mmol) and K₂CO₃ (295 mg, 2.14 mmol) were added. This mixture was heated for 19 h under reflux. Subsequently the K₂CO₃ was filtered off and the solvent removed under vacuum. The crude product (105 mg) was purified using flash-chromatography (Ø=3 cm, h = 20 cm, n-hexane:ethylacetate 1:1 + 2% N,N-Dimethylethylamine, 20 mL, R_{f} = 0.21).
Colourless solid, melting point: 86°C, Yield 74 mg (67%)
C₂₅H₃₇N₃O₂ (411.7)

| | C | H | N |
|---|---|---|---|
| Calc. | 73.0 | 9.06 | 10.2 |
| Found | 72.7 | 9.07 | 10.1 |

**MS** (ESI): m/z (rel. Int.) = 412 [MH⁺, 100].
**IR** (neat): ν̃ (cm⁻¹) = 3001 (C-H ₐᵣₒₘₐₜ), 2951, 2919 (C-H _{aliphat.}), 2851, 2806 (C-H), 1596, 1504 (C=C), 1126, 1060 (C-O).
**¹H-NMR** (CDCl₃): δ (ppm) = 0.90 (t, J = 7.0 Hz, 3H, NCH₂(CH₂)₆C*H*₃), 1.22 - 1.38 (m, 10H, NCH₂CH₂(C*H*₂)₅CH₃), 1.50 - 1.60 (m, 2H, NCH₂C*H*₂(CH₂)₅CH₃), 1.95 (dd, J = 12.5/1.6 Hz, 2H, N(CH₂C*H*₂)₂), 2.00 - 2.09 (m, 1H, N(CH₂C*H*_{2 axial})₂), 2.13 (td, J = 12.9/3.9 Hz, 1H, N(CH₂C*H*_{2 axial})₂), 2.39 - 2.48 (m, 3H, NC*H*₂(CH₂)₆CH₃ (2H), NC*H*₂CH₂)₂ (1H)), 2.51 (t breit, J = 10.6 Hz, 1 H, N(C*H*₂CH₂)₂), 2.80 - 2.90 (m, 2H, N(C*H*₂CH₂)₂), 2.92 (dd, J = 15.7/7.0 Hz, 1H, C*H*₂CHOCH₃), 2.98 (dd, J = 15.7/3.1 Hz, 1 H, C*H*₂CHOCH₃), 3.57 (s, 3H, OC*H*₃), 4.87 (dd, J = 6.7/3.5 Hz, 1 H, CH₂C*H*OCH₃), 7.33 (m, 1 H, Phenyl-C*H*, para), 7.43 - 7.51 (m, 4H, Phenyl-C*H*), 7.51 (s, 1H, Pyrazole-3-C*H*).
**¹³C-NMR (CDCl₃):** δ (ppm) = 14.3 (1C, NCH₂(CH₂)₆*C*H₃), 22.9 (1C, NCH₂(CH₂)₅*C*H₂CH₃), 27.4 (1C, NCH₂*C*H₂(CH₂)₅CH₃), 28.0 (1C, NCH₂(CH₂)₄*C*H₂CH₂CH₃), 29.5 (N(CH₂)₃*C*H₂CH₂CH₂-CH₃), 29.8 (1C, NCH₂(CH₂)₂*C*H₂(CH₂)₃CH₃), 31.2 (1C, *C*H₂CHOCH₃), 32.1 (1C, NCH₂CH₂CH₂(CH₂)₄CH₃), 36.7 (1C, N(CH₂*C*H₂)₂), 39.5 (1C, N(CH₂*C*H₂)₂), 49.6 (1C, N(*C*H₂CH₂)₂), 49.7 (1C, N(*C*H₂CH₂)₂), 56.9 (1C, O*C*H₃), 59.4 (1C, N*C*H₂(CH₂)₆CH₃), 71.9 (1C, Spiro-*C*), 77.4 (1C, Pyrazole-4-*C*), 96.9 (1C, CH₂*C*HOCH₃), 122.8 (2C, Phenyl-*C*H, ortho), 127.3 (1 C, Phenyl-*C*H, para), 129.5 (2C, Phenyl-*C*H, meta), 133.7 (1C, Phenyl-*C*, quartär), 135.9 (1C, Pyrazole-3-*C*H), 139.5 (1C, Pyrazole-5-*C*).

### Example 15: 1-(Cyclohexan-1-ylmethyl)-6'-methoxy-1'-phenyl-6',7'-dihydro-1'H-spiro[piperidin-4,4'-pyrano[4,3-c]pyrazole]

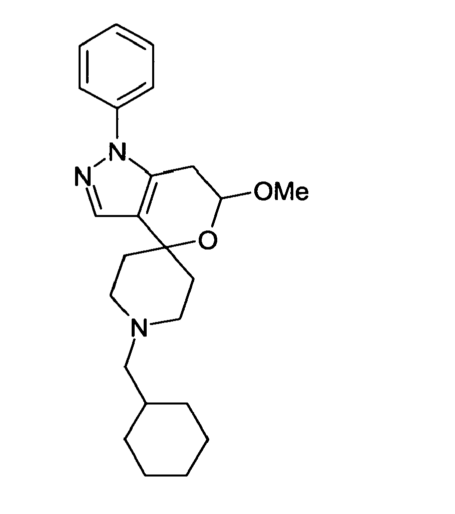

### Experimental procedure:

To a solution of crude example 6 (80 mg, 0.27 mmol) in acetonitrile (5 mL) (Bromomethyl)cyclohexane (48.5 µL, 0.35 mmol) and K₂CO₃ (295 mg, 2.14 mmol) were added. This mixture was heated for 26 h under reflux. Subsequently K₂CO₃ was filtered off and the solvent removed under vacuum. The crude product (103 mg) was purified using flash-chromatography (Ø = 2.5 cm, h = 15 cm, n-hexane:ethylacetate 7:3 + 1 % N,N-Dimethylethylamine, 10 mL, R_{f} = 0.18).
Colourless solid, melting point: 151 °C, Yield 82 mg (77%) C₂₄H₃₃N₃O₂ (395.6)

| | C | H | N |
|---|---|---|---|
| Calc. | 72.9 | 8.41 | 10.6 |
| Found | 72.7 | 8.39 | 10.4 |

**MS** (ESI): m/z (rel. Int.) = 396 [MH⁺, 100], 813 [2 x M + Na⁺, 7].
**IR** (neat): ν̃ (cm⁻¹) = 2918 (C-H _{aliphat.}), 2848 (C-H), 1599, 1505 (C=C), 1114, 1059 (CO).
**¹H-NMR** (CDCl₃): δ (ppm) = 0.84 - 0.97 (m, 2H, NCH₂C₆*H*₁₁), 1.18 - 1.29 (m, 4H, NCH₂C₆*H*₁₁), 1.47 - 1.59 (m, 1H, NCH₂C₆*H*₁₁), 1.62 - 1.87 (m, 4H, NCH₂C₆*H*₁₁), 1.90 - 1.98 (m, 2H, N(CH₂C*H*₂)₂), 2.02 - 2.15 (m, 2H, N(CH₂C*H*₂)₂), 2.21 (d, J = 7.0 Hz, 2H, NC*H*₂C₆H₁₁), 2.37 (td, J = 11.4/2.4 Hz, 1H, N(C*H*₂CH₂)₂), 2.45 (td, J = 11.7/2.4 Hz, 1H, N(C*H*₂CH₂)₂), 2.70 - 2.79 (m, 2H, N(C*H*₂CH₂)₂), 2.90 (dd, J = 15.7/7.0 Hz, 1 H, C*H*₂CHOCH₃), 2.97 (dd, J = 15.7/3.5 Hz, 1 H, C*H*₂CHOCH₃), 3.56 (s, 3H, OC*H*₃), 4.84 (dd, J = 6.7/3.5 Hz, 1H, CH₂C*H*OCH₃), 7.30 - 7.35 (m, 1H, Phenyl-C*H*, para), 7.41 - 7.48 (m, 4H, Phenyl-C*H*), 7.51 (s, 1H, Pyrazole-3-C*H*).

### Example 16: 6'-Methoxy-1'-phenyl-1-(2-phenylethyl)-6',7'-dihydro-1'H-spiro[piperidin-4,4'-pyrano[4,3-c]pyrazole]

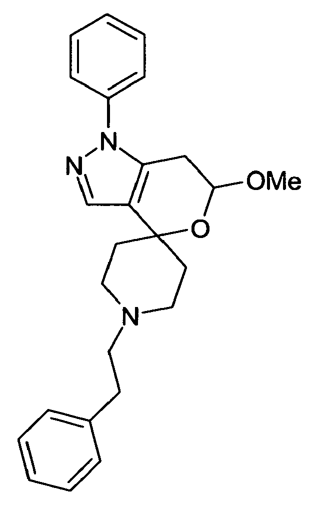

### Experimental procedure:

To a solution of crude example 6 (80 mg, 0.27 mmol) in abs. dichloroethane (1.5 mL) were added consecutively freshly distilled phenyl-acetaldehyde (31.2 µL, 0.27 mmol) and sodiumtriacetoxyborhydride (84.9 mg, 0.40 mmol). This reaction mixture was stirred for 1.5 h at room temperature. After addition of a saturated solution of NaHCO₃ (6 mL) and water (∼ 5 mL), it was extracted 3 times with CH₂Cl₂. Subsequently the pooled organic phases were dried over K₂CO₃ and filtered. After removal of the solvent under vacuum the crude product (125 mg) was purified using flash-chromatography (Ø=3 cm, h = 18 cm, n-hexane:ethylacetate 1:1 + 2% N,N-Dimethylethylamine, 20 mL, R_{f} = 0.39).
Slightly yellow resin, Yield 91 mg (84 %).
C₂₅H₂₉N₃O₂ (403.6)

| | C | H | N |
|---|---|---|---|
| Calc. | 74.4 | 7.24 | 10.4 |
| Found | 73.9 | 7.24 | 10.1 |

**MS** (ESI): m/z (rel. Int.) = 404 [MH⁺, 100].
**IR** (neat): ν̃ (cm⁻¹) = 3060, 3026 (C-H _{aromat.}), 2919 (C-H _{aliphat.}), 2809 (C-H), 1599, 1504 (C=C), 1137, 1113 (C-O), 758, 696 (C-H).
**¹H-NMR** (CDCl₃): δ (ppm) = 1.95 - 2.11 (m, 3H, N(CH₂C*H*₂)₂), 2.17 (t breit, J = 11.0 Hz, 1 H, N(CH₂C*H*₂)₂), 2.54 (t breit, J = 11.0 Hz, 1 H, N(C*H*₂CH₂)₂), 2.63 (t breit, J = 11.0 Hz, 1 H, N(C*H*₂CH₂)₂), 2.68 - 2.76 (m, 2H,NCH₂C*H*₂Ph), 2.83 - 3.02 (m, 6H, N(C*H*₂CH₂)₂ (2H), NC*H*₂CH₂Ph (2H), C*H*₂CHOCH₃) (2H)), 3.56 (s, 3H, OC*H*₃), 4.87 (dd, J = 6.7/3.5 Hz, 1H, CH₂C*H*OCH₃), 7.19 - 7.37 (m, 5H, Phenyl-C*H*), 7.41 - 7.50 (m, 5H, Phenyl-C*H*), 7.51 (s, 1 H, Pyrazole-3-C*H*).
**¹³C-NMR** (CDCl₃): δ (ppm) = 31.3 (1C, CH₂CHOCH₃), 34.1 (1C, NCH₂CH₂Ph), 37.1 (1C, N(CH₂CH₂)₂), 39.8 (1C, N(CH₂*C*H₂)₂), 49.6 (1C, N(*C*H₂CH₂)₂), 49.8 (1C, N(*C*H₂CH₂)₂), 57.0 (1C, O*C*H₃), 61.1 (1C, NCH₂*C*H₂Ph), 71.8 (1C, Spiro-*C*), 77.5 (1C, Pyrazole-4-*C*), 97.0 (1C, CH₂CHOCH₃), 122.9 (2C, Phenyl-*C*H), 126.4 (1C, Phenyl-*C*, quartär), 127.4, 128.7, 129.0, 129.5 (8C, Phenyl-*C*H), 133.8 (1C, Phenyl-*C*, quartär), 135.9 (1C, Pyrazole-3-*C*H), 139.5 (1C, Pyrazole-5-*C*).

### Example 17: 6'-Methoxy-1'-phenyl-1-(3-phenylpropyl)-6',7'-dihydro-1'H-spiro[piperidin-4,4'-pyrano[4,3-c]pyrazole]

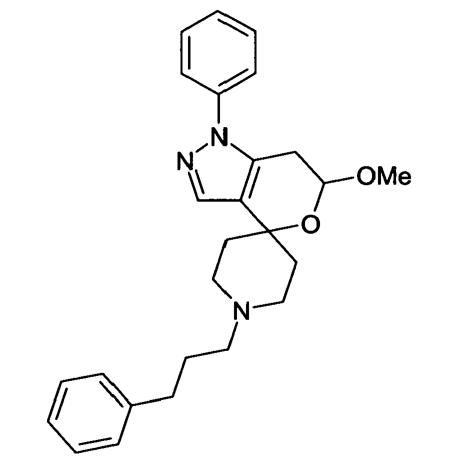

### Experimental procedure:

To a solution of crude example 6 (120 mg, 0.40 mmol) in acetonitrile (10 mL) 1-Bromo-3-phenylpropane (72.8 µL, 0.48 mmol) and K₂CO₃ (443.1 mg, 3.21 mmol) were added. This mixture was heated for 19 h under reflux. Subsequently it was filtered and the solvent removed under vacuum. The crude product (158 mg) was purified using flash-chromatography (Ø = 3 cm, h = 15 cm, n-hexane:ethylacetate 1:1 + 2% N,N-Dimethylethylamine, 20 mL, R_{f} = 0.14).
Colourless resin, Yield 121 mg (72%)
C₂₆H₃₁N₃O₂ (417.6)

| | C | H | N |
|---|---|---|---|
| Calc. | 74.8 | 7.48 | 10.1 |
| Found | 74.3 | 7.50 | 9.82 |

**MS** (ESI): m/z (rel. Int.) = 418 [MH⁺, 100].
**IR** (neat): ν̃ (cm⁻¹) = 3060, 3025 (C-H _{aromat.}), 2941 (C-H _{aliphat.}), 2808 (C-H), 1599, 1504 (C=C), 1137, 1115 (C-O), 758, 695 (C-H).
**¹H-NMR** (CDCl₃): δ (ppm) = 1.85 - 2.16 (m, 6H, N(CH₂C*H*₂)₂ (4H), NCH₂C*H*₂CH₂Ph (2H)), 2.38 - 2.58 (m, 4H, NCH₂CH₂C*H*₂Ph (2H), N(C*H*₂CH₂)₂ (2H)), 2.68 (t, J = 7.8 Hz, 2H, NC*H*₂CH₂CH₂Ph), 2.77 - 2.87 (m, 2H, N(C*H*₂CH₂)₂), 2.90 (dd, J = 15.7/7.0 Hz, 1H, C*H*₂CHOCH₃), 2.97 (dd, J = 15.3/3.5 Hz, 1H, C*H*₂CHOCH₃), 3.55 (s, 3H, OC*H*₃), 4.85 (dd, J = 7.0/3.1 Hz, 1H, CH₂C*H*OCH₃), 7.17 - 7.36 (m, 6H, Phenyl-C*H*), 7.41 - 7.48 (m, 4H, Phenyl-C*H*), 7.50 (s, 1 H, Pyrazole-3-CH).
**¹³C-NMR** (CDCl₃): δ (ppm) = 29.0 (1C, NCH₂*C*H₂CH₂Ph), 31.2 (1C, *C*H₂CHOCH₃), 34.1 (1C, N*C*H₂CH₂CH₂Ph), 36.7 (1C, N(CH₂*C*H₂)₂), 39.5 (1C, N(CH₂*C*H₂)₂), 49.5 (1C, N(*C*H₂CH₂)₂), 49.7 (1C, N(*C*H₂CH₂)₂), 56.9 (1C, O*C*H₃), 58.6 (1C, NCH₂CH₂CH₂Ph), 71.8 (1C, Spiro-*C*), 77.5 (1C, Pyrazole-4-*C*), 96.9 (1C, CH₂CHOCh₃), 122.8 (2C, Phenyl-*C*H), 124.2 (1C, Phenyl-*C*, quartär), 126.0, 127.3, 128.56, 128.63, 129.5 (8C, Phenyl-*C*H), 133.7 (1C, Phenyl-*C*, quartär), 135.8 (1C, Pyrazole-3-*C*H), 139.5 (1C, Pyrazole-5-*C*).

### Example 18: 6'-Methoxy-1'-phenyl-1-(4-phenylbutyl)-6',7'-dihydro-1'H-spiro[piperidin-4,4'-pyrano[4,3-c]pyrazole]

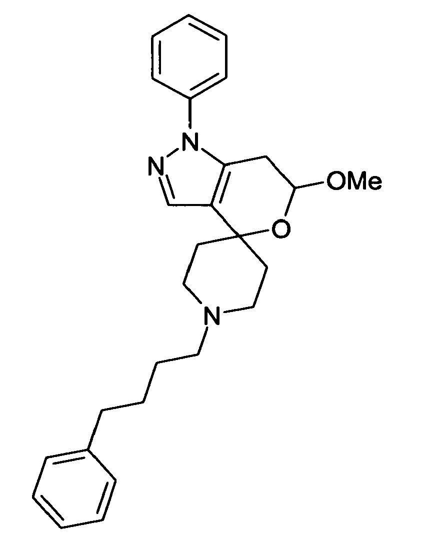

### Experimental procedure:

To a solution of crude example 6 (120 mg, 0.40 mmol) in acetonitrile (8 mL) were added consecutively K₂CO₃ (443 mg, 3.21 mmol), tetrabutylammoniumiodide (29.6 mg, 0.08 mmol) and 1-Chlor-4-phenylbutane (79.1 µL, 0.48 mmol). The mixture was heated for 26 h under reflux. Subsequently the K₂CO₃ was filtered off and the solvent removed under vacuum. The residue was dissolved in ethylacetate, diluted with water and several times extracted. The pooled organic phases were dried over K₂CO₃, filtered and following that the solvent was removed under vacuum. The crude product (157 mg) was purified using flash-chromatography (Ø=3 cm, h = 15 cm, n-hexane:ethylacetate 1:1 + 2% N,N-Dimethylethylamine, 20 mL, R_{f} = 0.17).
Colourless resin, Yield 83 mg (48%).
C₂₇H₃₃N₃O₂ (431.6)

| | C | H | N |
|---|---|---|---|
| Calc. | 75.1 | 7.71 | 9.74 |
| Found | 74.7 | 7.87 | 9.64 |

**MS** (ESI): m/z (rel. Int.) = 432 [MH⁺, 100].
**IR** (neat): ν̃ (cm⁻¹) = 3060, 3025 (C-H ₐᵣₒₘₐₜ), 2930, 2857 (C-H _{aliphat.}), 2807 (C-H), 1599, 1504 (C=C), 1137, 1115 (C-O), 758, 696 (C-H).
**¹H-NMR** (CDCl₃): δ (ppm) = 1.56 - 1.72 (m, 4H, NCH₂C*H*₂C*H*₂CH₂Ph), 1.91 - 2.17 (m, 4H, N(CH₂C*H*₂)₂), 2.38 - 2.55 (m, 4H, N(C*H*₂CH₂)₂ (2H), NCH₂CH₂CH₂C*H*₂Ph (2H)), 2.66 (t, J = 7.4 Hz, 2H, NC*H*₂CH₂CH₂CH₂Ph), 2.77 - 2.86 (m, 2H, N(C*H*₂CH₂)₂), 2.90 (dd, J = 15.6/6.8 Hz, 1H, C*H*₂CHOCH₃), 2.98 (dd, J = 15.4/3.4 Hz, 1 H, C*H*₂CHOCH₃), 3.55 (s, 3H, OC*H*₃), 4.85 (dd, J = 6.8/3.5 Hz, 1H, CH₂C*H*OCH₃), 7.15 - 7.21 (m, 3H, Phenyl-C*H*), 7.23 - 7.35 (m, 3H, Phenyl-C*H*), 7.42 - 7.48 (m, 4H, Phenyl-C*H*), 7.49 (s, 1 H, Pyrazole-3-C*H*).
**¹³C-NMR** (CDCl₃): δ (ppm) = 27.2, 29.7 (je 1C, NCH₂*C*H₂CH₂CH₂Ph), 31.2 (1C, *C*H₂CHOCH₃), 36.1 (1C, N*C*H₂CH₂CH₂CH₂Ph), 36.6 (1C, N(CH₂*C*H₂)₂), 39.4 (1C, N(CH₂*C*H₂)₂), 49.5 (1C, N(*C*H₂CH₂)₂), 49.7 (1C, N(*C*H₂CH₂)₂), 56.9 (1C, O*C*H₃), 59.0 (1C, NCH₂CH₂CH₂*C*H₂Ph), 71.8 (1C, Spiro-*C*), 77.5 (1C, Pyrazole-4-*C*), 96.9 (1C, CH₂*C*HOCH₃), 122.8 (2C, Phenyl-*C*H), 124.3 (1C, Phenyl-*C*, quartär), 125.9, 127.3, 128.5, 128.6, 129.5 (8C, Phenyl-*C*H), 133.7 (1C, Phenyl-*C*, quartär), 135.8 (1C, Pyrazole-3-*C*H), 139.5 (1C, Pyrazole-5-*C*).

### Example 19: 1-(Furan-2-ylmethyl)-6'-methoxy-1'-phenyl-6',7'-dihydro-1'H-spiro[piperidin-4,4'-pyrano[4,3-c]pyrazole]

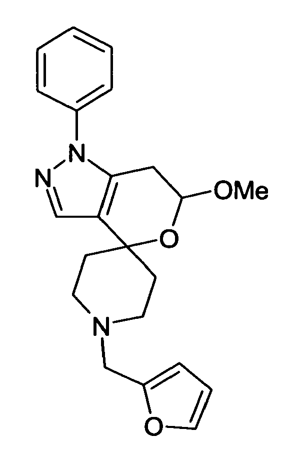

### Experimental procedure:

To a solution of crude example 6 (100 mg, 0.33 mmol) in abs. Dichloroethane (2 mL) were added consecutively freshly distilled Furan-2-carbaldehyde (27.7 µL, 0.33 mmol) and sodiumtriacetoxyborhydride (106.2 mg, 0.50 mmol). This reaction mixture was stirred for 7 h at room temperature. After addition of a saturated solution of NaHCO₃ (6 mL) and water (∼ 8 mL), it was extracted 3 times with CH₂Cl₂. Subsequently the pooled organic phases were dried over K₂CO₃ and filtered. After removal of the solvent under vacuum the crude product (112 mg), was prified using flash-chromatography (Ø=3 cm, h = 18 cm, n-hexane:ethylacetate 1:1 + 2% N,N-Dimethylethylamine, 20 mL, R_{f} = 0.33).
Colourless resin, Yield 99 mg (78 %).
C₂₂H₂₅N₃O₃ (379.5)

| | C | H | N |
|---|---|---|---|
| Calc. | 69.6 | 6.64 | 11.1 |
| Found | 69.1 | 6.66 | 10.6 |

**MS** (ESI): m/z (rel. Int.) = 380 [MH⁺, 100].
**IR** (neat): ν̃ (cm⁻¹) = 3111 (C-H _{aromat.}), 2919 (C-H _{aliphat.}), 2812 (C-H), 1598, 1504 (C=C), 1139, 1111 (C-O), 758, 695 (C-H).
**¹H-NMR** (CDCl₃): δ (ppm) = 1.91 - 2.07 (m, 3H, N(CH₂C*H*₂)₂), 2.14 (td, J = 12.9/4.4 Hz, 1 H, N(CH₂C*H*₂)₂), 2.50 (td, J = 11.5/2.9 Hz, 1 H, N(C*H*₂CH₂)₂), 2.59 (td, J = 11.7/2.4 Hz, 1H, N(C*H*₂CH₂)₂), 2.77 - 2.86 (m, 2H, N(C*H*₂CH₂)₂), 2.88 (dd, J = 15.7/7.0 Hz, 1H, C*H*₂CHOCH₃), 2.97 (dd, J = 15.7/3.1 Hz, 1 H, *C*H₂CHOCH₃), 3.53 (s, 3H, OC*H*₃), 3.61 (d, J = 14.1 Hz, 1 H, NC*H*₂Furyl), 3.65 (d, J = 14.1 Hz, 1 H, NC*H*₂Furyl), 4.84 (dd, J = 6.9/3.3 Hz, 1H, CH₂C*H*OCH₃), 6.24 (d, J = 2.7 Hz, 1H, Furan-3-CH), 6.32 - 6.36 (m, 1H, Furan-4-C*H*), 7.30 - 7.36 (m, 1H, Phenyl-CH, para), 7.39 - 7.50 (m, 6H, Phenyl-C*H* (4), Pyrazole-3-C*H* (1H), Furan-5-C*H* (1H)).
**¹³C-NMR** (CDCl₃): δ (ppm) = 31.2 (1C, *C*H₂CHOCH₃), 36.5 (1C, N(CH₂*C*H₂)₂), 39.3 (1C, N(CH₂*C*H₂)₂), 49.2 (1C, N(*C*H₂CH₂)₂), 49.3 (1C, N(*C*H₂CH₂)₂), 55.4 (1C, N*C*H₂Furyl), 56.9 (1C, O*C*H₃), 71.6 (1C, Spiro-*C*), 77.5 (1C, Pyrazole-4-*C*), 96.8 (1C, CH₂*C*HOCH₃), 109.1 (1C, Furan-3'-*C*H), 110.3 (1C, Furan-4'-*C*H), 122.8 (2C, Phenyl-*C*H, ortho), 124.3 (1C, Phenyl-*C*, quartär), 127.3 (1C, Phenyl-*C*H, para), 129.5 (2C, Phenyl-*C*H, meta), 133.8 (1C, Furan-2'-*C*), 135.8 (1C, Pyrazole-3-*C*H), 139.5 (1C, Pyrazole-5-*C*), 142.5 (1C, Furan-5'-*C*H).

### Example 20: 6'-Methoxy-1-(4-methoxybenzyl)-1'-phenyl-6',7'-dihydro-1'H-spiro[piperidin-4,4'-pyrano[4,3-c]pyrazole]

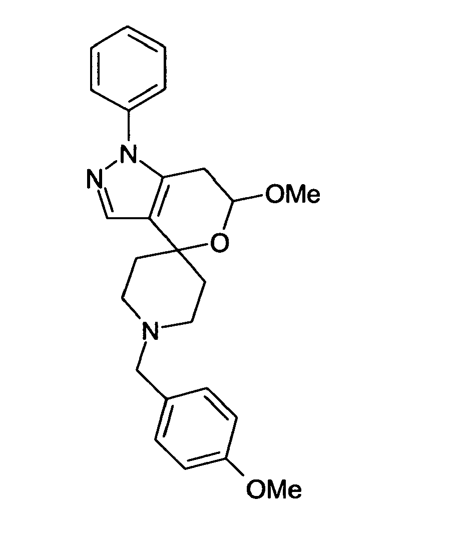

### Experimental procedure:

To a solution of crude example 6 (80 mg, 0.27 mmol) in abs. dichloroethane (1.5 mL) were added consecutively freshly distillied p-Methoxy-benzaldehyde (32.4 µL, 0.27 mmol) and sodiumtriacetoxyborhydride (84.9 mg, 0.40 mmol). This reaction mixture was stirred for 17 h at room temperature. After adding a saturated solution of NaHCO₃ (8 mL) and water (∼ 5 mL), it was extracted 3 times with CH₂Cl₂. Subsequently the pooled organic phases were dried over K₂CO₃ and filtered. Following removal of the solvent under vacuum the crude product (126 mg) was purified using flash-chromatography (Ø=3 cm, h = 20 cm, n-hexane:ethylacetate 1:1 + 2% N,N-Dimethylethylamine, 20 mL, R_{f} = 0.13).
Colourless resin, Yield 79 mg (70 %).
C₂₅H₂₉N₃O₃ (419.6)

| | C | H | N |
|---|---|---|---|
| Calc. | 71.6 | 6.97 | 10.0 |
| Found | 71.5 | 7.12. | 9.79 |

**MS** (ESI): m/z (rel. Int.) = 420 [MH⁺, 100], 838 [2 x M, 67].
**IR** (neat): ν̃ (cm⁻¹) = 2916 (C-H _{aliphat.}), 2833 (C-H), 1599, 1505 (C=C), 1114, 1059 (CO).
**¹H-NMR** (CDCl₃): δ (ppm) = 1.89 - 1.96 (m, 2H, N(CH₂C*H*₂)₂), 1.99 - 2.06 (m, 1H, N(CH₂C*H*_{2 axial})₂), 2.05 - 2.15 (m, 1 H, N(CH₂C*H*₂ₐₓᵢₐₗ)₂), 2.45 (t breit, J = 11.7 Hz, 1H, N(C*H*₂CH₂)₂), 2.55 (t breit, J = 11.7 Hz, 1H, N(C*H*₂CH₂)₂), 2.73 - 2.82 (m, 2H, N(C*H*₂CH₂)₂), 2.90 (dd, J = 15.7/6.3 Hz, 1H, C*H*₂CHOCH₃), 2.98 (dd, J = 15.7/3.9 Hz, 1 H, C*H*₂CHOCH₃), 3.55 (s, 5H, OC*H*₃ (3H), NCH₂Ph (2H)), 3.81 (s, 3H, PhOCH₃), 4.85 (dd, J = 6.7/3.5 Hz, 1H, CH₂C*H*OCH₃), 6.88 (d, J = 8.6 Hz, 2H, Benzyl-C*H*, ortho), 7.24 - 7.36 (m, 3H, Phenyl-C*H*, para (1 H), Benzyl-C*H*, meta (2H)), 7.41 - 7.48 (m, 4H, Phenyl-C*H*, ortho + meta), 7.50 (s, 1H, Pyrazole-3-C*H*).
¹³C-NMR (CDCl₃): δ (ppm) = 31.2 (1C, CH₂CHOCH₃), 36.7 (1C, N(CH₂*C*H₂)₂), 39.5 (1C, N(CH₂*C*H₂)₂), 49.3 (1C, N(*C*H₂CH₂)₂), 49.4 (1C, N(*C*H₂CH₂)₂), 55.5 (1C, PhenylO*C*H₃), 56.9 (1C, O*C*H₃), 63.0 (1C, N*C*H₂Ph), 71.9 (1C, Spiro-*C*), 77.5 (1C, Pyrazole-4-*C*), 96.8 (1C, CH₂*C*HOCH₃), 113.8 (2C, Benzyl-2',6'-*C*H), 114.2 (1C, Benzyl-4'-*C*, quartär), 122.8 (2C, Phenyl-*C*H, ortho), 124.4 (1C, Phenyl-*C*, quartär), 127.3 (1C, Phenyl-*C*H, para), 129.5 (2C, Phenyl-*C*H, meta), 130.7 (2C, Benzyl-3',5'-*C*H), 133.8 (1C, Benzyl-1'-*C*), 135.9 (1C, Pyrazole-3-*C*H), 139.5 (1C, Pyrazole-5-*C*).

### Example 21: 1-(4-Fluorbenzyl)-6'-methoxy-1'-phenyl-6',7'-dihydro-1'H-spiro[piperidin-4,4'-pyrano[4,3-c]pyrazole]

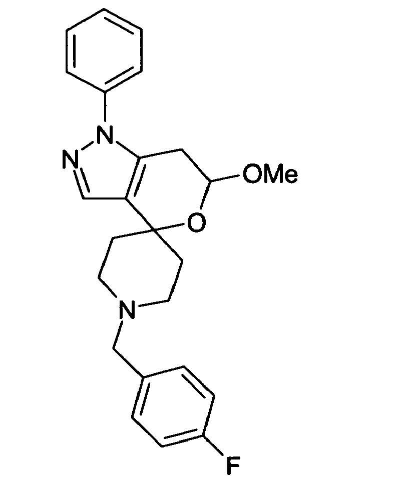

### Experimental procedure:

To a solution of crude example 6 (100 mg, 0.33 mmol) in abs. dichloroethane (2 mL) were added consecutively the freshly distilled p-fluorobenzaldehyde (36.0 µL, 0.33 mmol) and sodiumtriacetoxyborhydride (106.2 mg, 0.50 mmol). This reaction mixture was stirred for 19 h at room temperature. Following addition of a saturated solution of NaHCO₃-Solution (10 mL) and water (∼ 8 mL), it was extracted 3 times with CH₂Cl₂. Subsequently the pooled organic phases were dried over K₂CO₃ and filtered. After rermoval of th solvent under vacuum the crude product (145 mg) was purified using flash-chromatography (Ø=3 cm, h = 20 cm, n-hexane:ethylacetate 1:1 + 2% N,N-dimethylethylamine, 20 mL, R_{f} = 0.20).
Colourless solid, melting point: 158 °C, Yield 107 mg (79 %).
C₂₄H₂₆FN₃O₂ (407.5)

| | C | H | N |
|---|---|---|---|
| Calc. | 70.7 | 6.43 | 10.3 |
| Found | 70.5 | 6.34 | 10.3 |

**MS** (ESI): m/z (rel. Int.) = 408 [MH⁺, 100], 814 [2 x M, 13], 837 [2 x M + Na⁺].
**IR** (neat): ν̃ (cm⁻¹) = 3062 (C-H ₐᵣₒₘₐₜ.), 2941, 2917 (C-H ₐₗᵢₚₕₐₜ), 2814 (C-H), 1599, 1505 (C=C), 1113, 1058 (C-O).
**¹H-NMR** (CDCl₃): δ (ppm) = 1.86 - 1.98 (m, 2H, N(CH₂C*H*₂)₂), 2.00 - 2.06 (m, 1H, N(CH₂C*H*_{2 axial})₂), 2.09 (td, J = 12.9/4.4 Hz, 1 H, N(CH₂C*H*_{2 axial})₂), 2.45 (t breit, J = 11.0 Hz, 1H, N(C*H*₂CH₂)₂), 2.56 (t breit, J = 11.0 Hz, 1 H, N(C*H*₂CH₂)₂), 2.71 - 2.81 (m, 2H, N(C*H*₂CH₂)₂), 2.90 (dd, J = 15.3/6.7 Hz, 1H, C*H*₂CHOCH₃), 2.98 (dd, J = 15.3/3.5 Hz, 1H, C*H*₂CHOCH₃), 3.55 (s, 5H, OC*H*₃ (3H), NC*H*₂Ph (2H)), 4.86 (dd, J = 6.7/3.5 Hz, 1H, CH₂C*H*OCH₃), 7.03 (t, J = 8.6 Hz, 2H, Phenyl-C*H*), 7.30 - 7.38 (m, 3H, Phenyl-C*H*), 7.41 - 7.48 (m, 4H, Phenyl-C*H*), 7.50 (s, 1 H, Pyrazole-3-C*H*).
**¹³C-NMR** (CDCl₃): δ (ppm) = 31.2 (1C, *C*H₂CHOCH₃), 36.7 (1C, N(CH₂*C*H₂)₂), 39.4 (1C, N(CH₂*C*H₂)₂), 49.3 (1C, N(*C*H₂CH₂)₂), 49.4 (1C, N(*C*H₂CH₂)₂), 56.9 (1C, O*C*H₃), 62.7 (1C, N*C*H₂Ph), 71.8 (1C, Spiro-*C*), 77.4 (1C, Pyrazole-4-*C*), 96.8 (1C, CH₂*C*HOCH₃), 115.1, 115.3 (2C, Benzyl-2',6'-*C*H), 122.8 (2C, Phenyl-*C*H, ortho), 124.3 (1C, Benzyl-4'-*C*, quartär), 127.3 (1C, Phenyl-*C*H, para), 129.4 (2C, Phenyl-*C*H, meta), 130.9 (2C, Benzyl-3'5'-CH), 133.7 (1C, Phenyl-*C*, quartär), 134.3 (1C, Benzyl-1'-*C*, quartär), 135.8 (1C, Pyrazole-3-*C*H), 139.4 (1C, Pyrazole-5-*C*).

### Example H: 1-Methylpyrazole-5-carbaldehyde

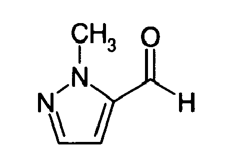

### Experimental procedure:

Under N₂-atmosphere a Solution of n-butyllithium in hexane (1.5 M, 35.7 mL, 53.6 mmol) was added slowly to a solution of 1-Methylpyrazole (4.0 g, 48.7 mmol) in abs. THF (70 mL), which was cooled to -78 °C with a mixture of dry ice and acetone. The reaction mixture was heated to 0 °C and stirred for 30 min. Subsequently it was again cooled cooled to -78 °C with a mixture of dry ice and acetone and abs. DMF (4.5 mL, 58.5 mmol) was slowly added. This mixture was stirred for 1 h at -78 °C, subsequently heated to 0 °C, stirred for a further 2 h, heated to room temperature and finally stirred for a further 16 h. Then it was hydrolysed with water (- 20 mL) and subsequently extreacted three times with CH₂Cl₂. The pooled organic phases were dried (K₂CO₃), filtered and the solvent was removed under vacuum (30 °C, 200 mbar) upto a volume of approx. 10 mL. The crude product was purified using flash-chromatography (Ø= 8 cm, h = 18 cm, n-Hexan:Ethylacetat = 7:3, 65 mL, R_{f} = 0.28).

Slightly yellow product, that was directly reacted further.
C₅H₆N₂O (110.1)
**MS** (ESI): m/z (rel.Int) = 110 [M⁺, 12].
**IR** (neat): ν̃ (cm⁻¹) = 2952, 2836 (C-H _{aliphat.}), 1684 (C=O).
**¹H-NMR** (CDCl₃): δ (ppm) = 4.18 (s, 3H, NC*H*₃), 6.89 (d, J = 5.0 Hz, 1H, Pyrazole-4-C*H*), 7.52 (d, J = 5.0 Hz, 1H, Pyrazole-3-C*H*), 9.83 (s, 1H, ArC*H*O).

In the spectrum the signals of the solvent (ethylacetate) are also visible.

### Example I: 5-(2-Methoxyvinyl)-1-methylpyrazole

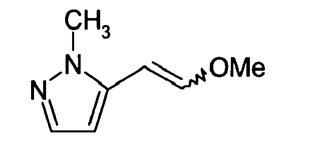

### Experimental procedure:

Under N₂-atmosphere dry MeOCH₂P⁺Ph₃Cl- (16.2 g, 47.2 mmol) was weight into and suspended in abs. THF (100 mL) for 30 minutes. Subsequently the suspension was cooled to -50 °C with a mixture of dry ice and acetone and subsequently a solution of KO^{t}Bu in THF (1M, 54.5 mL, 54.5 mmol) was added slowly. After a further 15 minutes of stirring, the aldehyde of Example H (4 g, 36.3 mmol) was dissolved in abs. THF (30 mL) and added dropwise at -50 °C. The reaction mixture was slowly brought to room temperature over night while stirring. After addition of water (∼ 40 mL) it was extracted 3 times with CH₂Cl₂. The pooled organic phases were dried over K₂CO₃, filtered and the solvent removed under vacuum (35 °C, 150 mbar) upto a volume of approx. 15 mL. The crude product was purified using flash-chromatography (Ø= 8 cm, h = 18 cm, n-hexane:ethylacetate = 5:5, 65 mL, R_{f} = 0.20).
Slightly yellow product, that was directly reacted further.
C₇H₁₀N₂O (138.2)
**MS** (ESI): m/z (rel.Int) = 139 [MH⁺, 22], 294 [2 x M + NH₄⁺, 100].
**IR** (neat): ν̃ (cm⁻¹) = 2940 (C-H _{aliphat.}), 1644 (C=C _{Alken}), 1100 (C-O).
**¹H-NMR** (CDCl₃): δ (ppm) = 3.70 (s, 3H, OC*H*₃, trans-Isomer), 3.79, 3.80, 3.81 (je s, 9H, NC*H*₃, cis-Isomer + trans-Isomer (6H), OC*H*₃ cis-Isomer (3H)), 5.23 (d, J = 6.7 Hz, 1H, C*H*=CHOCH₃, cis-Isomer), 5.60 (d, J = 12.9 Hz, 1H, C*H*=CHOCH₃, trans-Isomer), 6.11 (d, J = 1.6 Hz, 1H, Pyrazole-4-C*H*, trans-Isomer), 6.20 (d, J = 6.7 Hz, 1H, CH=C*H*OCH₃, cis-isomer), 6.55 (d, J = 1.6 Hz, 1H, Pyrazole-4-C*H*, cis-Isomer), 6.96 (d, J = 12.9 Hz, 1H, CH=C*H*OCH₃, trans-Isomer), 7.36 (d, J = 1.6 Hz, 1 H, Pyrazole-3-C*H*, trans-Isomer), 7.41 (d, J = 1.2 Hz, 1H, Pyrazole-3-C*H*, cis-Isomer).

In the spectrum the signals of the solvent (ethylacetate) are also visible. The ratio of the both isomers cis/trans is 2:3.

### Example J: 2-(4-Brom-1-methylpyrazole-5-yl)acetaldehyde-dimethylacetal

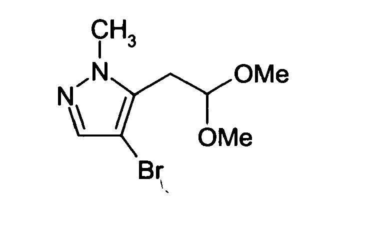

### Experimental procedure:

To a solution of Example I (3.0 g, 21.7 mmol) in abs. MeOH (50 mL) was added p-toluolsulphonic acid monohydrate (4.1 g, 21.7 mmol) and heated for 115 h under reflux. Subsequently HC(OCH₃)₃ (3.6 mL, 32.6 mmol) was added and cooled to 0 °C. After adding PBB (6.9 g, 21.7 mmol) it was stirred for 1 h at 0 °C and then a further 18 h at room temperature. Subsequently following addition of 2N NaOH (∼ 20 mL) and H₂O (∼ 15 mL) it was extracted 3 times with CH₂Cl₂. The pooled organic phases were dried over K₂CO₃, filtered and the solvent removed under lowered pressure. The crude product received was purified using flash-chromatography. (∅=8 cm, h = 18 cm, n-hexane:ethylacetate = 7:3, 85 mL, R_{f} = 0.22).

Colourless solid, melting point: 29 °C, Yield 3.5 g (29% in 4 steps, related to 4.0 g 1-Methylpyrazole).
C₈H₁₃BrN₂O₂ (249.1)
**MS** (ESI): m/z (rel.Int) = 249 [⁷⁹Br-MH⁺, 19], 251 [⁸¹Br-MH⁺, 18], 271 [⁷⁹Br-M + Na⁺, 97], 273 [⁸¹Br-M + Na⁺, 100], 520 [⁷⁹Br-2x MH + Na⁺, 86], 522 [⁸¹Br-2x MH + Na⁺, 43].
**IR** (neat): ν̃ (cm⁻¹) = 2936 (C-H _{aliphat.}), 2832 (C-H), 1118, 1070 (C-O).
**¹H-NMR** (CDCl₃): δ (ppm) = 2.96 (d, J = 5.5 Hz, 2H, C*H*₂CH(OCH₃)₂), 3.37 (s, 6H, (OC*H*₃)₂), 3.86 (s, 3H, NC*H*₃), 4.45 (t, J = 5.5 Hz, 1 H, CH₂C*H*(OCH₃)₂), 7.40 (s, 1 H, Pyrazole-3-C*H*).

### Example K: 2-[4-(1-Benzyl-4-hydroxypiperidin-4-yl)-1-methylpyrazole-5-yl]acetaldehyddimethylacetal

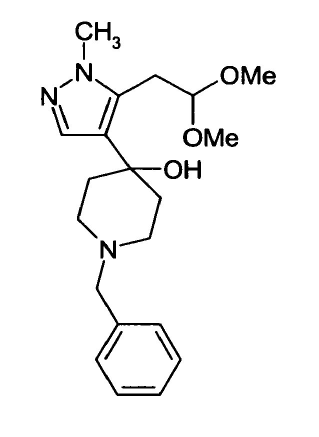

### Experimental procedure:

Under N₂-atmosphere n-butyl lithium in n-hexane (1.5 M , 5.9 mL, 8.83 mmol) was slowly added at -78 °C to a solution of the bromated Example J (2.0 g, 8.03 mmol) in abs THF (25 mL). The reaction mixture was stirred for 15 Minuten at -78 °C. Subsequently 1-Benzyl-piperidin-4-one (1.7 mL, 9.64 mmol) in abs. THF (4 mL) was added dropwise at -78 °C. After stirring for 4.5 h at -78 °C the mixture was heated to room temperature, stirred for a further 17 h stirred and subsequently diluted with water (∼ 12 mL) until no further precipitate is forming. Following extraction with CH₂Cl₂ the pooled organic phases were dried over K₂CO₃ and filtered. The solvent was removed under vacuum and the crude product (3.6 g) purified using flash-chromatography (Ø=8 cm, h = 15 cm, ethylacetate:n-hexane = 7:3 + 2% N,N-dimethylethylamine, 65 mL, R_{f} = 0.06).

Slightly yellow oil, that crystallises after storage in the fridge to a slightly yellow solid, melting point: 81°C, Yield 1.93 g (67%).
C₂₀H₂₉N₃O₃ (359.5)
**MS** (ESI): m/z (rel.Int) = 360 [MH⁺, 100].
**IR** (neat): ν̃ (cm⁻¹) = 3404 (O-H), 3060, 3027 (C-H _{aromat.}), 2938, 2829 (C-H), 1117, 1064 (C-O).
**¹H-NMR** (CDCl₃): δ (ppm) = 1.69 - 1.76 (m, 2H, N(CH₂C*H*₂)₂), 1.95 - 2.04 (m, 2H, N(CH₂C*H*₂)₂), 2.35 - 2.48 (m, 2H, N(C*H*₂CH₂)₂), 2.61 - 2.72 (m, 2H, N(C*H*₂CH₂)₂), 3.14 (d, J = 5.6 Hz, 2H, C*H*₂CH(OCH₃)₂), 3.18 (s breit, 1H, O*H*), 3.30 (s, 6H, CH(OC*H*₃)₂), 3.51 (s breit, 2H, NC*H*₂Phenyl), 3.74 (s, 3H, NC*H*₃), 4.42 (t, J = 5.6 Hz, 1H, CH₂C*H*(OCH₃)₂), 7.23 - 7.31 (m, 6H, Phenyl-CH (5H), Pyrazole-3-C*H* (1 H)).

### Example 22: 1-Benzyl-6'-methoxy-1'-methyl-6',7'-dihydro-1'H-spiro[piperidin-4,4'-pyrano[4,3-c]pyrazole]

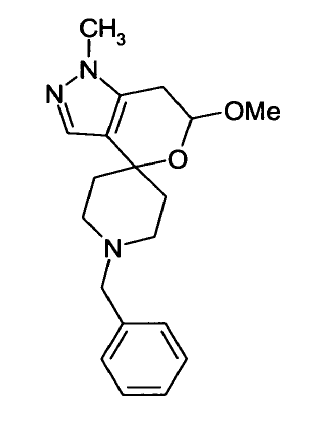

### Experimental procedure:

To a solution Example K (1.0 g, 2.8 mmol) in abs. methanol (30 mL) p-toluol sulphonic acid monohydrate (5.3 g, 27.8 mmol) was added and stirred at room temperature. After stirring for 24 h it was neutralised with a solution of NaOH (2 M), diluted with water and extracted 3 times with CH₂Cl₂. The pooled organic phases were dried over K₂CO₃, filtered and the solvent removed under lowered pressure. The crude product (1.3 g) was purified using flash-chromatography (Ø= 8 cm, h = 18 cm, n-hexane:ethylacetate 1:1 + 2% N,N-dimethylethylamine, 65 mL, R_{f} = 0.26 (EE + 2% N,N-dimethylethylamine)).
Colourless solid, melting point: 112 °C, Yield 374 mg (41 %).
C₁₉H₂₅N₃O₂ (327.5)

| | C | H | N |
|---|---|---|---|
| Calc. | 69.7 | 7.70 | 12.8 |
| Found | 69.4 | 7.70 | 13.0 |

**MS** (ESI): m/z (rel.Int) = 328 [MH⁺, 100].
**IR** (neat): ν̃ (cm⁻¹) = 3025 (C-H _{aromat.}), 2938, 2809 (C-H _{aliphat.}), 1576 (C=C), 1116, 1059, 1036 (C-O).
**¹H-NMR** (CDCl₃): δ (ppm) = 1.71 - 1.84 (m, 2H, N(CH₂C*H*₂)₂), 1.85 - 2.03 (m, 2H, N(CH₂C*H*₂)₂), 2.36 (td, J = 11.7/3.0 Hz, 1 H, N(C*H*₂CH₂)₂), 2.46 (td, J = 11.7/2.9 Hz, 1H, N(C*H*₂CH₂)₂), 2.57 (dd, J = 15.3/7.2 Hz, 1H, C*H*₂CHOCH₃), 2.62 - 2.72 (m, 2H, N(C*H*₂CH₂)₂), 2.77 (dd, J = 15.3/3.3 Hz, 1H, C*H*₂CHOCH₃), 3.49 (s, 3H, CH₂CHOC*H*₃), 3.67 (d, J = 13.2 Hz, 1H, NC*H*₂Phenyl), 3.70 (d, J = 13.2 Hz, 1H, NC*H*₂Phenyl), 3.66 (s, 3H, NC*H*₃), 4.82 (dd, J = 7.2/3.6 Hz, 1H, CH₂C*H*OCH₃), 7.20 - 7.31 (m, 6H, Phenyl-C*H* (5H), Pyrazole-3-C*H* (1H)).

### Example 23: 6'-Methoxy-1'-methyl-6',7'-dihydro-1'H-spiro[piperidin-4,4'-pyrano[4,3-c]pyrazole]

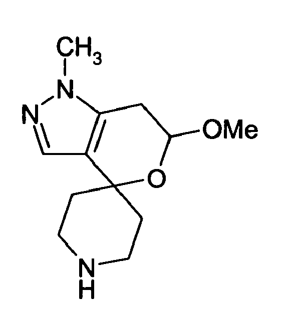

### Experimental procedure:

To a solution of Example 22 (360 mg, 1.09 mmol) in abs. methanol (10 mL) were added consecutively dried ammoniumformiate (347 mg, 5.50 mmol) and 10% Pd/C (28 mg) each in one dose respectively. Folowing that it was stirred for 25 minutes under reflux and subsequently the catalysator filtered over a fluted filter. After thorough rinsing with methanol the solvent was removed under vacuum. The crude product (208 mg) was redissolved in acetonitrile and following a further removal of the solvent under vacuum a yellow oil is formed, which is used in following reactions without further purification.
Yellow Oil, Yield 208 mg (96%).
C₁₂H₁₉N₃O₂ (237.3)
**MS** (ESI): m/z (rel.Int) = 238 [MH⁺, 100].
**IR** (neat): ν̃ (cm⁻¹) = 3308 (N-H), 2956, 2911 (C-H _{aliphat.}), 1061 (C-O).
**¹H-NMR** (CDCl₃): δ (ppm) = 1.89 - 2.02 (m, 2H, N(CH₂C*H₂*)₂), 2.03 - 2.29 (m, 2H, N(CH₂C*H*₂)₂), 2.64 (dd, J = 15.5/6.0 Hz, 1H, C*H*₂CHOCH₃), 2.81 (dd, J = 15.6/3.7 Hz, 1 H, C*H*₂CHOCH₃), 3.19 - 3.36 (m, 4H, N(C*H₂*CH₂)₂), 3.47 (s, 3H, OC*H*₃), 3.69 (s, 3H, NC*H*₃), 4.86 (dd, J = 6.0/3.8 Hz, 1H, CH₂C*H*OCH₃), 7.27 (s, 1H, Pyrazole-3-C*H*).

### Example 24: 6'-Methoxy-1'-methyl-1-(3-phenylpropyl)-6',7'-dihydro-1'H-spiro[piperidin-4,4'-pyrano[4,3-c]pyrazolel

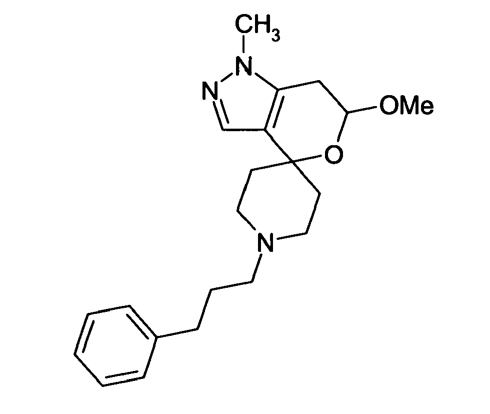

### Experimental procedure:

To a solution of crude example 23 (64 mg, 0.27 mmol) in acetonitrile (5 mL) 1-Bromo-3-phenylpropane (48.9 µL, 0.32 mmol) and K₂CO₃ (298.2 mg, 2.16 mmol) were added. This mixture was heated for 21 h under reflux. Subsequently it was filtered and the solvent removed under vacuum. The crude product (106 mg) was purified using flash-chromatography (Ø=2 cm, h = 18 cm, n-hexane:ethylacetate 3:7 + 2% N,N-dimethylethylamine, 10 mL, R_{f} = 0.13).

Colourless solid, melting point: 115 °C, Yield 74 mg (77%).
C₂₁H₂₉N₃O₂ (355.5)

| | C | H | N |
|---|---|---|---|
| Calc. | 71.0 | 8.22 | 11.8 |
| Found | 70.5 | 8.15 | 11.6 |

**MS** (ESI): m/z (rel.Int) = 356 [MH⁺, 100], 378 [M + Na⁺, 26].
**IR** (neat): ν̃ (cm⁻¹) = 3025 (C-H _{aromat.}), 2940 (C-H _{aliphat.}), 1116, 1059 (C-O).
**¹H-NMR** (CDCl₃): δ (ppm) = 1.73 - 1.86 (m, 4H, N(CH₂C*H*₂)₂ (2H), NCH₂C*H*₂CH₂Ph (2H)), 1.88 - 2.04 (m, 2H, N(CH₂C*H*₂)₂), 2.28 - 2.47 (m, 4H, N(C*H*₂CH₂)₂ (2H), NCH₂CH₂C*H*₂Ph (2H)), 2.54 - 2.64 (m, 3H, NC*H*₂CH₂CH₂Ph (2H), C*H*₂CHOCH₃ (1 H)), 2.68 - 2.76 (m, 2H, N(C*H*₂CH₂)₂), 2.78 (dd, J = 15.3/3.3 Hz, 1H, C*H*₂CHOCH₃), 3.50 (s, 3H, OC*H*₃), 3.69 (s, 3H, NC*H*₃), 4.82 (dd, J = 7.2/3.6 Hz, 1H, CH₂C*H*OCH₃), 7.09 - 7.26 (m, 6H, Phenyl-*C*H (5H), Pyrazole-3-C*H* (1 H)).

### Example 25: 1-(4-Fluorbenzyl)-6'-methoxy-1'-methyl-6',7'-dihydro-1'H-spiro[piperidin-4,4'-pyrano[4,3-c]pyrazole]

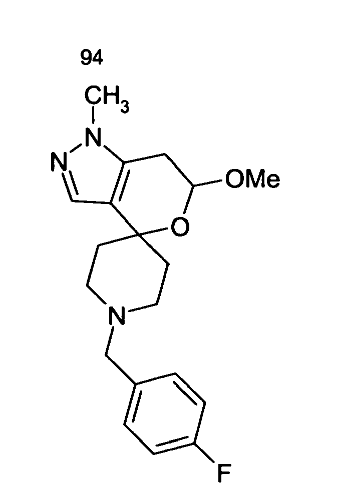

### Experimental procedure:

To a solution of crude example 23 (90 mg, 0.38 mmol) in abs. dichloroethane (2 mL) were added cosecutively freshly distilled p-fluorobenzaldehyde (40.9 µL, 0.38 mmol) and sodiumtriacetoxyborhydride (120.6 mg, 0.57 mmol). This reaction mixture was stirred for 19 h at room temperature. After addition of a saturated solution of NaHCO₃ (∼ 6 mL) and water (∼ 8 mL), it was extracted 3 times with CH₂Cl₂. Subsequently the pooled organic phases were dried over K₂CO₃ and filtered. Following removal of the solvent under vacuum the crude product (136 mg) was purified using flash-chromatography (Ø = 3.5 cm, h = 15 cm, n-hexane:ethylacetate 1:1 + 2% N,N-dimethylethylamine, 20 mL, R_{f} = 0.11).
Colourless solid, melting point: 127 °C, Yield 87 mg (66 %).
C₁₉H₂₄FN₃O₂ (345.5)

| | C | H | N |
|---|---|---|---|
| Calc. | 66.1 | 7.00 | 12.2 |
| Found | | | |

**MS** (ESI): m/z (rel. Int) = 346 [MH⁺, 100].
**IR** (neat): ν̃ (cm⁻¹) = 2941 (C-H _{aliphat.}), 1602, 1507 (C=C), 1115, 1059 (C-O).
**¹H-NMR** (CDCl₃): δ (ppm) = 1.70 - 1.82 (m, 2H, N(CH₂C*H*₂)₂), 1.85 - 2.02 (m, 2H, N(CH₂C*H*₂)₂), 2.35 (td, J = 11.5/2.8 Hz, 1 H, N(C*H*₂CH₂)₂), 2.45 (td, J = 11.7/2.9 Hz, 1 H, N(C*H*₂CH₂)₂), 2.57 (dd, J = 15.4/7.1 Hz, 1 H, C*H*₂CHOCH₃), 2.62 - 2.71 (m, 2H, N(C*H*₂CH₂)₂), 2.76 (dd, J = 15.4/3.5 Hz, 1H, C*H*₂CHOCH₃), 3.47 (s, 2H, NC*H*₂Ph), 3.49 (s, 3H, OC*H*₃), 3.66 (s, 3H, OC*H*₃), 4.81 (dd, J = 7.2/3.6 Hz, 1H, CH₂C*H*OCH₃), 6.94 (d, J = 8.7 Hz, 2H, Phenyl-C*H*), 7.21 - 7.29 (m, 3H, Phenyl-C*H* (2H), Pyrazole-3-C*H* (1H)).

### Example 26: 1-Isopentyl-6'-methoxy-1'-methyl-6',7'-dihydro-1'H-spiro[piperidin-4,4'-pyrano[4,3-c]pyrazole]

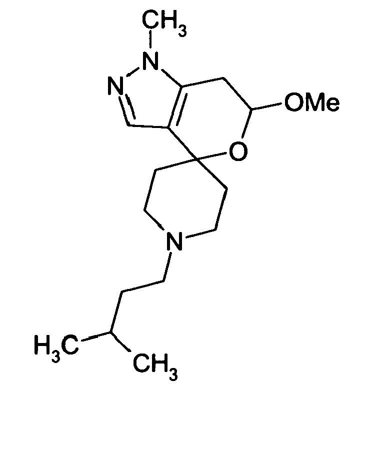

### Experimental procedure:

To a solution of crude example 23 (73 mg, 0.31 mmol) in abs. dichloroethane (3 mL) were added consecutively freshly distilled Isovaleraldehyde (32.8 µL, 0.31 mmol) and Sodiumtriacetoxyborhydride (97.1 mg, 0.46 mmol). This reaction mixture was stirred for 19 h at room temperature. After adding of a saturated solution of NaHCO₃ (∼ 6 mL) and water (∼ 5 mL), it was extracted 3 times with CH₂Cl₂. Subsequently the pooled organic phases were dried over K₂CO₃ and filtered. Folowing removal of the solvent under vacuum the crude product (114 mg), was prurified using flash-chromatography (∅ = 3.5 cm, h = 15 cm, n-hexane:ethylacetate 3:7 + 2% N,N-dimethylethylamine, 20 mL, R_{f} = 0.08).
Colourless solid, melting point: 85 °C, Yield 74 mg (79%).
C₁₇H₂₉N₃O₂ (307.5)

| | C | H | N |
|---|---|---|---|
| Calc. | 66.4 | 9.51 | 13.7 |
| Found | 66.1 | 9.52 | 13.3 |

**MS** (ESI): m/z (rel.Int) = 308 [MH⁺, 100].
**IR** (neat): ν̃ (cm⁻¹) = 2951 (C-H aliphat), 1060 (C-O).
**¹H-NMR** (CDCl₃): δ (ppm) = 0.77 (d, J = 6.6 Hz, 6H, NCH₂CH₂CH(C*H*₃)₂), 1.25 - 1.32 (m, 2H, NCH₂C*H*₂CH(CH₃)₂), 1.40 - 1.51 (m, 1H, NCH₂CH₂C*H*(CH₃)₂), 1.66 - 1.75 (m, 2H, N(CH₂C*H*₂)₂), 1.80 - 1.91 (m, 2H, N(CH₂C*H*₂)₂), 2.20 - 2.30 (m, 3H, N(C*H*₂CH₂)₂) (1H), NC*H*₂CH₂CH(CH₃)₂) (2H)), 2.32 (td, J = 11.7/2.5 Hz, 1H, N(C*H*₂CH₂)₂), 2.50 (dd, J = 15.3/7.1 Hz, 1H, C*H*₂CHOCH₃), 2.59 - 2.66 (m, 2H, N(C*H*₂CH₂)₂), 2.68 (dd, J = 15.3/3.6 Hz, 1H, C*H*₂CHOCH₃), 3.42 (s, 3H, OC*H*₃), 3.58 (s, 3H, NC*H*₃), 4.74 (dd, J = 7.1 /3.6 Hz, 1H, CH₂C*H*OCH₃), 7.12 (s, 1 H, Pyrazole-3-C*H*).

### Example 27: 6'-Methoxy-1'-methyl-1-propyl-6',7'-dihydro-1'H-spiro[piperidin-4,4'-pyrano[4,3-c]pyrazole]

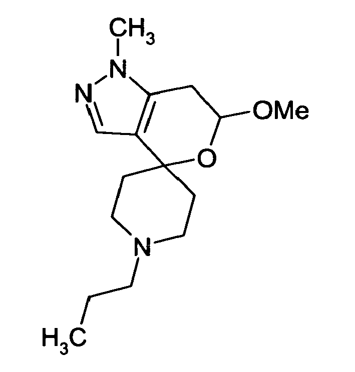

### Experimental procedure:

To a solution of crude example 23 (90 mg, 0.38 mmol) in acetonitrile (5 mL) 1-bromopropane (44.8 µL, 0.49 mmol) and K₂CO₃ (419.3 mg, 3.03 mmol) were added. This mixture was heated for 47 h under reflux. Subsequently the K₂CO₃ was filtered off and the solvent removed under vacuum. The crude product (86 mg) was purified using flash-chromatography (Ø = 2.5 cm, h = 15 cm, n-hexane:ethylacetate 3:7 + 2% N,N-dimethylethylamine, 10 mL, R_{f} = 0.11).
Colourless solid, melting point: 94 °C, Yield 63 mg (60 %).
C₁₅H₂₅N₃O₂ (279.4)

| | C | H | N |
|---|---|---|---|
| Calc. | 64.5 | 9.02 | 15.0 |
| Found | 64.2 | 8.95 | 14.6 |

**MS** (ESI): m/z (rel.Int) = 280 [MH⁺, 100].
**IR** (neat): ν̃ (cm⁻¹) = 2948 (C-H _{aliphat.}), 2873 (C-H), 1122, 1057 (C-O).
**¹H-NMR** (CDCl₃): δ (ppm) = 0.86 (t, J = 7.3 Hz, 3H, NCH₂CH₂C*H*₃), 1.42 - 1.56 (m, 2H, NCH₂C*H*₂CH₃), 1.73 - 1.85 (m, 2H, N(CH₂C*H*₂)₂), 1.88 - 2.04 (m, 2H, N(CH₂C*H*₂)₂), 2.26 - 2.34 (m, 3H, N(C*H*₂CH₂)₂) (1 H), NC*H*₂CH₂CH₃) (2H)), 2.40 (td, J = 12.0/2.7 Hz, 1H, N(C*H*₂CH₂)₂), 2.58 (dd, J = 15.5/7.2 Hz, 1H, C*H*₂CHOCH₃), 2.66 - 2.74 (m, 2H, N(C*H*₂CH₂)₂), 2.77 (dd, J = 15.5/3.6 Hz, 1H, C*H*₂CHOCH₃), 3.51 (s, 3H, OC*H*₃), 3.67 (s, 3H, NC*H*₃), 4.82 (dd, J = 7.2/3.3 Hz, 1H, CH₂C*H*OCH₃), 7.19 (s, 1H, Pyrazole-3-C*H*).

### Example 28: 1 -Benzyl-1'-methyl-6'.7'-dihydro-1'H-spiro[piperidin-4,4'-pyrano[4,3-c]pyrazole]-6'-ol

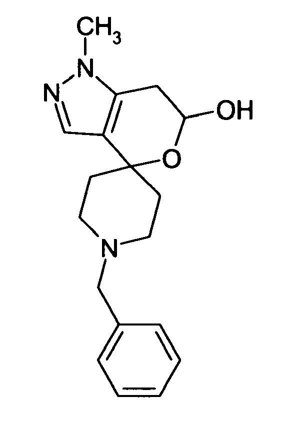

### Experimental procedure:

A solution of Example K (1.0 g, 2.78 mmol) in aqueous HCl (2 N, 60 mL) was stirred for 47 h at room temperature. Subsequently it was neutralised with ∼ 60 mL of a solution of NaOH (2 N), diluted with water and exrtracted 3 times with CH₂Cl₂. The pooled organic phases were dried over K₂CO₃, filtered and the solvent removed under lowered pressure. The crude product (1.01 g) was purified using flash-chromatography (∅=6 cm, h = 15 cm, ethylacetate + 2% N,N-dimethylethylamine, 65 mL, R_{f}= 0.08).
Colourless solid, melting point: 165 °C, Yield 673 mg (77 %).
C₁₈H₂₃N₃O₂ (313.4)

| | C | H | N |
|---|---|---|---|
| Calc. | 69.0 | 7.40 | 13.4 |
| Found | 68.7 | 7.36 | 12.9 |

**MS** (ESI): m/z (rel.Int) = 313 [M⁺, 18], 312 [(M - H)⁺, 100].
**IR** (neat): ν̃ (cm⁻¹) = 3072 (O-H), 2921 (C-H _{aliphat.}), 2835 (C-H), 1109, 1043 (C-O).
**¹H-NMR** (CDCl₃): δ (ppm) = 1.70 - 1.86 (m, 2H, N(CH₂C*H*₂)₂), 1.87 - 2.03 (m, 2H, N(CH₂C*H*₂)₂), 2.36 (td, J = 11.4/3.1 Hz, 1H, N(C*H*₂CH₂)₂), 2.45 (td, J = 11.6/2.9 Hz, 1 H, N(C*H*₂CH₂)₂), 2.55 (dd, J = 15.3/7.2 Hz, 1 H, C*H*₂CHOCH₃), 2.59 - 2.70 (m, 2H, N(C*H*₂CH₂)₂), 2.83 (dd, J = 15.3/3.3 Hz, 1 H, C*H*₂CHOCH₃), 3.61 (d, J = 14.1 Hz, 1H, NC*H*₂Ph), 2.64 (d, J = 14.3 Hz, 1H, NC*H*₂Ph), 3.68 (s, 3H, NC*H*₃), 5.27 (dd, J = 7.3/3.5 Hz, 1H, CH₂C*H*OCH₃), 7.17 - 7.30 (m, 6H, Phenyl-C*H* (5H), Pyrazole-3-C*H* (1 H)).

### Example 29: 1-Benzyl-1'-methyl-1'H-spiro[piperidin-4,4'-pyrano[4,3-c]pyrazole]

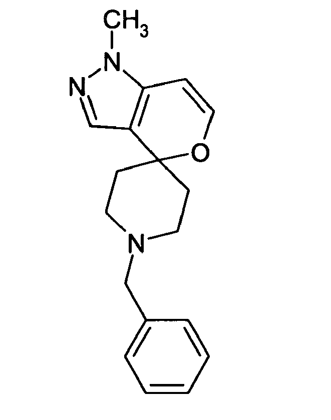

### Experimental procedure:

Example 28 (400 mg, 1.28 mmol) was dissolved in abs. CH₂Cl₂ (18 mL) under N₂-atmosphere. Subsequently it was cooled to 0 °C and consecutively NEt₃ (425.7 µL, 3.06 mmol) and MeSO₂Cl (118.9 µL, 1.53 mmol) were slowly added. The reaction solution was stirred for 2 h at room temperature and subsequently for 1 h under reflux, before being neutralised with a saturated solution of NaHCO₃ (~16 mL) and extrated for 3 times with CH₂Cl₂. The pooled organic phases were dried over K₂CO₃, filtered and the solvent removed in vacuum. The crude product (415 mg) was purified using flash-chromatography (Ø= 4 cm, h = 15 cm, n-hexane:ethylacetate 3:7 + 2% N,N-dimethylethylamine, 30 mL, R_{f} = 0.21).
Colourless solid, melting point: 115 °C, Yield 296 mg (79%).
C₁₈H₂₁N₃O (295.4)

| | C | H | N |
|---|---|---|---|
| Calc. | 73.2 | 7.17 | 14.2 |
| Found | 73.0 | 6.91 | 14.1 |

**MS** (ESI): m/z (rel.Int) = 296 [MH⁺, 100].
**IR** (neat): ν̃ (cm⁻¹) = 3091, 3006 (C-H _{aromat.}), 2961, 2939, 2916 (C-H ₐₗᵢₚₕₐₜ.), 1611 (C=C), 1045 (C-O).
**¹H-NMR** (CDCl₃): δ (ppm) = 1.83 (t, J = 11.2 Hz, 2H, N(CH₂C*H*₂)₂), 2.02 - 2.11 (m, 2H, N(CH₂C*H*₂)₂), 2.37 (t, J = 11.2 Hz, 2H, N(C*H*₂CH₂)₂), 2.57 - 2.66 (m, 2H, N(C*H*₂CH₂)₂), 3.50 (s, 2H, NC*H*₂Ph), 3.67 (s, 3H, NC*H*₃), 5.56 (dd, J = 6.0/0.7 Hz, 1H, ArC*H*=CH), 6.40 (d, J = 6.0 Hz, 1H, ArCH=C*H*), 7.08 (d, J = 0.7 Hz, 1H, Pyrazole-3-C*H*), 7.17 - 7.31 (m, 5H, Phenyl-C*H*).

### Example 30: 1-Benzyl-1'-methyl-6',7'-dihydro-1'H-spirori[piperidin-4,4'-pyrano[4,3-c]pyrazole]

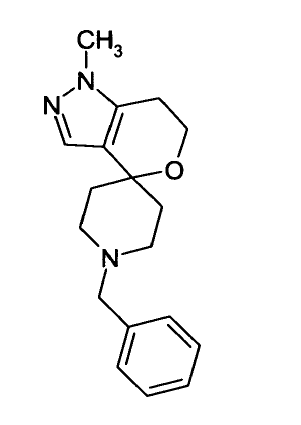

### Experimental procedure:

50 mg (0.17 mmol) of Example 29 were dissolved in glacial acetic acid (5 mL) and 10 % Pd/C (35 mg) added. It was stirred for 1h at room temperature under H₂-atmosphere (Ballon), filtered through a fluted filter and thoroughly rinsed with HCl (2N) and H₂O. Subsequently the filtrate was strongly alkalized with a solution of NaOH (2N) and extracted with CH₂Cl₂. The pooled organic phases were dried over K₂CO₃, filtered and the solvent removed under vacuum abdestilliert. The crude product (52 mg) was purified using flash-chromatography (Ø=2 cm, h = 15 cm, n-hexane:ethylacetate 1:1 + 2% N,N-dimethylethylamine, 10 mL, R_{f} = 0.09).
Colourless oil, Yield 37 mg (74 %).
C₁₈H₂₃N₃O (297.4)

| | C | H | N |
|---|---|---|---|
| Calc. | 72.7 | 7.80 | 14.1 |
| Found | 72.4 | 7.88 | 13.9 |

**MS** (ESI): m/z (rel.Int) = 298 [MH⁺, 100].
**IR** (neat): ν̃ (cm⁻¹) = 3026 (C-H _{aromat.}), 2936 (C-H _{aliphat.}), 2810 (C-H), 1577, 1508 (C=C), 1070 (C-O).
**¹H-NMR** (CDCl₃): δ (ppm) = 1.81 - 1.96 (m, 4H, N(CH₂C*H*₂)₂), 2.41 (td, J = 11.5/3.1 Hz, 2H, N(C*H*₂CH₂)₂), 2.62 (t, J = 5.7 Hz, 2H, C*H*₂CH₂O), 2.69 (dt, J = 11.7/3.9 Hz, 2H, N(C*H*₂CH₂)₂), 3.57 (s, 2H, NC*H*₂Ph), 3.74 (s, 3H, NC*H*₃), 3.91 (t, J = 5.7 Hz, 2H, CH₂C*H*₂O), 7.23 - 7.28 (m, 1H, Phenyl-C*H*, para), 7.29 - 7.37 (m, 5H, Phenyl-C*H* (4H), Pyrazole-3-C*H* (1H)).

### Example 31: 1'-Methyl-6',7'-dihydro-1'H-spiro[piperidin-4,4'-pyrano[4,3-c]pyrazole]

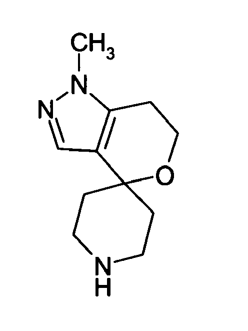

### Experimental procedure:

To a solution of Example 30 (345 mg, 1.16 mmol) in abs. methanol (15 mL) were added consecutively dried ammoniumformiate (365.8 mg, 5.80 mmol) and 10% Pd/C (27.6 mg) each in one dose respectively. Subsequently it was stirred for 25 minutes under reflux and following that the catalysator filtered off through a fluted filter. After thorough rinsing with methanol the solvent was removed under vacuum. The crude product (colourless solid) was subsequently redissolved in H₂O and extracted with CH₂Cl₂ : MeOH 2:1. After drying the poled organic phases over K₂CO₃ the solvent was again removed under vacuum. A yellow oil was formed, which was used in following reactions without further purification.
Yellow oil, R_{f} = 0.09 (MeOH + 2 % NH₃ conc.), Yield 216 mg (90 %).
C₁₁H₁₇N₃O (207.3)
**MS** (ESI): m/z (rel.Int) = 208 [MH⁺, 100].
**IR** (neat): ν̃ (cm⁻¹) = 2967 (C-H _{aliphat.}), 1510 (N-H), 1066 (C-O).
**¹H-NMR** (CDCl₃): δ (ppm) = 1.75 - 1.88 (m, 4H, N(CH₂C*H*₂)₂), 2.63 (t, J = 5.5 Hz, 2H, ArC*H*₂CH₂O), 2.92 (dt, J = 11.7/3.9 Hz, 2H, N(C*H*₂CH₂)₂), 3.04 (td, J = 11.7/3.9 Hz, 2H, N(C*H*₂CH₂)₂), 3.75 (s, 3H, NC*H*₃), 3.92 (t, J = 5.5 Hz, 2H, ArCH₂C*H*₂O), 7.28 (s, 1 H, Pyrazole-3-C*H*).

In addition more compounds according to the invention are synthesized using the method of Reaction Scheme B in an analoguous way as in examples 7 to 21. This reaction and the resulting compounds (examples 31, 32 and 33) are described in Scheme E: 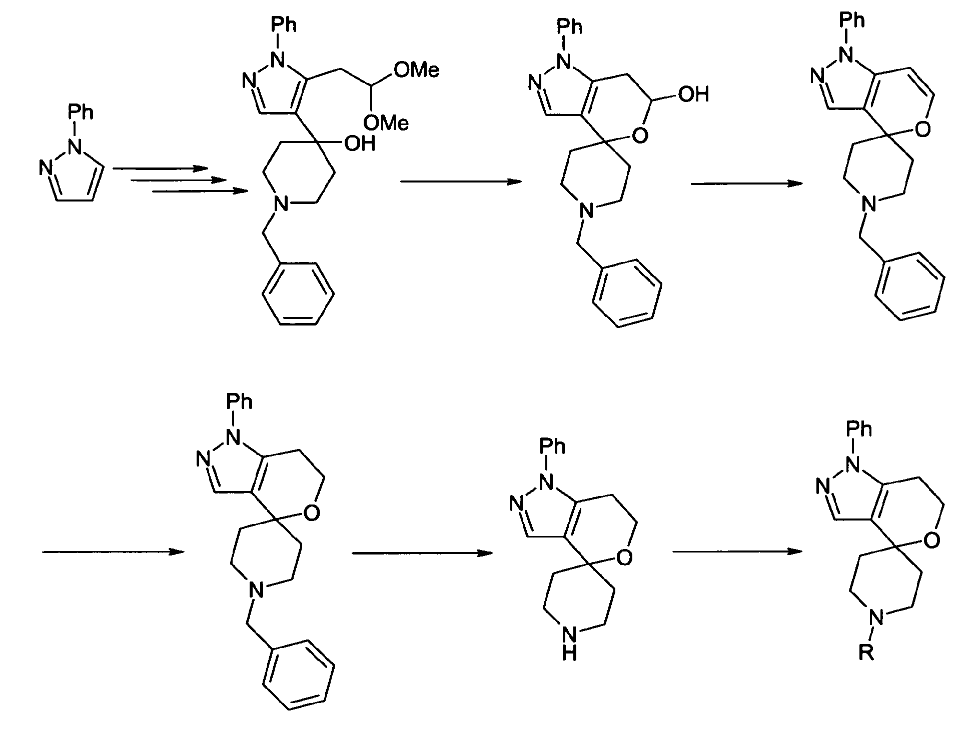 with R being here either:

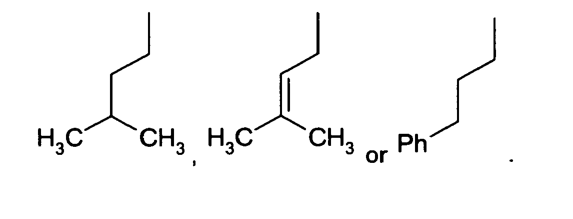

In addition more compounds according to the invention are synthesized using the method of Reaction Schemes C and D in an analoguous way as in examples 24 to 30. This reaction and the resulting compounds (examples 34, 35, and 36) are described in Scheme F: 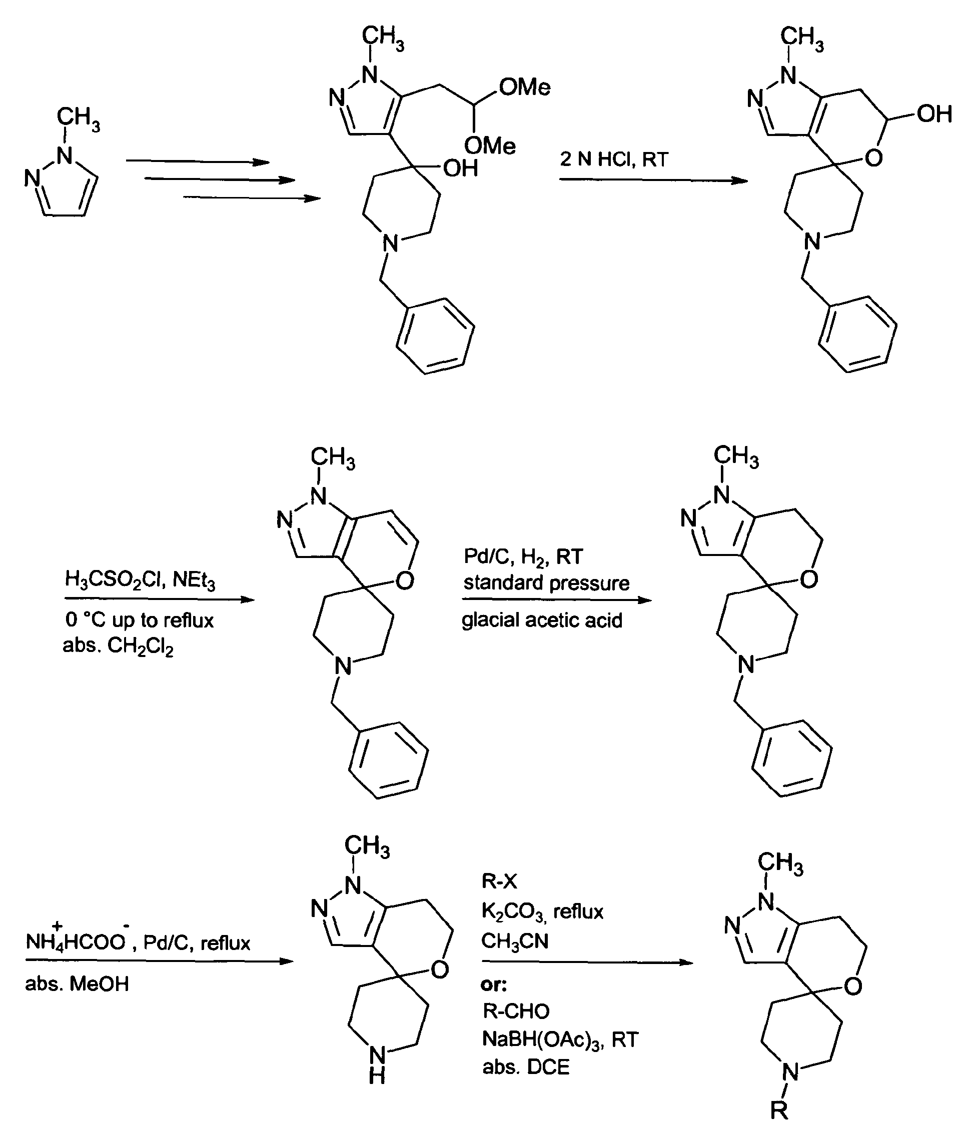 with R being here either:

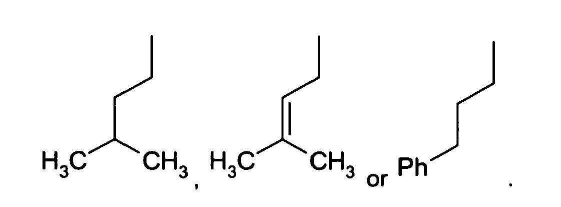

### BIOLOGICAL ACTIVITY

### A) In-Vitro

Some representative compounds of the invention were tested for their activity as sigma (sigma-1 and sigma-2) inhibitors. The following protocols were followed:

### Sigma-1 (Version A)

Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use. Each assay tube contained 10 µL of [³H](+)-pentazocine (final concentration of 0.5 nM), 900 µL of the tissue suspension to a final assay volume of 1 mL and a final tissue concentration of approximately 30 mg tissue net weight/mL. Non-specific binding was defined by addition of a final concentration of 1 µM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

### Sigma 1 (Version B)

In brief the a,-receptor preparation was prepared from guinea pig brain. The brains were homogenized in 5 to 6 times of volume sucrose solution (0.32M) and homogenized. The homogenate was centrifuged at 2900 rpm, 4°C, 10 min). the supernatant was centrifuged again (23500 x g, 4°C, 20 min). The pellet was resuspended in Tris-buffer, incubated for 30 min at room temperature and centrifuged f (23500 x g, 4°C. 20 min). The pellet was resuspended in cold TRIS-buffer and homogenized. Then the protein content was measured (approx. 1.5 mg/mL) and the homogenate frozen at -80°C for later use.

The radioligand used was [³H]-(+)-Pentazocin in TRIS-buffer. In a volume of 200 µL 50 µL TRIS-buffer, 50 µL compound solution of varying concentration, 50 µL radioligand- solution (8 nM; resulting in 2 nM in the assay) and finally 50 µL of receptor preparation (approx. 1.5 mg /mL) were given into a well of a microplate equipped with a filter. The plate was closed and stirred for 2.5 h at 37 °C and 500 rpm. Following that the solvents were removed by a harvester through the filter. After rinsing with H₂O the filter was measured in a scintillation counter ([³H]-protocol).

### Sigma-2 (Version A)

Binding studies for σ2-receptor were performed as described (Radesca et al., 1991) with some modifications. In brief, brains from sigma receptor type I (σ1) knockout mice were homogenized in a volume of 10 mL/g tissue net weight of ice-cold 10 mM Tris-HCl, pH 7.4, containing 320 mM sucrose (Tris-sucrose buffer) with a Potter-Elvehjem homogenizer (10 strokes at 500 r.p.m.) The homogenates were then centrifuged at 1000g for 10 min at 4°C, and the supernatants were saved. The pellets were resuspended by vortexing in 2 mUg ice-cold Tris-sucrose buffer and centrifuged again at 1000g for 10 min. The combined 1000g supernatants were centrifuged at 31000g for 15 min at 4°C. The pellets were resuspended by vortexing in 3 mUg 10 mM Tris-HCl, pH 7.4, and the suspension was kept at 25°C for 15 min. Following centrifugation at 31000g for 15 min, the pellets were resuspended by gentle Potter Elvehjem homogenization to a volume of 1.53 mUg in 10 mM Tris-HCl pH 7.4.

The assay tubes contained 10 µL of [³H]-DTG (final concentration of 3 nM), 400 µL of the tissue suspension (5.3 mUg in 50 mM Tris-HCl, pH 8.0) to a final assay volume of 0.5 mL. Non-specific binding was defined by addition of a final concentration of 1 µM haloperidol. All tubes were incubated at 25°C for 120 min before termination of the reaction by rapid filtration over Schleicher & Schuell GF 3362 glass fibre filters [previously soaked in a solution of 0,5% polyethylenimine for at least 1 h]. Filters were washed with three times with 5 mL volumes of cold Tris-HCl buffer (10 mM, pH 8.0). Following addition of scintillation cocktail samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

### References

DeHaven-Hudkins, D. L., L.C. Fleissner, and F. Y. Ford-Rice, 1992, "Characterization of the binding of [3H](+)pentazocine to σ recognition sites in guinea pig brain", Eur. J. Pharmacol. 227, 371-378.
Radesca, L., W.D. Bowen, and L. Di Paolo, B.R. de Costa, 1991, Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands, J. Med. Chem. 34, 3065-3074.
Langa, F., Codony X., Tovar V., Lavado A., Giménez E., Cozar P., Cantero M., Dordal A., Hernández E., Pérez R., Monroy X., Zamanillo D., Guitart X., Montoliu LI., 2003, Generation and phenotypic analysis of sigma receptor type I (Sigma1) knockout mice, European Journal of Neuroscience, Vol. 18, 2188-2196.
Lowry, O.H., N.J. Rosebrough, A.L. Farr, and R.J. Randall, 1951, Protein measurement with the Folin phenol reagent, J. Biol. Chem, 193, 265.

### SIGMA 2 (Version B)

In brief the σ₂-Receptorpreparation was prepared from rat liver. The livers were homogenized in 5 to 6 times of volume sucrose solution (0.32M) and homogenized.

The homogenate was centrifuged at 2900 rpm, 4°C, 10 min). the supernatant was centrifuged again (31000 x g, 4°C, 20 min). The pellet was resuspended in TRIS-buffer, incubated for 30 min at room temperature while stirring and centrifuged f (31000 x g, 4°C, 20 min). The pellet was resuspended in cold TRIS-buffer pH 8 and homogenized. Then the protein content was measured (approx. 2 mg/mL) and the homogenate frozen at -80°C for later use.

The radioligand used was [³H]-Ditolylguanidin in TRIS-buffer pH 8. The σ₁-receptor binding sites were masked through (+)-Pentazocin-solution in TRIS-Puffer pH 8.

In a volume of 200 *µ*L 50 *µ*L compound solution of varying concentration, 50 *µ*L (+)-Pentazocin- solution (2 *µ*M; resulting in 500 nM in the assay), 50 *µ*L radioligand-solution (12 nM; resulting in 3 nM in the assay) and finally 50 *µ*L of receptor preparation (approx. 2 mg /mL) were given into a well of a microplate equipped with a filter. The plate was closed and stirred for 2 h at room temperature and 500 rpm. Following that the solvents were removed by a harvester through the filter. After rinsing with H₂O the filter was measured in a scintillation counter ([³H]-protocol).

Some of the results obtained (through the versions B) are shown in table (I).

**Table (I)**

| **Example** | **Binding σ1 Ki [nM]** | **Binding σ2 Ki [nM] or [Inhibition at 1µM]** |
|---|---|---|
| **2** | 1200 | 12% |
| **3** | 0.50 +/- 0.218 | 1750 |
| **4** | 1.28 +/- 0.28 | 1050 |
| **5** | 1,46 +/- 0,14 | 22% |
| **7** | 33,3 +/- 7,4 | 3030 |
| **8** | 210 +/- 61 | 3210 |
| **9** | 8.04 +/- 3.81 | 752 |
| **10** | 6,32 +/- 1,82 | 933 |
| **11** | 0,82 +/- 0,11 | 340 +/- 56 |
| **12** | 0,97 +/- 0,27 | 316 +/- 96 |
| **13** | 1,00 | - |
| **14** | 2.86 +/- 0.81 | 209 +/- 38 |
| **15** | 0.363 | - |
| **16** | 2,73 +/- 0,93 | 570 |
| **17** | 4.92 | 833 |
| **18** | 3.22 +/- 0.65 | 428 |
| **19** | 2,21 +/- 0,64 | 5220 |
| **20** | 1,54 +/- 0,50 | 925 |
| **21** | 0.94 +/- 0.41 | 687 |
| **22** | 21.4+/-4.7 | - |
| **24** | 96.2 | - |
| **25** | 23.3 | 46 % |
| **26** | 26.4 | - |
| **27** | 2980 | - |
| **28** | 190+/-13 | - |
| **29** | 15.2 | - |
| **30** | 9.48 | 191 |

### B) In-Vivo

### EFFECT ON CAPSAICIN IN DEVELOPMENT OF MECHANICAL ALLODYNIA

This model uses the von-Frey Filaments and is a model to test the effects or symptoms of neuropathic pain, allodynia etc.

Interest of the model:
- The injection of 1 µg of capsaicin to experimental animals produces acute pain followed by hyperalgesia/allodynia
- The mechanisms involved in capsaicin-induced acute pain and hyperalgesia are relatively well known (mainly activation of peripheral nociceptors and sensitization of spinal cord neurons, respectively)

The test protocol for all tests with of Frey filaments: After habituation mice were first treated with the test-compound (or solvent in controls). Then 1 µg capsaicin (1% DMSO) is injected into their paw resulting in developing pain in the effected paw. The effected paw is then treated with a mechanical stimulus and the latency time before the paw is withdrawn is measured.

## Claims

1. Compound of general formula (I), 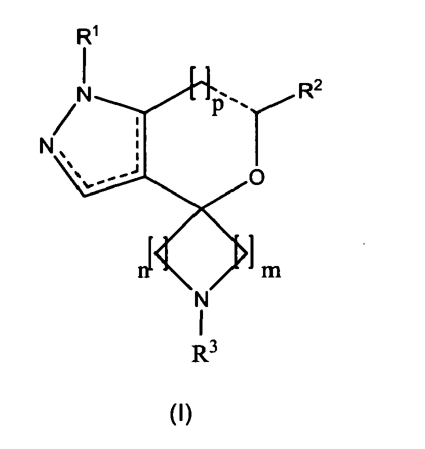 wherein
m is selected from 1, 2 or 3, n is selected from 1, 2 or 3, and m + n is either 3, 4 or 5;
p is selected from 0 or 1;
the dotted line ......... is either a double or a single bond;
if p is 1, the dotted line ......... is either a double or a single bond;
if p is 0, the dotted line ......... is a single bond;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; O-R with R being H or an optionally at least monosubstituted, linear or branched C₁₋₆-aliphatic group;
R³ is selected from hydrogen; an optionally at least monosubstituted, linear or branched C₁₋₁₈-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl; or COOR' with R' being either H or C₁₋₄-alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

2. Compound according to claim 1, **characterized in that** R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferably R¹ is selected from CH₃ or phenyl.

3. Compound according to any of claims 1 or 2, **characterized in that** R² is selected from H; or OR with R being H or an optionally at least monosubstituted, linear or branched C₁₋₆-aliphatic group; especially R² is selected from H or OR with R being H or a linear or branched C₁₋₄-alkyl group, more preferably R² is selected from H, OH or OCH₃.

4. Compound according to any of claims 1 to 3, **characterized in that** R³ is selected from H; an optionally at least monosubstituted, linear or branched C₁₋₁₈-aliphatic group; an optionally at least monosubstituted alkyl-aryl; an optionally at least monosubstituted alkyl-heterocyclyl; or an optionally at least monosubstituted alkyl-cycloalkyl.

5. Compound according to any of claims 1 to 4, **characterized in that** m and n are selected from 1 or 2 and m+n are selected from 3 or 4.

6. Compound according to claim 1, having a general formula la, 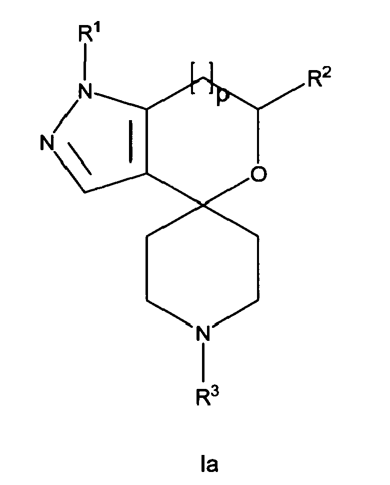 wherein
p is selected from 0 or 1;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; or OR with R being H or an optionally at least monosubstituted, linear or branched C₁₋₆-aliphatic group;
R³ is selected from hydrogen; an optionally at least monosubstituted, linear or branched C₁₋₁₈-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; an optionally at least monosubstituted alkyl-cycloalkyl; or COOR' with R' being either H or C₁₋₄-alkyl.

7. Compound according to claim 6, **characterized in that** R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferably R¹ is selected from CH₃ or phenyl.

8. Compound according to any of claims 6 or 7, **characterized in that** R² is selected from H; OR with R being H or an optionally at least monosubstituted, linear or branched C₁₋₆-alkyl group; especially R² is selected from H; OH or a linear or branched OC₁₋₄-alkyl group; more preferably R² is selected from H, OH or OCH₃.

9. Compound according to any of claims 6 to 8, **characterized in that** R³ is selected from H; an optionally at least monosubstituted, linear or branched C₁₋₁₈-aliphatic group; an optionally at least monosubstituted alkyl-aryl; an optionally at least monosubstituted alkyl-heterocyclyl; or an optionally at least monosubstituted alkyl-cycloalkyl.

10. Compound according to any of claims 6 to 9, **characterized in that** R³ is selected from
hydrogen, C₁₋₁₀-alkyl, linear or branched; C₁₋₁₀-alkenyl, linear or branched; optionally at least mono-substituted C₁₋₆-alkyl-aryl; optionally at least mono-substituted C₁₋₆-alkyl-heterocyclyl; or optionally at least mono-substituted C₁₋₆-alkyl-cycloalkyl;
preferably R³ is selected from hydrogen; C₁₋₁₀-alkyl, linear or branched; C₃₋₈-alkenyl, linear or branched; optionally at least mono-substituted C₁₋₆-alkyl-aryl; optionally at least mono-substituted C₁₋₆-alkyl-heterocyclyl; or optionally at least mono-substituted C₁₋₆-alkyl-C₄₋₈-cycloalkyl.

11. Compound according to claim 1 or 6, having a general formula la, 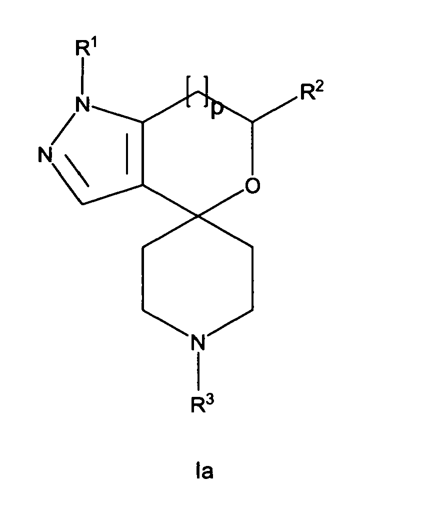 wherein
p is selected from 0 or 1;
R¹ is selected from linear or branched C₁₋₄-alkyl; or optionally at least monosubstituted aryl;
R² is selected from H; OH or a linear or branched OC₁₋₄-alkyl group;
R³ is selected from hydrogen; C₁₋₁₀-alkyl, linear or branched; C₃₋₈-alkenyl, linear or branched; optionally at least mono-substituted C₁₋₆-alkyl-aryl; optionally at least mono-substituted C₁₋₆-alkyl-heterocyclyl; or optionally at least mono-substituted C₁₋₆-alkyl-C₄₋₈-cycloalkyl.

12. Compound according to claim 1, **characterized in that** the compound is selected from
• 6'-Methoxy-1'-phenyl-4',6'-dihydro-1'H-spiro[piperidin-4,4'-furo[3,4-c]pyrazole];
• 1-Benzyl-6'-methoxy-1'-phenyl-4',6'-dihydro-1'H-spiro[piperidin-4,4'-furo[3,4-c]pyrazole];
• 1-Butyl-6'-methoxy-1'-phenyl-4',6'-dihydro-1'H-spiro[piperidin-4,4'-furo[3,4-c]pyrazole];
• 1-Benzyl-6'-methoxy-1'-phenyl-6',7'-dihydro-1'H-spiro[piperidin-4,4'-pyrano[4,3-c]pyrazole];
• 6'-Methoxy-1'-phenyl-1-propyl-6',7'-dihydro-1'H-spiro[piperidin-4,4'-pyrano[4,3-c]pyrazole];
• 1-Isopropyl-6'-methoxy-1'-phenyl-6',7'-dihydro-1'H-spiro[piperidin-4,4'-pyrano[4,3-c]pyrazole];
• 1-Butyl-6'-methoxy-1'-phenyl-6',7'-dihydro-1'H-spiro[piperidin-4,4'-pyrano[4,3-c]pyrazole];
• 1-Isobutyl-6'-methoxy-1'-phenyl-6',7'-dihydro-1'H-spiro[piperidin-4,4'-pyrano[4,3-c]pyrazole];
• 6'-Methoxy-1-pentyl-1'-phenyl-6',7'-dihydro-1'H-spiro[piperidin-4,4'-pyrano[4,3-c]pyrazole];
• 1-Isopentyl-6'-methoxy-1'-phenyl-6',7'-dihydro-1'H-spiro[piperidin-4,4'-pyrano[4,3-c]pyrazole];
• 6'-Methoxy-1-(3-methylbut-2-en-1-yl)-1'-phenyl-6',7'-dihydro-1'H-spiro[piperidin-4,4'-pyrano[4,3-c]pyrazole];
• 6'-Methoxy-1-octyl-1'-phenyl-6',7'-dihydro-1'H-spiro[piperidin-4,4'-pyrano[4,3-c]pyrazole];
• 1-(Cyclohexan-1-ylmethyl)-6'-methoxy-1'-phenyl-6',7'-dihydro-1'H-spiro[piperidin-4,4'-pyrano[4,3-c]pyrazole];
• 6'-Methoxy-1'-phenyl-1-(2-phenylethyl)-6',7'-dihydro-1'H-spiro[piperidin-4,4'-pyrano[4,3-c]pyrazole];
• 6'-Methoxy-1'-phenyl-1-(3-phenylpropyl)-6',7'-dihydro-1'H-spiro[piperidin-4,4'-pyrano[4,3-c]pyrazole];
• 6'-Methoxy-1'-phenyl-1-(4-phenylbutyl)-6',7'-dihydro-1'H-spiro[piperidin-4,4'-pyrano[4,3-c]pyrazole];
• 1-(Furan-2-ylmethyl)-6'-methoxy-1'-phenyl-6',7'-dihydro-1'H-spiro[piperidin-4,4'-pyrano[4,3-c]pyrazole];
• 6'-Methoxy-1-(4-methoxybenzyl)-1'-phenyl-6',7'-dihydro-1'H-spiro[piperidin-4,4'-pyrano[4,3-c]pyrazole];
• 1-(4-Fluorbenzyl)-6'-methoxy-1'-phenyl-6',7'-dihydro-1'H-spiro[piperidin-4,4'-pyrano[4,3-c]pyrazole];
• 1-Benzyl-6'-methoxy-1'-methyl-6', 7'-dihydro-1'H-spiro[piperidin-4,4'-pyrano[4,3-c]pyrazole];
• 6'-Methoxy-1'-methyl-6',7'-dihydro-1'H-spiro[piperidin-4,4'-pyrano[4,3-c]pyrazole];
• 6'-Methoxy-1'-methyl-1-(3-phenylpropyl)-6',7'-dihydro-1'H-spiro[piperidin-4,4'-pyrano[4,3-c]pyrazole];
• 1-(4-Fluorbenzyl)-6'-methoxy-1'-methyl-6',7'-dihydro-1'H-spiro[piperidin-4,4'-pyrano[4,3-c]pyrazole];
• 1-Isopentyl-6'-methoxy-1'-methyl-6', 7'-dihydro-1'H-spiro[piperidin-4,4'-pyrano[4,3-c]pyrazole];
• 6'-Methoxy-1'-methyl-1-propyl-6',7'-dihydro-1'H-spiro[piperidin-4,4'-pyrano[4,3-c]pyrazole];
• 1-Benzyl-1'-methyl-6',7'-dihydro-1'H-spiro[piperidin-4,4'-pyrano[4,3-c]pyrazole]-6'-ol;
• 1-Benzyl-1'-methyl-1'H-spiro[piperidin-4,4'-pyrano[4, 3-c]pyrazole];
• 1-Benzyl-1'-methyl-6',7'-dihydro-1'H-spiro[piperidin-4,4'-pyrano[4,3-c]pyrazole]; or
• 1'-Methyl-6',7'-dihydro-1'H-spiro[piperidin-4,4'-pyrano[4,3-c]pyrazole];
• 1-Benzyl-6'-hydroxy-1'-phenyl-6',7'-dihydro-1'H-spiro[piperidin-4,4'-pyrano[4,3-c]pyrazole];
• 1-Benzyl-1'-phenyl-6',7'-dihydro-1'H-spiro[piperidin-4,4'-pyrano[4,3-c]pyrazole];
• 1'-phenyl-6',7'-dihydro-1'H-spiro[piperidin-4,4'-pyrano[4,3-c]pyrazole];
• 1-Isopentyl-1'-phenyl-6',7'-dihydro-1'H-spiro[piperidin-4,4'-pyrano[4,3-c]pyrazole];
• 1-(3-methylbut-2-en-1-yl)-1'-phenyl-6',7'-dihydro-1'H-spiro[piperidin-4,4'-pyrano[4,3-c]pyrazole];
• 1'-phenyl-1-(3-phenylpropyl)-6',7'-dihydro-1'H-spiro[piperidin-4,4'-pyrano[4,3-c]pyrazole];
• 1-Isopentyl-1'-methyl-6',7'-dihydro-1'H-spiro[piperidin-4,4'-pyrano[4,3-c]pyrazole];
• 1-(3-methylbut-2-en-1-yl)-1'-methyl-6',7'-dihydro-1'H-spiro[piperidin-4,4'-pyrano[4,3-c]pyrazole];
• 1'-methyl-1-(3-phenylpropyl)-6', 7'-dihydro-1'H-spiro[piperidin-4,4'-pyrano[4,3-c]pyrazole];
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
preferably from
• 6'-Methoxy-1'-phenyl-4',6'-dihydro-1'H-spiro[piperidin-4,4'-furo[3,4-c]pyrazole];
• 1-Benzyl-6'-methoxy-1'-phenyl-4',6'-dihydro-1'H-spiro[piperidin-4,4'-furo[3,4-c]pyrazole];
• 1-Butyl-6'-methoxy-1'-phenyl-4',6'-dihydro-1'H-spiro[piperidin-4,4'-furo[3,4-c]pyrazole];
• 1-Benzyl-6'-methoxy-1'-phenyl-6',7'-dihydro-1'H-spiro[piperidin-4,4'-pyrano[4,3-c]pyrazole];
• 6'-Methoxy-1'-phenyl-1-propyl-6',7'-dihydro-1'H-spiro[piperidin-4,4'-pyrano[4,3-c]pyrazole];
• 1-Isopropyl-6'-methoxy-1'-phenyl-6',7'-dihydro-1'H-spiro[piperidin-4,4'-pyrano[4,3-c]pyrazole];
• 1-Butyl-6'-methoxy-1'-phenyl-6',7'-dihydro-1'H-spiro[piperidin-4,4'-pyrano[4,3-c]pyrazole];
• 1-Isobutyl-6'-methoxy-1'-phenyl-6',7'-dihydro-1'H-spiro[piperidin-4,4'-pyrano[4,3-c]pyrazole];
• 6'-Methoxy-1-pentyl-1'-phenyl-6',7'-dihydro-1'H-spiro[piperidin-4,4'-pyrano[4,3-c]pyrazole];
• 1-Isopentyl-6'-methoxy-1'-phenyl-6',7'-dihydro-1'H-spiro[piperidin-4,4'-pyrano[4,3-c]pyrazole];
• 6'-Methoxy-1-(3-methylbut-2-en-1-yl)-1'-phenyl-6',7'-dihydro-1'H-spiro[piperidin-4,4'-pyrano[4,3-c]pyrazole];
• 6'-Methoxy-1-octyl-1'-phenyl-6',7'-dihydro-1'H-spiro[piperidin-4,4'-pyrano[4,3-c]pyrazole];
• 1-(Cydohexan-1-ylmethyl)-6'-methoxy-1'-phenyl-6',7'-dihydro-1'H-spiro[piperidin-4,4'-pyrano[4,3-c]pyrazole];
• 6'-Methoxy-1'-phenyl-1-(2-phenylethyl)-6',7'-dihydro-1'H-spiro[piperidin-4,4'-pyrano[4,3-c]pyrazole];
• 6'-Methoxy-1'-phenyl-1-(3-phenylpropyl)-6',7'-dihydro-1'H-spiro[piperidin-4,4'-pyrano[4,3-c]pyrazole];
• 6'-Methoxy-1'-phenyl-1-(4-phenylbutyl)-6',7'-dihydro-1'H-spiro[piperidin-4,4'-pyrano[4,3-c]pyrazole];
• 1-(Furan-2-ylmethyl)-6'-methoxy-1'-phenyl-6',7'-dihydro-1'H-spiro[piperidin-4,4'-pyrano[4,3-c]pyrazole];
• 6'-Methoxy-1-(4-methoxybenzyl)-1'-phenyl-6',7'-dihydro-1'H-spiro[piperidin-4,4'-pyrano[4,3-c]pyrazole];
• 1-(4-Fluorbenzyl)-6'-methoxy-1'-phenyl-6',7'-dihydro-1'H-spiro[piperidin-4,4'-pyrano[4,3-c]pyrazole];
• 1-Benzyl-6'-methoxy-1'-methyl-6',7'-dihydro-1'H-spiro[piperidin-4,4'-pyrano[4,3-c]pyrazole];
• 6'-Methoxy-1'-methyl-6',7'-dihydro-1'H-spiro[piperidin-4,4'-pyrano[4,3-c]pyrazole];
• 6'-Methoxy-1'-methyl-1-(3-phenylpropyl)-6', 7'-dihydro-1'H-spiro[piperidin-4,4'-pyrano[4,3-c]pyrazole];
• 1-(4-Fluorbenzyl)-6'-methoxy-1'-methyl-6', 7'-dihydro-1'H-spiro[piperidin-4,4'-pyrano[4,3-c]pyrazole];
• 1-Isopentyl-6'-methoxy-1'-methyl-6',7'-dihydro-1'H-spiro[piperidin-4,4'-pyrano[4,3-c]pyrazole];
• 6'-Methoxy-1'-methyl-1-propyl-6',7'-dihydro-1'H-spiro[piperidin-4,4'-pyrano[4,3-c]pyrazole];
• 1-Benzyl-1'-methyl-6',7'-dihydro-1'H-spiro[piperidin-4,4'-pyrano[4,3-c]pyrazole]-6'-ol;
• 1-Benzyl-1'-methyl-1'H-spiro[piperidin-4,4'-pyrano[4,3-c]pyrazole];
• 1-Benzyl-1'-methyl-6',7'-dihydro-1'H-spiro[piperidin-4,4'-pyrano[4,3-c]pyrazole]; or
• 1'-Methyl-6',7'-dihydro-1'H-spiro[piperidin-4,4'-pyrano[4,3-c]pyrazole]; optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

13. Process for the production of a compound according to claim 1, wherein a compound of formula III 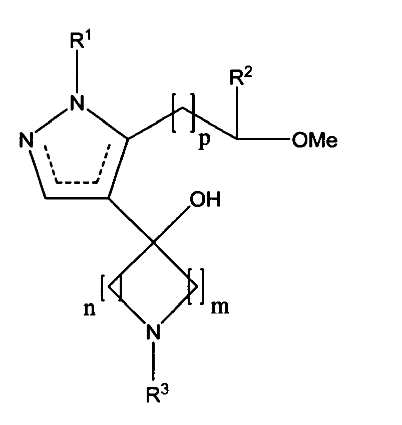 , wherein R¹, R², R³, m, n and p and the dotted line are as defined in claim 1 is reacted with acid to form acompound according the formula I.

14. Process according to claim 13, wherein in the compound of general formula III
• R³ is C(O)OR' or benzyl; and/or
• R² is OCH₃; and/or
• R¹ is C₁₋₆ alkyl or aryl; and/or
• m and n are 2.

15. Process for the production of a compound according to formula Ia, wherein a compound of formula IIIa 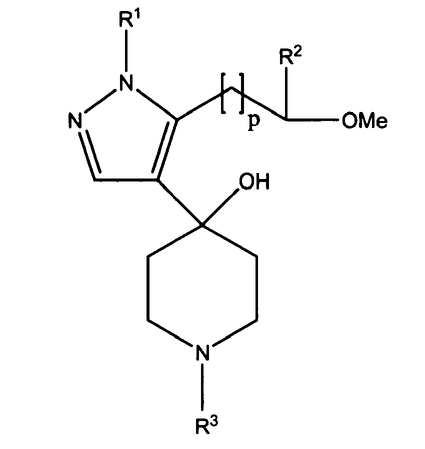 , wherein R¹, R², R³ and p are as defined in claim 1 is reacted with acid to form a compound according the formula Ia.

16. Process according to claim 15, wherein in the compound according to formula IIIa
• R³ is C(O)OR' or benzyl; and/or
• R² is OCH₃; and/or
• R¹ is C₁₋₆ alkyl or aryl.

17. Process according to claims 13 to 16, wherein as a next step a compound according to formula I or Ia in which p is 0 and R³ is C(O)OR' is reacted with a KOH solution in water to form a compound according to formulas I or Ia with R³ being H.

18. Process according to claims 13 to 16, wherein as a next step a compound according to formula I or la in which p is 1 and R³ is benzyl is reacted with NH₄⁺ HCOO⁻ with a Pd/C catalysator to form a compound according to formulas I or la with R³ being H.

19. Process according to claims 17 or 18, wherein as a next step a compound according to formula I or Ia in which R³ is hydrogen is reacted with a compound R³-X with X being a leaving group and K₂CO₃ under reflux to form a compound according to formulas I or Ia with R³ being as defined in claims 1 to 11, except H.

20. A pharmaceutical composition which comprises a compound as defined in any of claims 1-12 or a pharmaceutically acceptable salt, prodrug, isomer or solvate thereof, and a pharmaceutically acceptable carrier, adjuvant or vehicle.

21. Use of a compound as defined in any of claims 1-12 in the manufacture of a medicament.

22. Use of a compound as defined in any of claims 1-12 in the manufacture of a medicament for the treatment or prophylaxis of a sigma receptor mediated disease or condition.

23. Use according to claim 22 wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

24. Use according to claim 22 wherein the disease is pain, especially neuropathic pain, inflammatory pain or other pain conditions, allodynia and/or hyperalgesia, especially mechanical allodynia.

25. Use of a compound as defined in any of claims 1-12 as pharmacological tool or as anxiolytic or immunosuppressant.
